# EUROPEAN PATENT SPECIFICATION

(11) **EP 1 799 868 B1**
(45) Date of publication and mention of the grant of the patent: **18.01.2012**
(21) Application number: 05810417.5
(22) Date of filing: 26.08.2005
(51) Int. Cl.: G01N 33/576, C07K 14/18

(54) **HCV NON-STRUCTURAL PROTEIN MUTANTS AND USES THEREOF**
NICHTSTRUKTURELLE HCV-PROTEINMUTANTEN UND VERWENDUNGSMÖGLICHKEITEN DAFÜR
MUTANTS DE PROTEINES NON STRUCTURELLES DU VHC ET LEURS UTILISATIONS

(30) Priority: 27.08.2004 US 604858 P; 12.10.2004 US 618390 P; 22.10.2004 US 621502 P; 25.10.2004 US 621790 P
(43) Date of publication of application: 27.06.2007
(73) Proprietor: Novartis Vaccines and Diagnostics, Inc., Emeryville, CA 94662-8097 (US)
(72) Inventor: CHIEN, David Y., c/o Chiron Corporation, Emeryville, CA 94608 (US); COIT, Doris, c/o Chiron Corporation, Emeryville, CA 94608 (US); HU, Celine Yuan-Hwei, c/o Chiron Corporation, Emeryville, CA 94608 (US); LIN, Sansan, c/o Chiron Corporation, Emeryville, CA 94608 (US); MEDINA-SELBY, Angelica, c/o Chiron Corporation, Emeryville, CA 94608 (US)
(74) Representative: Marshall, Cameron John
(86) International application number: PCT/US2005/030325
(87) International publication number: WO 2006/024020

(56) References cited:
- WO-A-01/96870
- WO-A2-01/38360
- WO-A2-2004/005473
- CHIEN DAVID Y ET AL: "Use of a novel hepatitis C virus (HCV) major-epitope chimeric polypeptide for diagnosis of HCV infection" JOURNAL OF CLINICAL MICROBIOLOGY, WASHINGTON, DC, US, vol. 37, no. 5, 1 May 1999 (1999-05-01), pages 1393-1397, XP002155465 ISSN: 0095-1137

## Description

### Technical Field

The present invention pertains generally to hepatitis C virus (HCV) constructs and methods of using the same. More particularly, the invention relates to immunogenic, immunoreactive HCV proteins with modified NS3 protease domains such that proteolytic activity of the NS3 molecule is inhibited. The modified proteins retain conformational epitopes and are therefore useful in immunoassays for diagnosing HCV infection as well as for stimulating immune responses against HCV.

### Background Of The Invention

Hepatitis C Virus (HCV) is the principal cause of parenteral non-A, non-B hepatitis (NANBH) which is transmitted largely through body blood transfusion and body fluid exchange. The virus is present in 0.4 to 2.0% of the general population in the United States. Chronic hepatitis develops in about 50% of infections and of these, approximately 20% of infected individuals develop liver cirrhosis which sometimes leads to hepatocellular carcinoma. Accordingly, the study and control of the disease is of medical importance.

HCV was first identified and characterized as a cause of NANBH by Houghton et al. The viral genomic sequence of HCV is known, as are methods for obtaining the sequence. See, e.g., International Publication Nos. WO 89/04669; WO 90/11089; and WO 90/14436. HCV has a 9.5 kb positive-sense, single-stranded RNA genome and is a member of the Flaviridae family of viruses. At least six distinct, but related genotypes of HCV, based on phylogenetic analyses, have been identified (Simmonds et al., J. Gen. Virol. (1993) 74:2391-2399). The virus encodes a single polyprotein having more than 3000 amino acid residues (Choo et al., Science (1989) 244:359-362; Choo et al., Proc. Natl. Acad. Sci. USA (1991) 88:2451-2455; Han et al., Proc. Natl. Acad. Sci. USA (1991) 88:1711-1715). The polyprotein is processed co- and post-translationally into both structural and non-structural (NS) proteins.

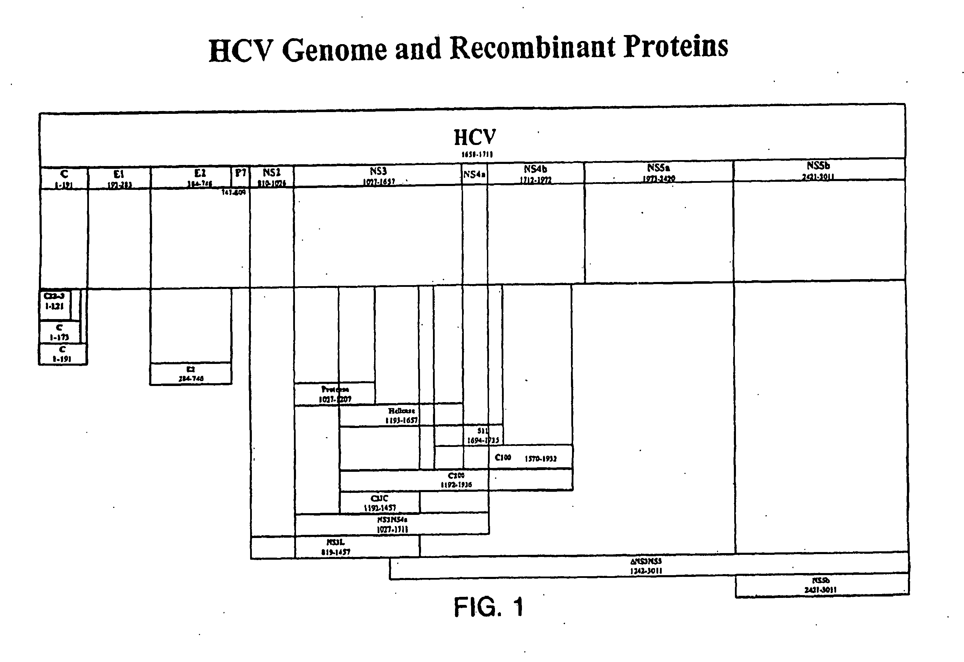

In particular, as shown in Figure 1, several proteins are encoded by the HCV genome. The order and nomenclature of the cleavage products of the HCV polyprotein is as follows: NH₂-C-E1-E2-P7-NS2-NS3-NS4a-NS4b-NS5a-NS5b-COOH. Initial cleavage of the polyprotein is catalyzed by host proteases which liberate three structural proteins, the N-terminal nucleocapsid protein (termed "core") and two envelope glycoproteins, "E1" (also known as E) and "E2" (also known as E2/NS1), as well as nonstructural (NS) proteins that contain the viral enzymes. The NS regions are termed NS2, NS3, NS4, NS4a, NS4b, NS5a and NS5b. NS2 is an integral membrane protein with proteolytic activity. NS2, either alone or in combination with NS3, cleaves the NS2-NS3 scissile bond which in turn generates the NS3 N-terminus and releases a large polyprotein that includes both serine protease and RNA helicase activities. The NS3 protease serves to process the remaining polyprotein. In particular, the HCV NS3 protein is a 630 amino acid protein containing three functional domains. The serine-like protease domain is located in the amino terminus, whereas helicase and NTPase activity are in the carboxy terminus. The serine protease of NS3 is responsible for the cleavage at the junction of NS3/4a, NS4a/b, NS4b/5a and NS5a/b.

A number of general and specific polypeptides useful as immunological and diagnostic reagents for HCV, derived from the HCV polyprotein, have been described. See, e.g., Houghton et al., European Publication Nos. 318,216 and 388,232; Choo et al., Science (1989) 244:359-362; Kno et al., Science (1989) 244:362-364; Houghton et al., Hepatology (1991) 14:381-388; Chien et al., Proc. Natl. Acad. Sci. USA (1992) 89:10011-10015; Chien et al., J. Gastroent. Hepatol. (1993) 8:S33-39; Chien et al., International Publication No. WO 93/00365; Chien, D.Y., International Publication No. WO 94/01778. These publications provide an extensive background on HCV generally, as well as on the manufacture and uses of HCV polypeptide immunological reagents.

Sensitive, specific methods for screening and identifying carriers of HCV and HCV-contaminated blood or blood products would provide an important advance in medicine. Post-transfusion hepatitis (PTH) occurs in approximately 10% of transfused patients, and HCV has accounted for up to 90% of these cases. Patient care as well as the prevention and transmission of HCV by blood and blood products or by close personal contact require reliable diagnostic and prognostic tools. Accordingly, several assays have been developed for the serodiagnosis of HCV infection. See, e.g., Choo et al., Science (1989) 244:359-362; Kuo et al., Science (1989) 244:362-364; Choo et al., Br. Med. Bull. (1990) 46:423-441; Ebeling et al., Lancet (1990) 335:982-983; van der Poel et al., Lancet (1990) 335:558-560; van der Poel et al., Lancet (1991) 337:317-319; Chien, D.Y., International Publication No. WO 94/01778; Valenzuela et al., International Publication No. WO 97/44469; and Kashiwakuma et al., U.S. Patent No. 5,871,904.

U.S. Patent No. 6,632,601 describes immunoassays using NS3/4a conformational epitopes, in combination with multiple epitope fusion antigens (MEFAs). The assays provide sensitive and reliable methods for detecting early HCV seroconversion. NS3/4a, expressed in yeast and purified under non-denaturing conditions as described in U.S. Patent No. 6,632,601, contains both protease and helicase function. Because NS3/4a purified in this manner preserves the native conformation, it has been found to be more sensitive than the c200 or c33c antigens in early seroconversion antibody detection. In antibody assays using NS3/4a and MEFA 7.1 as antigens, seroconversion antibodies were detected 2-14 days earlier than currently marketed HCV assays. However, the NS3/4 protein undergoes self-hydrolysis and cleaves MEFA 7.1 due to the NS3 protease activity.

International Publication Nos. WO 04/00547 and WO 01/38360 describe HCV proteins including mutated NS3 protease domains with reduced proteolytic activity. However, there remains a need for sensitive, accurate diagnostic and prognostic tools in order to provide adequate patient care as well as to prevent transmission of HCV by blood and blood products or by close personal contact.
WO 01/38360 and WO 2004/005473 disclose polypeptides comprising a modified HCV NS3 protease domain, wherein the protease activity of the domain is inhibited, as well as immunoassay supports comprising the polypeptides. WO 2004/005473 also discloses amino acid substitutions corresponding to His-1083, Asp-1105 and/ or Ser-1165 including substitution of Ala for the amino acid corresponding to Ser-1165.
WO 01/96870 discloses a conformational epitope of NS3/4a which differs from the native sequence at amino acid positions 1428 and 1429 of the HCV-1 full length sequence in that the Thr normally occurring at position 1428 has been mutated to Pro and the Ser normally occurring at position 1429 has been mutated to Ile. This epitope is used in immunoassays for the diagnosis of HCV.

### Summary of the Invention

The present invention is based in part, on the finding that the use of HCV NS3 polypeptides with modified protease domains provides superior reagents for detecting HCV infection. The modified NS3 polypeptides retain conformational epitopes and hence immunoreactivity, while eliminating protease activity. Because the NS3 polypeptides are modified to eliminate protease activity, they are especially useful with other HCV polypeptides that retain NS3 proteolytic cleavage sites and that would otherwise be proteolytically cleaved by a functional NS3 protease. The modified NS3 polypeptides can therefore be used alone or in combination with other HCV reagents, and in particular, with multiple epitope fusion antigens (MEFAs) for accurately and efficiently detecting the presence of HCV infection. The use of MEFAs provides the added advantages of decreased masking problems, improved selectivity and improved sensitivity for detecting antibodies by allowing a greater number of epitopes on a unit area of substrate. The assays described herein may be used to detect HCV infection caused by any of the six known genotypes of HCV.

The invention is directed to an isolated polypeptide comprising the aminoacid sequence of SEQ ID NO: 2, 4 or 6, wherein the polypeptide comprises on NS3 conformational epitope. In further embodiments the invention is directed to an immunoassay solid support comprising a polypeptide comprising the amino acid sequence of SEQ ID NO:2, 4 or 6 which reacts specifically with anti-HCV antibodies present in a biological sample from an HCV-infected individual.

In yet additional embodiments, the immunoassay solid support further comprises a multiple epitope fusion antigen bound to the support, wherein the polypeptide and/or the multiple epitope fusion antigen react specifically with anti-HCV antibodies present in a biological sample from an HCV-infected individual.

In certain embodiments, the multiple epitope fusion antigen comprises the amino acid sequence depicted in SEQ ID NO:10, or an amino acid sequence with at least 80% sequence identity thereto, such as at least 90% or at least at least 98% sequence identity sequence identity thereto, and that reacts specifically with anti-HCV antibodies present in a biological sample from an HCV-infected individual. In additional embodiments, the multiple epitope fusion antigen consists of the amino acid sequence depicted in SEQ ID NO:10.

In further embodiments, the invention is directed to a method of detecting HCV infection in a biological sample. The method comprises:
(a) providing an immunoassay solid support as described above;
(b) combining a biological sample with the solid support under conditions which allow anti-HCV antibodies, when present in the biological sample, to bind to the polypeptide and/or the multiple epitope fusion antigen to form a first immune complex;
(c) adding to the solid support from step (b) under complex forming conditions a detectably labeled antibody, wherein the labeled antibody is reactive with the immune complex; and
(d) detecting second immune complexes formed between the detectably labeled antibody and the first immune complex, if any, as an indication of HCV infection in the biological sample.

In still further embodiments, the invention is directed to an immunodiagnostic test kit comprising an immunoassay solid support as described above, and instructions for conducting the immunodiagnostic test.

In yet further embodiments, the invention is directed to a polynucleotide comprising a coding sequence for any one of the above polypeptides, and a recombinant vector comprising the polynucleotide and control elements operably linked to the polynucleotide whereby the coding sequence can be transcribed and translated in a host cell. In additional embodiments, the invention is directed to a host cell transformed with the recombinant vector. In still further embodiments, the invention is directed to a method of producing a recombinant polypeptide comprising:
(a) providing a population of host cells described above; and
(b) culturing the population of cells under conditions whereby the polypeptide encoded by the coding sequence present in the recombinant vector is expressed.

In additional embodiments, the invention is directed to a method of producing an immunoassay solid support, comprising:
(a) providing a solid support; and
(b) binding to the solid support at least one polypeptide as described above.
In certain embodiments, the method further comprises binding to the solid support at a discrete position a multiple epitope fusion antigen.

These and other aspects of the present invention will become evident upon reference to the following detailed description and attached drawings. In addition, various references are set forth herein which describe in more detail certain procedures or compositions.

### Brief Description of the Drawings

Figure 1 is a diagrammatic representation of the HCV genome, depicting the various regions of the polyprotein from which the present assay reagents (proteins and antibodies) are derived.

Figure 2 is a schematic drawing of a representative immunoassay using modified NS3 polypeptides according to the invention where HCV antigens are immobilized on a solid support.

Figure 3 (SEQ ID NOS:7 and 8) depicts the DNA and corresponding amino acid sequence of a representative native, unmodified NS3 protease domain.

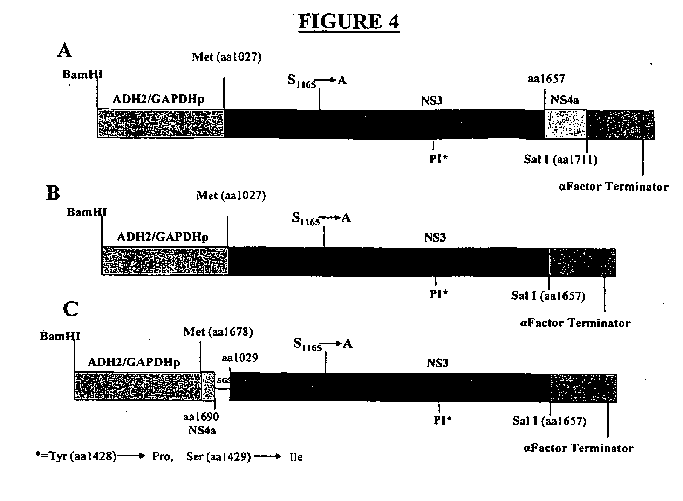

Figures 4A-4C are diagrammatic representations of mutant NS3 proteins according to the invention. In Figures 4A-4C, the Ser normally present at position 1165 is substituted with Ala. Additionally, all of the mutants depicted in Figures 4A-4C include a substitution of Pro for Thr normally present at position 1428, and Ile for Ser normally present at position 1429. In Figure 4A, the protein includes in N-terminal to C-terminal direction, an N-terminal Met, amino acids 1027-1657 of NS3 and amino acids 1658-1711 of NS4a and thus includes the full-length NS3 and NS4a domains of the HCV polyprotein. In Figure 4B, the protein includes in N-terminal to C-terminal direction, an N-terminal Met, and amino acids 1027 to 1657 of NS3 and thus includes the full-length NS3 domain of the HCV polyprotein. In Figure 4C, the protein includes in N-terminal to C-terminal direction, an N-terminal Met, amino acids 1678-1690 of NS4a, followed by the amino acid sequence Ser-Gly-Ser, then amino acids 1029 to 1657 of NS3.

Figures 5A-5C (SEQ ID NOS:1 and 2) depict the nucleotide sequence and corresponding amino acid sequence of the protein from Figure 4A, termed "NS34aPI.1165."

Figures 6A-6C (SEQ ID NOS:3 and 4) depict the nucleotide sequence and corresponding amino acid sequence of the protein from Figure 4B, termed "NS3PI.1165."

Figures 7A-7C (SEQ ID NOS:5 and 6) depict the nucleotide sequence and corresponding amino acid sequence of the protein from Figure 4C, termed "d.4a.t.NS3PI.1165."

Figure 8 is a diagrammatic representation of MEFA 7.1.

Figures 9A-9F (SEQ ID NOS:9 and 10) depict the DNA and corresponding amino acid sequence of MEFA 7.1.

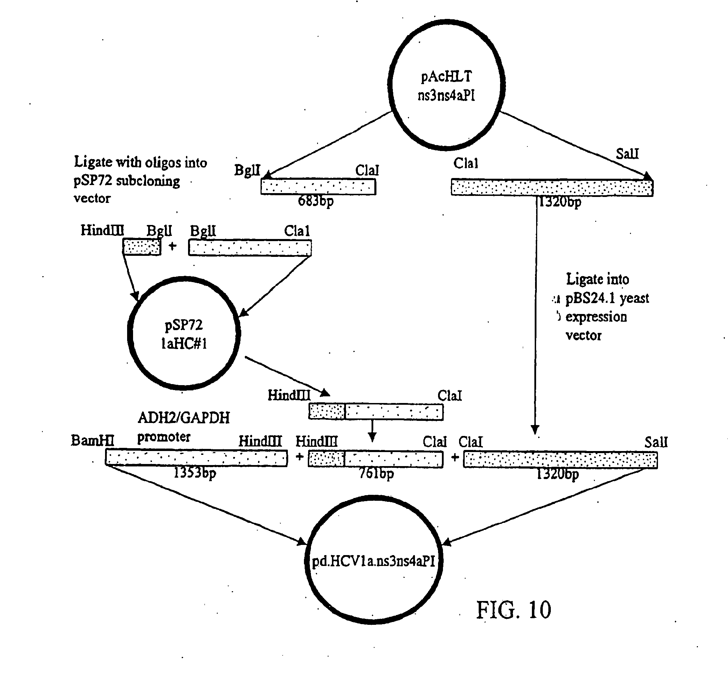

Figure 10 is a diagram of the construction of pd.HCV1a.ns3ns4aPI.

Figures 11A-11C show representative MEFAs for use with the subject immunoassays. Figure 11A is a diagrammatic representation of MEFA 3. Figure 11B is a diagrammatic representation of MEFA 5. Figure 11C is a diagrammatic representation of MEFA 6.

Figure 12 is a schematic drawing of a representative immunoassay format using modified NS3 polypeptides with a streptavidin-coated solid support.

Figure 13 is a representation of a Coomassie stained SDS-PAGE gel demonstrating that the NS3 mutant proteins do not undergo self-hydrolysis and do not cleave MEFA 7.1. Lane 1: NS34aPI; Lane 2: NS34aPI.1165; Lane 3: NS3PI.1165; Lane 4: NS34aPI + MEFA 7.1, t=0; Lane 5: NS34aPI + MEFA 7.1, t=40 min; Lane 6: NS34aPI + MEFA 7.1, t=2 hr; Lane 7: NS34aPI. 1165+ MEFA 7.1, t=0; Lane 8: NS34aPI.1165 + MEFA 7.1, t=40 min; Lane 9: NS34aPI.1165 + MEFA 7.1, t=2 hr; Lane 10: NS3PI.1165 + MEFA 7.1, t=0; Lane 11: NS3PI.1165 + MEFA 7.1, t=40 min; Lane 12: NS3PI.1165 + MEFA 7.1, t=2 hr; Lane 13: MEFA 7.1.

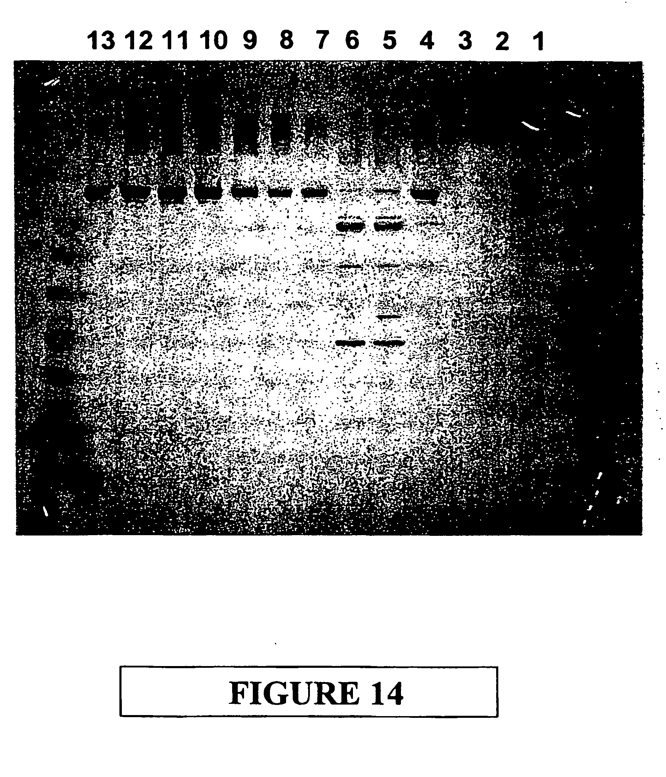

Figure 14 is a representation of a Western blot, using anti-SOD as the primary antibody against MEFA 7.1 and anti-mouse-HRP as the secondary antibody, demonstrating that the NS3 mutant proteins do not undergo self-hydrolysis and do not cleave MEFA 7.1. Lane 1: NS34aPI; Lane 2: NS34aPI.1165; Lane 3: NS3PI.1165; Lane 4: NS34aPI + MEFA 7.1, t=0; Lane 5: NS34aPI + MEFA 7.1, t=40 min; Lane 6: NS34aPI + MEFA 7.1, t=2 hr; Lane 7: NS34aPI.1165+ MEFA 7.1, t=0; Lane 8: NS34aPL 1165 + MEFA 7.1, t=40 min; Lane 9: NS34aPI.1165 + MEFA 7.1, t=2 hr; Lane 10: NS3PI.1165 + MEFA 7.1, t=0; Lane 11: NS3PI.1165 + MEFA 7.1, t=40 min; Lane 12: NS3PL 1165 + MEFA 7.1, t=2 hr; Lane 13: MEFA 7.1.

### Detailed Description of the Invention

The practice of the present invention will employ, unless otherwise indicated, conventional methods of chemistry, biochemistry, recombinant DNA techniques and immunology, within the skill of the art. Such techniques are explained fully in the literature. See, e.g., Fundamental Virology, 2nd Edition, vol. I & II (B.N. Fields and D.M. Knipe, eds.); Handbook of Experimental Immunology, Vols. I-IV (D.M. Weir and C.C. Blackwell eds., Blackwell Scientific Publications); T.E. Creighton, Proteins: Structures and Molecular Properties (W.H. Freeman and Company, 1993); A.L. Lehninger, Biochemistry (Worth Publishers, Inc., current addition); Sambrook, et al., Molecular Cloning: A Laboratory Manual (2nd Edition, 1989); Methods In Enzymology (S. Colowick and N. Kaplan eds., Academic Press, Inc.).

It must be noted that, as used in this specification and the appended claims, the singular forms "a", "an" and "the" include plural referents unless the content clearly dictates otherwise. Thus, for example, reference to "an antigen" includes a mixture of two or more antigens, and the like.

The following amino acid abbreviations are used throughout the text:

| | |
|---|---|
| Alanine: Ala (A) | Arginine: Arg (R) |
| Asparagine: Asn (N) | Aspartic acid: Asp (D) |
| Cysteine: Cys (C) | Glutamine: Gln (Q) |
| Glutamic acid: Glu (E) | Glycine: Gly (G) |
| Histidine: His (H) | Isoleucine: Ile (I) |
| Leucine: Leu (L) | Lysine: Lys (K) |
| Methionine: Met (M) | Phenylalanine: Phe (F) |
| Proline: Pro (P) | Serine: Ser (S) |
| Threonine: Thr (T) | Tryptophan: Trp (W) |
| Tyrosine: Tyr (Y) | Valine: Val (V) |

### I. Definitions

In describing the present invention, the following terms will be employed, and are intended to be defined as indicated below.

The terms "polypeptide" and "protein" refer to a polymer of amino acid residues and are not limited to a minimum length of the product. Thus, peptides, oligopeptides, dimers, multimers, and the like, are included within the definition. Both full-length proteins and fragments thereof are encompassed by the definition. The terms also include postexpression modifications of the polypeptide, for example, glycosylation, acetylation, phosphorylation and the like. Furthermore, for purposes of the present invention, a "polypeptide" refers to a protein which includes modifications, such as deletions, additions and substitutions (generally conservative in nature), to the native sequence, so long as the protein maintains the desired activity. These modifications may be deliberate, as through site-directed mutagenesis, or may be accidental, such as through mutations of hosts which produce the proteins or errors due to PCR amplification.

An HCV polypeptide is a polypeptide, as defined above, derived from the HCV polyprotein. The polypeptide need not be physically derived from HCV, but may be synthetically or recombinantly produced. Moreover, the polypeptide may be derived from any of the various HCV strains and isolates, such as, but not limited to, any of the isolates from strains 1, 2, 3, 4, 5 or 6 of HCV. A number of conserved and variable regions are known between these strains and, in general, the amino acid sequences of epitopes derived from these regions will have a high degree of sequence homology, e.g., amino acid sequence homology of more than 30%, preferably more than 40%, when the two sequences are aligned. Thus, for example, the term "NS3/4a" polypeptide refers to native NS3/4a from any of the various HCV strains, as well as NS3/4a analogs, muteins and immunogenic fragments, as defined further below. The complete genotypes of many of these strains are known. See, e.g., U.S. Patent No. 6,150,087 and GenBank Accession Nos. AJ238800 and AJ238799.

A polypeptide "derived from" an HCV polyprotein intends a polypeptide which comprises a sequence of one or more regions or portions of regions of the reference HCV polyprotein. Typically, the polypeptide is composed of regions or portions of regions that include epitopes, and will generally have an amino acid sequence substantially homologous to the reference polypeptide, as defined below. Thus, the term "derived from" is used to identify the original source of a molecule but is not meant to limit the method by which the molecule is made which can be, for example, by chemical synthesis or recombinant means.

The terms "analog" and "mutein" refer to biologically active derivatives of the reference molecule, or fragments of such derivatives, that retain desired activity, such as immunoreactivity in the assays described herein. In general, the term "analog" refers to compounds having a native polypeptide sequence and structure with one or more amino acid additions, substitutions (generally conservative in nature, or in the case of modified NS3, non-conservative in nature at the active proteolytic site) and/or deletions, relative to the native molecule, so long as the modifications do not destroy immunogenic activity. The term "mutein" refers to polypeptides having one or more amino acid-like molecules including but not limited to compounds comprising only amino and/or imino molecules, polypeptides containing one or more analogs of an amino acid (including, for example, unnatural amino acids, etc.), polypeptides with substituted linkages, as well as other modifications known in the art, both naturally occurring and non-naturally occurring (e.g., synthetic), cyclized, branched molecules and the like. The term also includes molecules comprising one or more *N*-substituted glycine residues (a "peptoid") and other synthetic amino acids or peptides. (See, e.g., U.S. Patent Nos. 5,831,005; 5,877,278; and 5,977,301; Nguyen et al., Chem Biol. (2000) 7:463-473; and Simon et al., Proc. Natl. Acad. Sci. USA (1992) 89:9367-9371 for descriptions of peptoids). Preferably, the analog or mutein has at least the same immunoactivity as the native molecule. Methods for making polypeptide analogs and muteins are known in the art and are described further below.

As explained above, analogs generally include substitutions that are conservative in nature, i.e., those substitutions that take place within a family of amino acids that are related in their side chains. Specifically, amino acids are generally divided into four families: (1) acidic -- aspartate and glutamate; (2) basiclysine, arginine, histidine; (3) non-polar -- alanine, valine, leucine, isoleucine, proline, phenylalanine, methionine, tryptophan; and (4) uncharged polar -- glycine, asparagine, glutamine, cysteine, serine threonine, tyrosine. Phenylalanine, tryptophan, and tyrosine are sometimes classified as aromatic amino acids. For example, it is reasonably predictable that an isolated replacement of leucine with isoleucine or valine, an aspartate with a glutamate, a threonine with a serine, or a similar conservative replacement of an amino acid with a structurally related amino acid, will not have a major effect on the biological activity. For example, the polypeptide of interest may include up to about 5-10 conservative or non-conservative amino acid substitutions, or even up to about 15-25 conservative or non-conservative amino acid substitutions, or any integer between 5-25, so long as the desired function of the molecule remains intact. One of skill in the art may readily determine regions of the molecule of interest that can tolerate change by reference to Hopp/Woods and Kyte-Doolittle plots, well known in the art.

By "modified NS3" is meant an NS3 polypeptide with a modification such that protease activity of the NS3 polypeptide is disrupted. The modified NS3 polypeptides therefore exhibit less protease activity as compared with the parent, unmodified NS3 polypeptide. The modification can include one or more amino acid additions, substitutions (generally non-conservative in nature) and/or deletions, relative to the native molecule, wherein the protease activity of the NS3 polypeptide is disrupted. Methods of measuring protease activity are discussed further below.

By "fragment" is intended a polypeptide consisting of only a part of the intact full-length polypeptide sequence and structure. The fragment can include a C-terminal deletion and/or an N-terminal deletion of the native polypeptide. An "immunogenic fragment" of a particular HCV protein will generally include at least about 5-10 contiguous amino acid residues of the full-length molecule, preferably at least about 15-25 contiguous amino acid residues of the full-length molecule, and most preferably at least about 20-50 or more contiguous amino acid residues of the full-length molecule, that define an epitope, or any integer between 5 amino acids and the full-length sequence, provided that the fragment in question retains immunoreactivity in the assays described herein. For example, preferred immunogenic fragments, include but are not limited to fragments of HCV core that comprise, e.g., amino acids 10-45, 10-53, 67-88, and 120-130 of the polyprotein, epitope 5-1-1 (in the NS4a/NS4b region of the viral genome) as well as defined epitopes derived from any of the regions of the polyprotein shown in Figure 1, such as but not limited to the E1, E2, NS3 (e.g., polypeptide c33c from the NS3 region), NS4 (e.g., polypeptide c100 from the NS3/NS4 regions), NS3/4a and NS5 regions of the HCV polyprotein, as well as any of the other various epitopes identified from the HCV polyprotein. See, e.g., Chien et al., Proc. Natl. Acad. Sci. USA (1992) 89:10011-10015; Chien et al., J. Gastroent. Hepatol. (1993) 8:S33-39; Chien et al., International Publication No. WO 93/00365; Chien, D.Y., International Publication No. WO 94/01778; U.S. Patent Nos. 6,150,087 and 6,121,020.

The term "epitope" as used herein refers to a sequence of at least about 3 to 5, preferably about 5 to 10 or 15, and not more than about 1,000 amino acids (or any integer therebetween), which define a sequence that by itself or as part of a larger sequence, binds to an antibody generated in response to such sequence. There is no critical upper limit to the length of the fragment, which may comprise nearly the full-length of the protein sequence, or even a fusion protein comprising two or more epitopes from the HCV polyprotein. An epitope for use in the subject invention is not limited to a polypeptide having the exact sequence of the portion of the parent protein from which it is derived. Indeed, viral genomes are in a state of constant flux and contain several variable domains which exhibit relatively high degrees of variability between isolates. Thus the term "epitope" encompasses sequences identical to the native sequence, as well as modifications to the native sequence, such as deletions, additions and substitutions (generally conservative in nature).

Regions of a given polypeptide that include an epitope can be identified using any number of epitope mapping techniques, well known in the art. See, e.g., Epitope Mapping Protocols in Methods in Molecular Biology, Vol. 66 (Glenn E. Morris, Ed., 1996) Humana Press, Totowa, New Jersey. For example, linear epitopes may be determined by e.g., concurrently synthesizing large numbers of peptides on solid supports, the peptides corresponding to portions of the protein molecule, and reacting the peptides with antibodies while the peptides are still attached to the supports. Such techniques are known in the art and described in, e.g., U.S. Patent No. 4,708,871; Geysen et al. (1984) Proc. Natl. Acad. Sci. USA 81:3998-4002; Geysen et al. (1985) Proc. Natl. Acad. Sci. USA 82:178-182; Geysen et al. (1986) Molec. Immunol. 23:709-715, all incorporated herein by reference in their entireties. Using such techniques, a number of epitopes of HCV have been identified. See, e.g., Chien et al., Viral Hepatitis and Liver Disease (1994) pp. 320-324, and further below. Similarly, conformational epitopes are readily identified by determining spatial conformation of amino acids such as by, e.g., x-ray crystallography and 2-dimensional nuclear magnetic resonance. See, e.g., *Epitope Mapping Protocols, supra.* Antigenic regions of proteins can also be identified using standard antigenicity and hydropathy plots, such as those calculated using, e.g., the Omiga version 1.0 software program available from the Oxford Molecular Group. This computer program employs the Hopp/Woods method, Hopp et al., Proc. Natl. Acad. Sci USA (1981) 78:3824-3828 for determining antigenicity profiles, and the Kyte-Doolittle technique, Kyte et al., J. Mol. Biol. (1982) 157:105-132 for hydropathy plots.

As used herein, the term "conformational epitope" refers to a portion of a full-length protein, or an analog or mutein thereof, having structural features native to the amino acid sequence encoding the epitope within the full-length natural protein. Native structural features include, but are not limited to, glycosylation and three dimensional structure. The length of the epitope-defining sequence can be subject to wide variations as these epitopes are believed to be formed by the three-dimensional shape of the antigen (e.g., folding). Thus, amino acids defining the epitope can be relatively few in number, but widely dispersed along the length of the molecule, being brought into correct epitope conformation via folding. The portions of the antigen between the residues defining the epitope may not be critical to the conformational structure of the epitope. For example, deletion or substitution of these intervening sequences may not affect the conformational epitope provided sequences critical to epitope conformation are maintained (e.g., cysteines involved in disulfide bonding, glycosylation sites, etc.).

Conformational epitopes present in the NS3/4a region are readily identified using methods discussed above. Moreover, the presence or absence of a conformational epitope in a given polypeptide can be readily determined through screening the antigen of interest with an antibody (polyclonal serum or monoclonal to the conformational epitope) and comparing its reactivity to that of a denatured version of the antigen which retains only linear epitopes (if any). In such screening using polyclonal antibodies, it may be advantageous to absorb the polyclonal serum first with the denatured antigen and see if it retains antibodies to the antigen of interest.

Preferably, a conformational epitope is produced recombinantly and is expressed in a cell from which it is extractable under conditions which preserve its desired structural features, e.g. without denaturation of the epitope. Such cells include bacteria, yeast, insect, and mammalian cells. Expression and isolation of recombinant conformational epitopes from the HCV polyprotein are described in e.g., International Publication Nos. WO 96/04301, WO 94/01778, WO 95/33053, WO 92/08734. Alternatively, it is possible to express the antigens and further renature the protein after recovery. It is also understood that chemical synthesis may also provide conformational antigen mimitopes that cross-react with the native antigen's conformational epitope.

The term "multiple epitope fusion antigen" or "MEFA" as used herein intends a polypeptide in which multiple viral antigens are arranged as a single, continuous chain of amino acids, which chain does not occur in nature. As used herein, the MEFAs are limited to HCV antigens. The HCV antigens may be connected directly to each other by peptide bonds or may be separated by intervening amino acid sequences. The fusion antigens may also contain sequences exogenous to the HCV polyprotein. Moreover, the HCV sequences present may be from multiple genotypes and/or isolates of HCV. Examples of particular MEFAs for use in the present immunoassays are detailed in, e.g., International Publication No. WO 97/44469; U.S. Patent Nos. 6,514,731, 6,428,792 and 6,632,601 .

An "antibody" intends a molecule that specifically binds to an epitope of interest present in an antigen. By "specifically binds" is meant that the antibody recognizes and interacts with the epitope in a "lock and key" type of interaction to form a complex between the antigen and antibody, as opposed to non-specific binding that might occur between the antibody and, for instance, the test substrate. Thus, for example, an HCV core antibody is a molecule that specifically binds to the HCV core protein. The term "antibody" as used herein includes antibodies obtained from both polyclonal and monoclonal preparations, as well as, the following: hybrid (chimeric) antibody molecules (see, for example, Winter et al. (1991) Nature 349:293-299; and U.S. Patent No. 4,816,567); F(ab')2 and F(ab) fragments; Fv molecules (non-covalent heterodimers, see, for example, Inbar et al. (1972) Proc Natl Acad Sci USA 69:2659-2662; and Ehrlich et al. (1980) Biochem 19:4091-4096); single-chain Fv molecules (sFv) (see, for example, Huston et al. (1988) Proc Natl Acad Sci USA 85:5879-5883); dimeric and trimeric antibody fragment constructs; minibodies (see, e.g., Pack et aL (1992) Biochem 31:1579-1584; Cumber et al. (1992) J Immunology 149B:120-126); humanized antibody molecules (see, for example, Riechmann et al. (1988) Nature 332:323-327; Verhoeyan et al. (1988) Science 239:1534-1536; and U.K. Patent Publication No. GB 2,276,169, published 21 September 1994); and, any functional fragments obtained from such molecules, wherein such fragments retain immunological binding properties of the parent antibody molecule.

As used herein, the term "monoclonal antibody" refers to an antibody composition having a homogeneous antibody population. The term is not limited regarding the species or source of the antibody, nor is it intended to be limited by the manner in which it is made. Thus, the term encompasses antibodies obtained from murine hybridomas, as well as human monoclonal antibodies obtained using human rather than murine hybridomas. See, e.g., Cote, et al. Monclonal Antibodies and Cancer Therapy, Alan R. Liss, 1985, p. 77.

By "isolated" is meant, when referring to a polypeptide, that the indicated molecule is separate and discrete from the whole organism with which the molecule is found in nature or is present in the substantial absence of other biological macromolecules of the same type. The term "isolated" with respect to a polynucleotide is a nucleic acid molecule devoid, in whole or part, of sequences normally associated with it in nature; or a sequence, as it exists in nature, but having heterologous sequences in association therewith; or a molecule disassociated from the chromosome.

By "equivalent antigenic determinant" is meant an antigenic determinant from different sub-species or strains of HCV, such as from strains 1, 2, or 3 of HCV. More specifically, epitopes are known, such as "5-1-1", occurring at approximately positions 1694-1735, numbered relative to the HCV-1 polyprotein sequence (see, Figure 1), and such-epitopes vary between the strains 1, 2, and 3. Thus, the epitope 5-1-1 from the three different strains are equivalent antigenic determinants and therefore are "copies" even though their sequences are not identical. In general the amino acid sequences of equivalent antigenic determinants will have a high degree of sequence homology, e.g., amino acid sequence homology of more than 30%, preferably more than 40%, when the two sequences are aligned.

"Homology" refers to the percent similarity between two polynucleotide or two polypeptide moieties. Two DNA, or two polypeptide sequences are "substantially homologous" to each other when the sequences exhibit at least about 50% , preferably at least about 75%, more preferably at least about 80%-85%, preferably at least about 90%, and most preferably at least about 95%-98% sequence similarity over a defined length of the molecules. As used herein, substantially homologous also refers to sequences showing complete identity to the specified DNA or polypeptide sequence.

In general, "identity" refers to an exact nucleotide-to-nucleotide or amino acid-to-amino acid correspondence of two polynucleotides or polypeptide sequences, respectively. Percent identity can be determined by a direct comparison of the sequence information between two molecules (the reference sequence and a sequence with unknown % identity to the reference sequence) by aligning the sequences, counting the exact number of matches between the two aligned sequences, dividing by the length of the reference sequence, and multiplying the result by 100.

Readily available computer programs can be used to aid in the analysis of homology and identity, such as ALIGN, Dayhoff, M.O. in Atlas of Protein Sequence and Structure M.O. Dayhoff ed., 5 Suppl. 3:353-358, National biomedical Research Foundation, Washington, DC, which adapts the local homology algorithm of Smith and Waterman Advances in Appl. Math. 2:482-489,1981 for peptide analysis. Programs for determining nucleotide sequence homology are available in the Wisconsin Sequence Analysis Package, Version 8 (available from Genetics Computer Group, Madison, WI) for example, the BESTFIT, FASTA and GAP programs, which also rely on the Smith and Waterman algorithm. These programs are readily utilized with the default parameters recommended by the manufacturer and described in the Wisconsin Sequence Analysis Package referred to above. For example, percent homology of a particular nucleotide sequence to a reference sequence can be determined using the homology algorithm of Smith and Waterman with a default scoring table and a gap penalty of six nucleotide positions.

Another method of establishing percent homology in the context of the present invention is to use the MPSRCH package of programs copyrighted by the University of Edinburgh, developed by John F. Collins and Shane S. Sturrok, and distributed by IntelliGenetics, Inc. (Mountain View, CA). From this suite of packages the Smith-Waterman algorithm can be employed where default parameters are used for the scoring table (for example, gap open penalty of 12, gap extension penalty of one, and a gap of six). From the data generated the "Match" value reflects "sequence homology." Other suitable programs for calculating the percent identity or similarity between sequences are generally known in the art, for example, another alignment program is BLAST, used with default parameters. For example, BLASTN and BLASTP can be used using the following default parameters: genetic code = standard; filter = none; strand = both; cutoff = 60; expect = 10; Matrix = BLOSUM62; Descriptions = 50 sequences; sort by = HIGH SCORE; Databases = non-redundant, GenBank + EMBL + DDBJ + PDB + GenBank CDS translations + Swiss protein + Spupdate + PIR. Details of these programs are readily available.

Alternatively, homology can be determined by hybridization of polynucleotides under conditions which form stable duplexes between homologous regions, followed by digestion with single-stranded-specific nuclease(s), and size determination of the digested fragments. DNA sequences that are substantially homologous can be identified in a Southern hybridization experiment under, for example, stringent conditions, as defined for that particular system. Defining appropriate hybridization conditions is within the skill of the art. See, e.g., Sambrook et al., *supra; DNA Cloning, supra; Nucleic Acid Hybridization, supra.*

A "coding sequence" or a sequence which "encodes" a selected polypeptide, is a nucleic acid molecule which is transcribed (in the case of DNA) and translated (in the case of mRNA) into a polypeptide *in vitro* or *in vivo* when placed under the control of appropriate regulatory sequences. The boundaries of the coding sequence are determined by a start codon at the 5' (amino) terminus and a translation stop codon at the 3' (carboxy) terminus. A transcription termination sequence may be located 3' to the coding sequence.

"Operably linked" refers to an arrangement of elements wherein the components so described are configured so as to perform their desired function. Thus, a given promoter operably linked to a coding sequence is capable of effecting the expression of the coding sequence when the proper transcription factors, etc., are present. The promoter need not be contiguous with the coding sequence, so long as it functions to direct the expression thereof. Thus, for example, intervening untranslated yet transcribed sequences can be present between the promoter sequence and the coding sequence, as can transcribed introns, and the promoter sequence can still be considered "operably linked" to the coding sequence.

"Recombinant" as used herein to describe a nucleic acid molecule means a polynucleotide of genomic, cDNA, viral, semisynthetic, or synthetic origin which, by virtue of its origin or manipulation is not associated with all or a portion of the polynucleotide with which it is associated in nature. The term "recombinant" as used with respect to a protein or polypeptide means a polypeptide produced by expression of a recombinant polynucleotide. In general, the gene of interest is cloned and then expressed in transformed organisms, as described further below. The host organism expresses the foreign gene to produce the protein under expression conditions.

A "control element" refers to a polynucleotide sequence which aids in the expression of a coding sequence to which it is linked. The term includes promoters, transcription termination sequences, upstream regulatory domains, polyadenylation signals, untranslated regions, including 5'-UTRs and 3'-UTRs and when appropriate, leader sequences and enhancers, which collectively provide for the transcription and translation of a coding sequence in a host cell.

A "promoter" as used herein is a DNA regulatory region capable of binding RNA polymerase in a host cell and initiating transcription of a downstream (3' direction) coding sequence operably linked thereto. For purposes of the present invention, a promoter sequence includes the minimum number of bases or elements necessary to initiate transcription of a gene of interest at levels detectable above background. Within the promoter sequence is a transcription initiation site, as well as protein binding domains (consensus sequences) responsible for the binding of RNA polymerase. Eucaryotic promoters will often, but not always, contain "TATA" boxes and "CAT" boxes.

A control sequence "directs the transcription" of a coding sequence in a cell when RNA polymerase will bind the promoter sequence and transcribe the coding sequence into mRNA, which is then translated into the polypeptide encoded by the coding sequence.

"Expression cassette" or "expression construct" refers to an assembly which is capable of directing the expression of the sequence(s) or gene(s) of interest. The expression cassette includes control elements, as described above, such as a promoter which is operably linked to (so as to direct transcription of) the sequence(s) or gene(s) of interest, and often includes a polyadenylation sequence as well. Within certain embodiments of the invention, the expression cassette described herein may be contained within a plasmid construct. In addition to the components of the expression cassette, the plasmid construct may also include, one or more selectable markers, a signal which allows the plasmid construct to exist as single-stranded DNA (e.g., a M13 origin of replication), at least one multiple cloning site, and a "mammalian" origin of replication (e.g., a SV40 or adenovirus origin of replication).

"Transformation," as used herein, refers to the insertion of an exogenous polynucleotide into a host cell, irrespective of the method used for insertion: for example, transformation by direct uptake, transfection, infection, and the like. For particular methods of transfection, see further below. The exogenous polynucleotide may be maintained as a nonintegrated vector, for example, an episome, or alternatively, may be integrated into the host genome.

A "host cell" is a cell which has been transformed, or is capable of transformation, by an exogenous DNA sequence.

As used herein, a "biological sample" refers to a sample of tissue or fluid isolated from a subject, that commonly includes antibodies produced by the subject. Typical samples that include such antibodies are known in the art and include but not limited to, blood, plasma, serum, fecal matter, urine, bone marrow, bile, spinal fluid, lymph fluid, samples of the skin, secretions of the skin, respiratory, intestinal, and genitourinary tracts, tears, saliva, milk, blood cells, organs, biopsies and also samples of *in vitro* cell culture constituents including but not limited to conditioned media resulting from the growth of cells and tissues in culture medium, e.g., recombinant cells, and cell components.

"Common solid support" intends a single solid matrix to which the HCV polypeptides used in the subject immunoassays are bound covalently or by noncovalent means such as hydrophobic adsorption.

"Immunologically reactive" or "immunoreactive" means that the antigen in question will react specifically with anti-HCV antibodies present in a biological sample from an HCV-infected individual.

"Immunogenic" intends that the antigen in question will elicit an immune reaction when administered to an individual.

"Immune complex" intends the combination formed when an antibody binds to an epitope on an antigen.

As used herein, the terms "label" and "detectable label" refer to a molecule capable of detection, including, but not limited to, radioactive isotopes, fluorescers, chemiluminescers, chromophores, enzymes, enzyme substrates, enzyme cofactors, enzyme inhibitors, chromophores, dyes, metal ions, metal sols, ligands (e.g., biotin, avidin, strepavidin or haptens) and the like. The term "fluorescer" refers to a substance or a portion thereof which is capable of exhibiting fluorescence in the detectable range. Particular examples of labels which may be used under the invention include, but are not limited to, horse radish peroxidase (HRP), fluorescein, FITC, rhodamine, dansyl, umbelliferone, dimethyl acridinium ester (DMAE), Texas red, luminol, NADPH and α-β-galactosidase.

### II. Modes of Carrying out the Invention

Before describing the present invention in detail, it is to be understood that this invention is not limited to particular formulations or process parameters as such may, of course, vary. It is also to be understood that the terminology used herein is for the purpose of describing particular embodiments of the invention only, and is not intended to be limiting.

Although a number of compositions and methods similar or equivalent to those described herein can be used in the practice of the present invention, the preferred materials and methods are described herein.

As noted above, the present invention is based on the discovery that HCV polypeptide mutants with modified NS3 domains, such that proteolytic activity of the NS3 protease is inhibited, can retain conformational epitopes and are therefore useful in immunoassays for detecting the presence of HCV infection. The NS3 mutants are especially useful in diagnostic methods for accurately detecting early HCV infection. The methods can be used to detect HCV infection during the early stages of HCV seroconversion, thereby increasing detection accuracy and reducing the incidence of false results.

In particular, the immunoassays described herein utilize highly immunoreactive, epitopes derived from the NS3/4a region of the HCV polyprotein with mutations that disrupt proteolytic activity but retain conformational epitopes. The modified NS3 polypeptides can be used alone in immunoassays or in combination with other HCV antigens, such as multiple epitope fusion antigens comprising various HCV polypeptides, either from the same or different HCV genotypes and isolates, such as multiple immunodominant epitopes, for example, major linear epitopes of HCV core, E1, E2, NS3, 5-1-1, c100-3 and NS5 sequences. The methods can be conveniently practiced in a single assay, using any of the several assay formats described below, such as but not limited to, assay formats which utilize a solid support to which the HCV antigens are bound.

In order to further an understanding of the invention, a more detailed discussion is provided below regarding modified NS3 polypeptides and HCV fusions (i.e., MEFAs), as well as production of the proteins, and methods of using the proteins.

### HCV Proteins

The genomes of HCV strains contain a single open reading frame of approximately 9,000 to 12,000 nucleotides, which is transcribed into a polyprotein. As shown in Figure 1 and Table 1, an HCV polyprotein, upon cleavage, produces at least ten distinct products, in the following order:
NH₂-Core-E1-E2-p7-NS2-NS3-NS4a-NS4b-NS5a-NS5b-COOH. The core polypeptide occurs at positions 1-191, numbered relative to HCV-1 (see, Choo et al. (1991) Proc. Natl. Acad. Sci. USA 88:2451-2455, for the HCV-1 genome). This polypeptide is further processed to produce an HCV polypeptide with approximately amino acids 1-173. The envelope polypeptides, E1 and E2, occur at about positions 192-383 and 384-746, respectively. The P7 domain is found at about positions 747-809. NS2 is an integral membrane protein with proteolytic activity and is found at about positions 810-1026 of the polyprotein. NS2, in combination with NS3, (found at about positions 1027-1657), cleaves the NS2-NS3 scissile bond which in turn generates the NS3 N-terminus and releases a large polyprotein that includes both serine protease and RNA helicase activities. The NS3 protease, found at about positions 1027-1207, serves to process the remaining polyprotein. The helicase activity is found at about positions 1193-1657. NS3 liberates an NS3 cofactor (NS4a, found at about positions 1658-1711), two proteins (NS4b found at about positions 1712-1972, and NS5a found at about positions 1973-2420), and an RNA-dependent RNA polymerase (NS5b found at about positions 2421-3011). Completion of polyprotein maturation is initiated by autocatalytic cleavage at the NS3-Ns4a junction, catalyzed by the NS3 serine protease.

| Table 1 | |
|---|---|
| Domain | Approximate Boundaries* |
| C (core) | 1-191 |
| E1 | 192-383 |
| E2 | 384-746 |
| P7 | 747-809 |
| NS2 | 810-1026 |
| NS3 | 1027-1657 |
| NS4a | 1658-1711 |
| NS4b | 1712-1972 |
| NS5a | 1973-2420 |
| NS5b | 2421-3011 |

*Numbered relative to HCV-1. See, Choo et al. (1991) *Proc. Natl. Acad. Sci. USA* 88:2451-2455.

The modified NS3 polypeptides of the invention are mutated to inhibit protease activity, such that further cleavage of a polypeptide including the modified NS3 domain, such as an NS3/4a polypeptide, as well as catalytic cleavage of additional HCV proteins used in combination with the modified NS3 polypeptide, is inhibited. The NS3 polypeptide can be modified by deletion of all or a portion of the NS3 protease domain. Alternatively, proteolytic activity can be inhibited by substitution of amino acids within active regions of the protease domain. Finally, additions of amino acids to active regions of the domain, such that the catalytic site is modified, will also serve to inhibit proteolytic activity. Preferably, the modifications made to reduce or eliminate protease activity do not disrupt the conformational epitopes in the native NS3 or NS3/4a proteins.

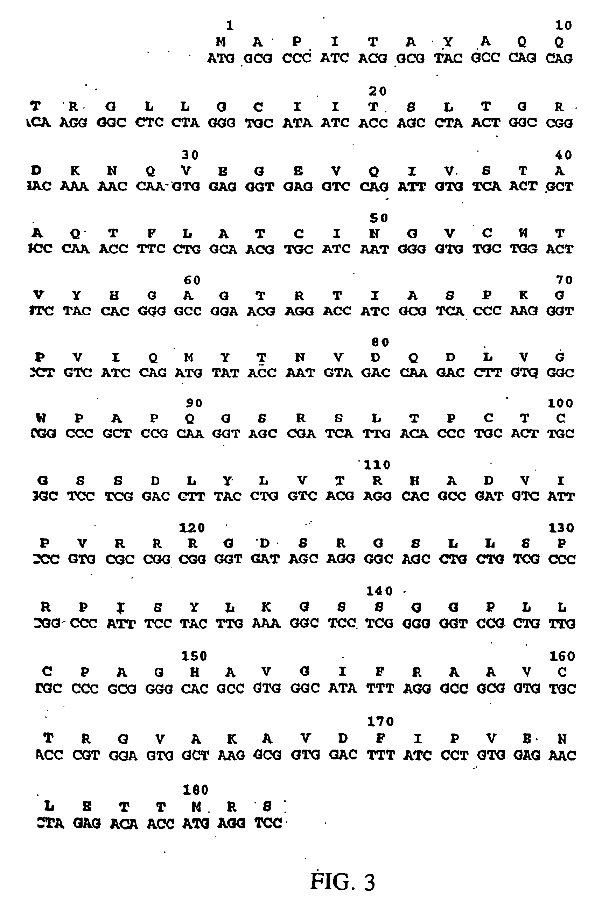

As explained above, the protease activity is found at about amino acid positions 1027-1207, numbered relative to the full-length HCV-1 polyprotein (see, Choo et al., *Proc. Natl. Acad. Sci. USA* (1991) 88:2451-2455), positions 2-182 of Figure 3. The structure of the NS3 protease and active site are known. See, e.g., De Francesco et al., *Antivir. Ther.* (1998) 3:99-109; Koch et al., *Biochemistry* (2001) 40:631-640. Thus, deletions or modifications to the native sequence will typically occur at or near the active site of the molecule. Particularly, it is desirable to modify or make deletions to one or more amino acids occurring at positions 1- or 2-182, preferably 1- or 2-170, or 1- or 2-155 of Figure 3. Preferred modifications are to the catalytic triad at the active site of the protease, i.e., H, D and/or S residues, in order to inactivate the protease. These residues occur at positions 1083, 1105 and 1165, respectively, numbered relative to the full-length HCV polyprotein (positions 58, 80 and 140, respectively, of Figure 3). Such modifications will suppress proteolytic cleavage activity of the NS3 protease while maintaining immunoreactivity. Particularly preferred substitutions are non-conservative in nature, such as a substitution of Ala for one or more of the amino acid residues normally found at positions 1083, 1105 and 1165 of the protease domain. One of skill in the art can readily determine portions of the NS3 protease to delete in order to disrupt activity.

Moreover, other appropriate amino acid modifications at these sites can be readily determined by one of skill in the art based on the known structure and function of the HCV NS3 protease as described in e.g., De Francesco et al., Antivir. Ther. (1998) 3 (Suppl 3):99-109; and Schechter and Berger, Biochim. Biophys. Res. Commun. (1967) 27:157-162. In particular, it is known that NS3 protease is a serine protease and the proteolytic mechanism is based on nucleophilic attack of the targeted peptidic bond by a serine. In many cases the nucleophilic property of the group is improved by the presence of a histidine, held in a "proton acceptor state" by an aspartate. Aligned side chains of serine, histidine and aspartate build the catalytic triad common to most serine proteases. The active site of serine proteases is shaped as a cleft where the polypeptide substrate binds. Schechter and Berger, Biochim. Biophys. Res. Common. (1967) 27:157-162 labeled amino acid residues from N to C terminus of the polypeptide substrate (Pi, ..., P3, P2, P1, P1', P2', P3', ..., Pj) and their respective binding subsites (Si,..., S3, S2, S1, S1', S2', S3',..., Sj) and found the cleavage is catalyzed between P1 and P1'. The NS3 protease adopts a chymotrypsin-like fold and includes a very long, solvent exposed substrate-binding site, consistent with the requirement for very long peptide substrates (P6-P4'). The NS3 protease has a preference for cysteine residues in the substrate P1 position. Thus, based on the known structure and function as described above and in the art, one of skill in the art can readily determine other amino acid substitutions, additions and deletions that will serve to disrupt the proteolytic activity of NS3 protease.

The presence or absence of NS3 proteolytic activity can be determined using methods known to those of skill in the art. For example, protease activity or lack thereof may be determined using the procedure described below in the examples, as well as using assays well known in the art. See, e.g., Takeshita et al., Anal. Biochem. (1997) 247:242-246; Kakiuchi et al., J. Biochem. (1997) 122:749-755; Sali et al., Biochemistry (1998) 37:3392.3401; Cho et al., J. Virol. Meth. (1998) 72:109-115; Cerretani et al., Anal. Biochem. (1999) 266:192-197; Zhang et al., Anal. Biochem. (1999) 270:268-275; Kakiuchi et al., J. Virol. Meth. (1999) 80:77-84; Fowler et al., J. Biomol. Screen. (2000) 5:153-158; and Kim et al., Anal. Biochem. (2000) 284:42-48.

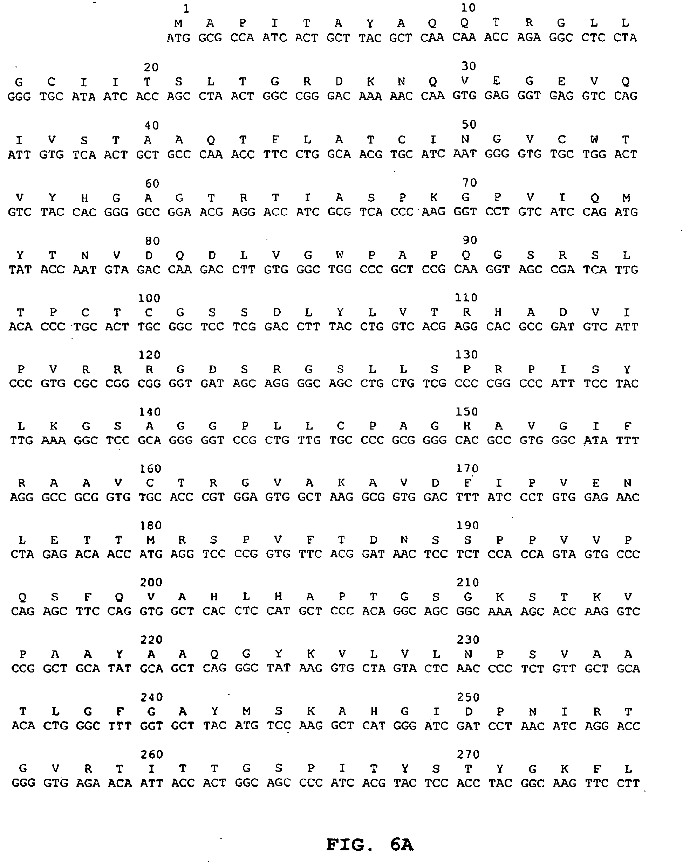

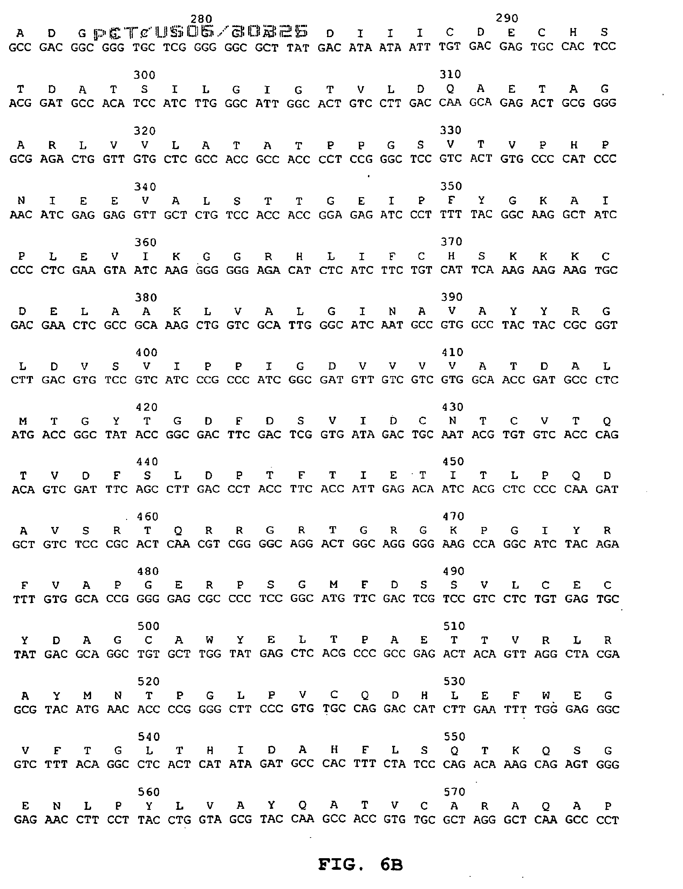

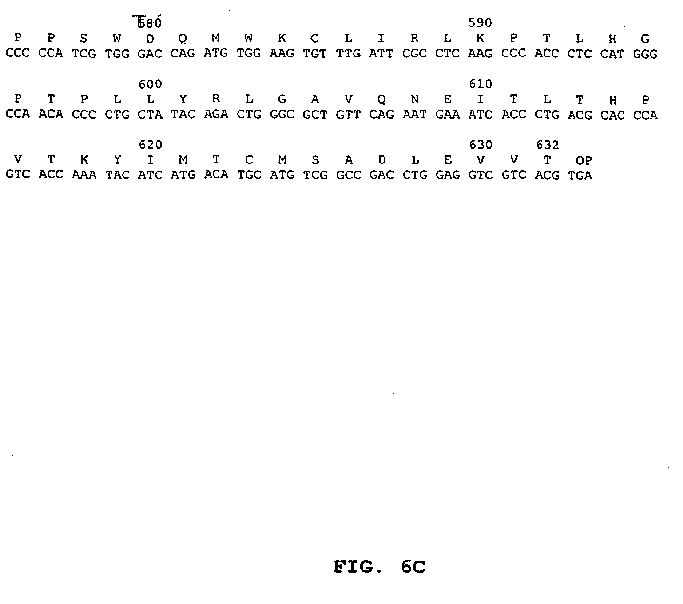

Additional mutations may also, but need not be, present in the NS3 molecule, such as mutations in the helicase domain, for example, substitution of one or both of the amino acid residues found at positions 1428 and 1429, such as Pro for Thr normally present at position 1428 and Ile for Ser normally present at position 1429. These mutations are termed "PI" mutants herein. Figure 4B shows a representative modified NS3 construct. The protein includes an N-terminal Met and amino acids 1027-1657 of NS3 and therefore includes the full-length NS3 domain of the HCV polyprotein. The Ser normally present at position 1165 is substituted with Ala. Moreover, the mutant includes the PI substitution described above. The DNA and amino acid sequences of this construct are depicted in Figures 6A-6C.

The modified NS3 polypeptides of the present invention can also include, in addition to a modified NS3 protease domain, the NS3 helicase domain (that is, the entire NS3 polypeptide sequence, and/or one or more polypeptides from one or more other regions of an HCV polyprotein. Preferably, the modified NS3 polypeptides will include the NS4a polypeptide or a fragment thereof. In fact, all the regions of the HCV polyprotein can be present in such fusions. These polypeptides may be derived from the same HCV isolate as the NS3 polypeptide, or from different strains and isolates including isolates having any of the various HCV genotypes, to provide increased protection against a broad range of HCV genotypes. Additionally, polypeptides can be selected based on the particular viral clades endemic in specific geographic regions where immunodiagnostics will be used. It is readily apparent that the modified HCV proteins provide an effective means of diagnosing HCV infection in a wide variety of contexts.

Nucleic acid and amino acid sequences of a number of HCV strains and isolates, including nucleic acid and amino acid sequences of the various regions of the HCV polyprotein, including Core, NS2, p7, E1, E2, NS3, NS4a, NS4b, NS5a, NS5b genes and polypeptides have been determined. For example, isolate HCV J1.1 is described in Kubo et al. (1989) Japan. Nucl. Acids Res. 17:10367-10372; Takeuchi et al.(1990) Gene 91:287-291; Takeuchi et al. (1990) J. Gen. Virol. 71:3027-3033; and Takeuchi et al. (1990) Nucl. Acids Res. 18:4626. The complete coding sequences of two independent isolates, HCV-J and BK, are described by Kato et al., (1990) Proc. Natl. Acad. Sci. USA 87:9524-9528 and Takamizawa et al., (1991) J. Virol. 65:1105-1113 respectively.

Publications that describe HCV-1 isolates include Choo et al. (1990) Brit. Med. Bull. 46:423-441; Choo et al. (1991) Proc. Natl. Acad. Sci. USA 88:2451-2455 and Han et al. (1991) Proc. Natl. Acad. Sci. USA 88:1711-1715. HCV isolates HC-J1 and HC-J4 are described in Okamoto et al. (1991) Japan J. Exp. Med. 60:167-177. HCV isolates HCT 18~, HCT 23, Th, HCT 27, EC1 and EC10 are described in Weiner et al. (1991) Virol. 180:842-848. HCV isolates Pt-1, HCV-K1 and HCV-K2 are described in Enomoto et al. (1990) Biochem. Biophys. Res. Commun. 170:1021-1025. HCV isolates A, C, D & E are described in Tsukiyama-Kohara et al. (1991) Virus Genes 5:243-254.

Each of the components of a fusion protein can be obtained from the same HCV strain or isolate or from different HCV strains or isolates. For example, the modified NS3 polypeptide can be derived from a first strain of HCV, and other HCV polypeptides present can be derived from a second strain of HCV. Alternatively, one or more of the other HCV polypeptides, for example NS2, NS4a, NS4b, Core, p7, E1 and/or E2, if present, can be derived from a first strain of HCV, and the remaining HCV polypeptides can be derived from a second strain of HCV. Additionally, each of the HCV polypeptides present can be derived from different HCV strains.

In certain embodiments, the modified NS3 protein also comprises an NS4a domain or portion thereof, e.g., an NS4a and/or an NS4b domain, or a fragment thereof. The modified NS3 protein (with or without the NS4a domain) can also be fused with other HCV regions, such as with an NS5a domain, a core polypeptide of an HCV, and the like. These regions need not be in the order in which they naturally occur in the native HCV polyprotein. Thus, for example, if present, the NS4a polypeptide may be fused to the N- and/or C-terminus of the NS3 polypeptide.

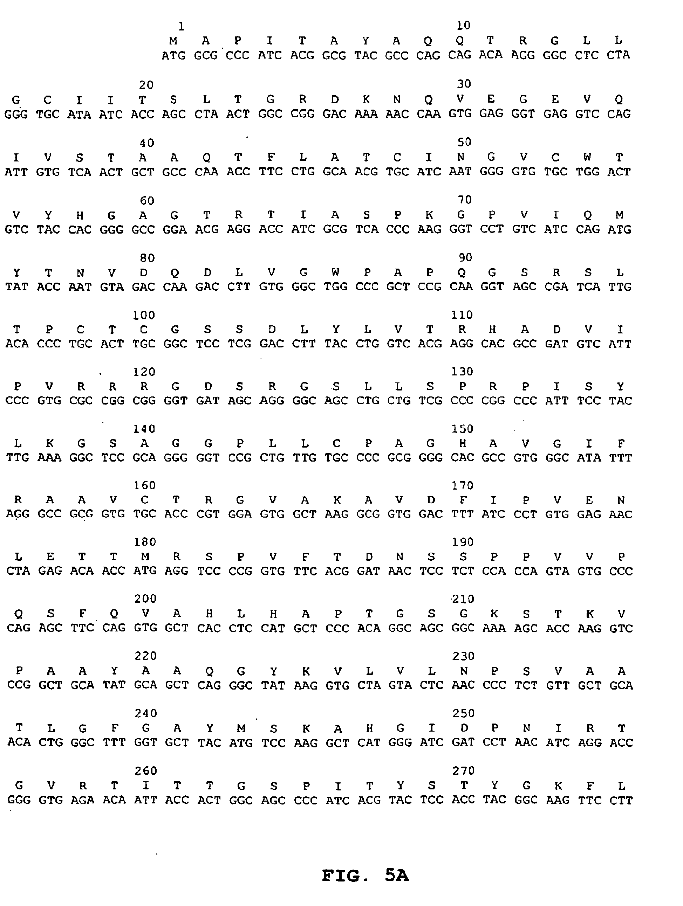

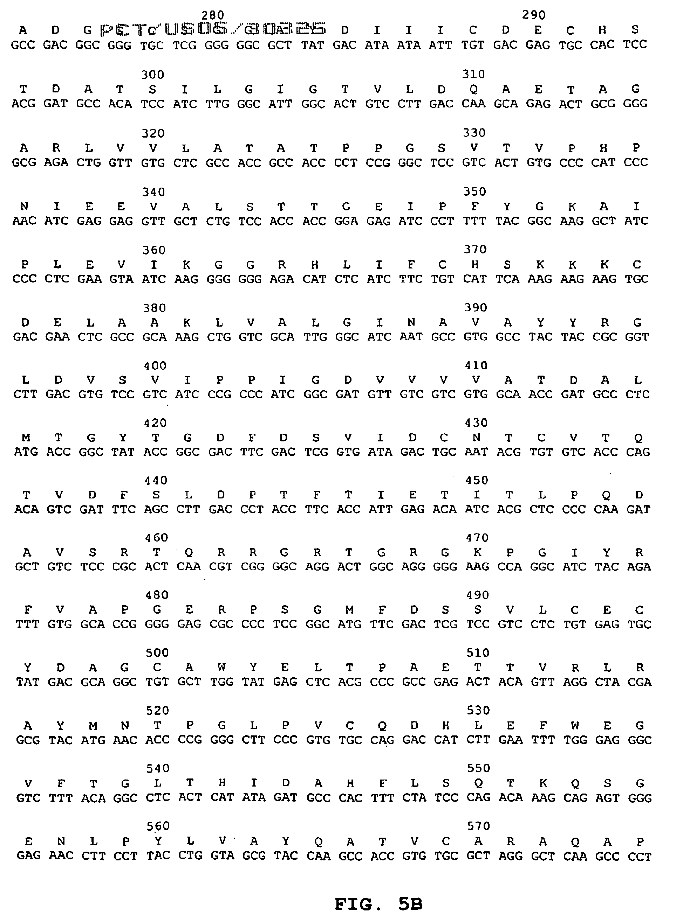

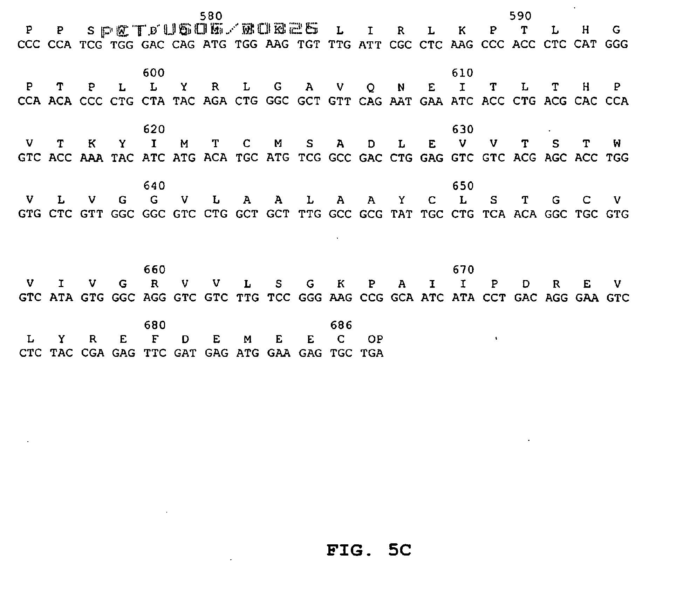

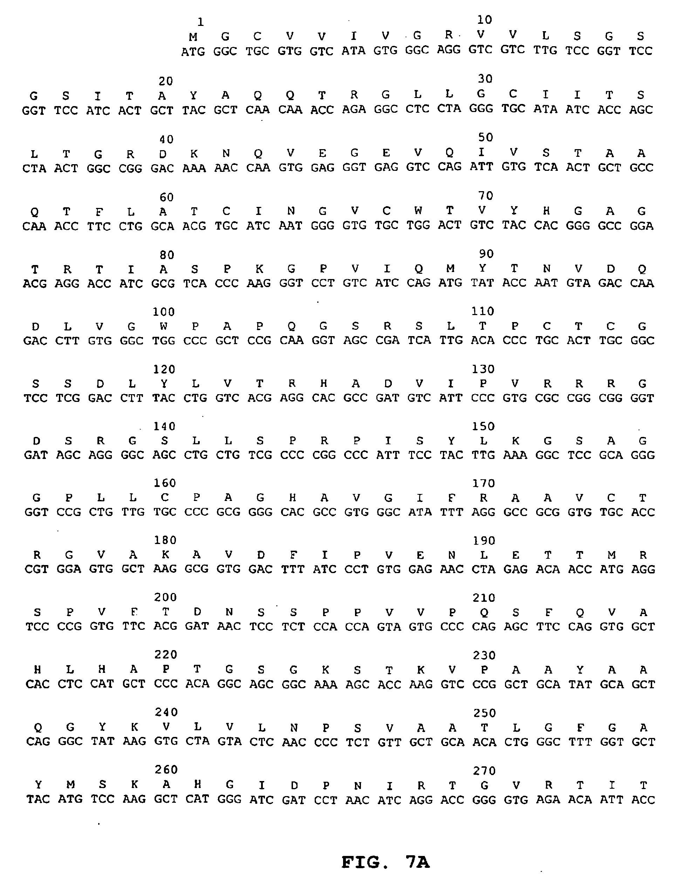

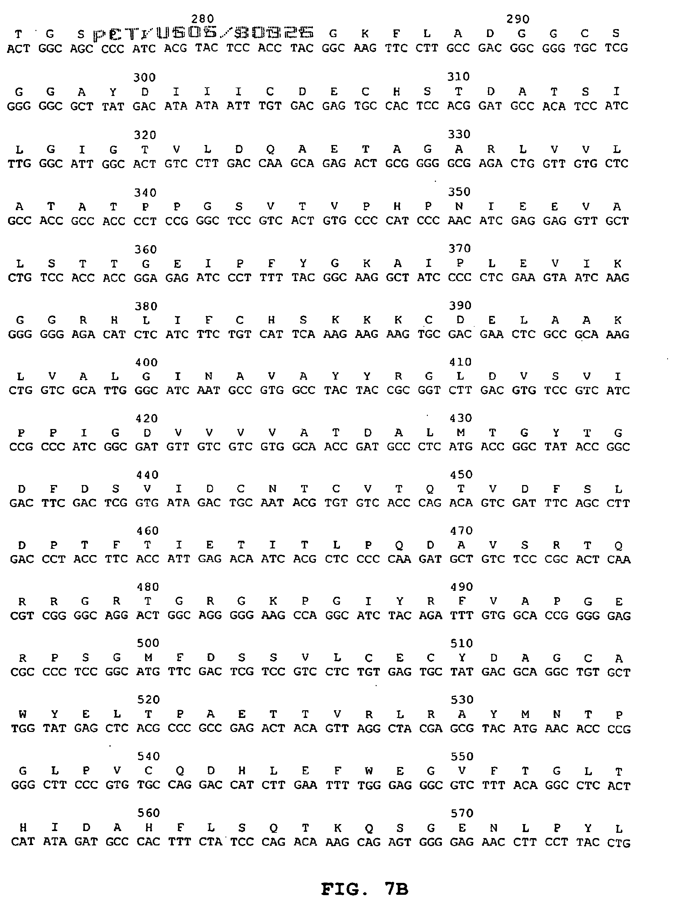

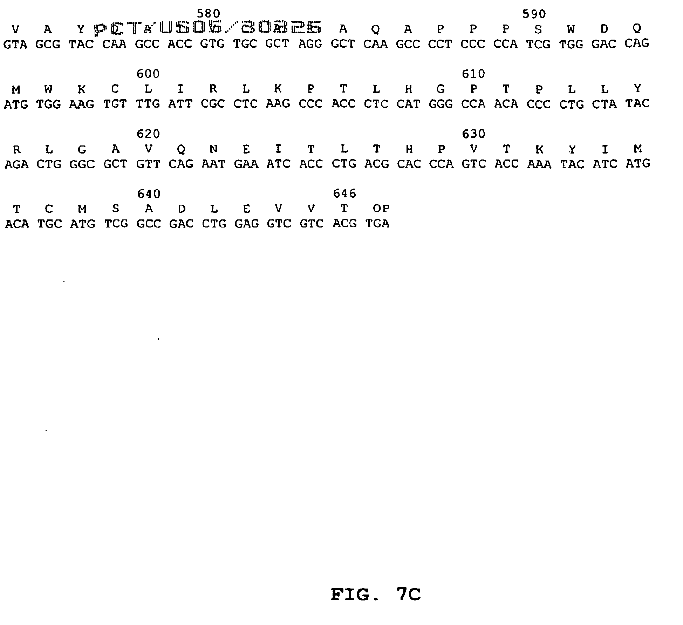

Figures 4A and 4C show representative modified NS3 polypeptides. In the figures, the Ser normally present at position 1165 is substituted with Ala. Additionally, the mutants include the PI substitution described above, i.e., a substitution of Pro for Thr normally present at position 1428, and Ile for Ser normally present at position 429. In Figure 4A, the protein includes in N-terminal to C-terminal direction, an N-terminal Met and amino acids 1027-1657 of NS3 and 1658-1711 of NS4a. This fusion therefore includes the full-length NS3 and NS4a domains of the HCV polyprotein. The nucleotide sequence and corresponding amino acid sequence of this protein is shown in Figures 5A-5C. In Figure 4C, the protein includes in N-terminal to C-terminal direction, an N-terminal Met and amino acids 1678-1690 of NS4a, followed by the amino acid sequence SGS, then amino acids 1029 to 1657 of NS3. Thus, this fusion includes 13 amino acids of the NS4a domain, followed by a tripeptide flexible linker sequence (termed a "turn" sequence) and then almost the entire NS3 domain (except the first two N-terminal amino acids). The turn sequence allows the NS4a to fold into the pocket within the protease. The nucleotide sequence and corresponding amino acid sequence of this protein is shown in Figures 7A-7C. The numbering herein is with reference to the full-length HCV-1 polyprotein and equivalent regions in other HCV serotypes is readily determined.

The modified NS3 polypeptides described above may be used in immunoassays with multiple epitope fusion antigens (termed "MEFAs"), as described in International Publication No. WO 97/44469 and U.S. Patent Nos. 6,514,731 and 6,428,792. Such MEFAs include multiple epitopes derived from two or more of the various viral regions of the HCV polyprotein shown in Figure 1 and Table 1 and described in detail above. The use of such modified NS3 polypeptides with the MEFAs assures that NS3 proteolytic cleavage of the MEFA will not occur, thus providing better reagents for use in HCV immunoassays.

The multiple HCV antigens are arranged as a single, continuous chain of amino acids, which chain does not occur in nature. Thus, the linear order of the epitopes is different than their linear order in the genome in which they occur. The linear order of the sequences of the MEFAs for use herein is preferably arranged for optimum antigenicity. Preferably, the epitopes are from more than one HCV strain, thus providing the added ability to detect multiple strains of HCV in a single assay. Thus, the MEFAs for use herein may comprise various immunogenic regions derived from the polyprotein described above. Moreover, a protein resulting from a frameshift in the core region of the polyprotein, such as described in International Publication No. WO 99/63941, may be used in the MEFAs or fusions with the modified NS3 described above. If desired, at least 2, 3, 4, 5, 6, 7, 8, 9, or 10 or more of one or more epitopes derived from the HCV polyprotein may occur in the fusion protein.

Additional HCV epitopes for use in the MEFAs include epitopes derived from the hypervariable region of E2, such as a region spanning amino acids 384-414 or 390-410, or the consensus sequence from this region, Gly-Ser-Ala-Ala-Arg-Thr-Thr-Ser-Gly-Phe-Val-Ser-Leu-Phe-Ala-Pro-Gly-Ala-Lys-Gln-Asn (SEQ ID NO:11), which represents a consensus sequence for amino acids 390-410 of the HCV type 1 genome. A representative E2 epitope present in a MEFA of the invention can comprise a hybrid epitope spanning amino acids 384-414. Such a hybrid E2 epitope can include a consensus sequence representing amino acids 390-410 fused to a consensus sequence from a second strain. Alternatively, the E2 epitope present may comprise a hybrid epitope spanning amino acids 390-444. Such a hybrid E2 epitope can include a consensus sequence representing amino acids 390-410 as detailed above, fused to e.g., the native amino acid sequence for amino acids 411-444 of HCV E2.

Additionally, the antigens may be derived from various HCV strains. Multiple viral strains of HCV are known, and epitopes derived from any of these strains can be used in a fusion protein. It is well known that any given species of organism varies from one individual organism to another and further that a given organism such as a virus can have a number of different strains. For example, as explained above, HCV includes at least 6 genotypes. Each of these genotypes includes equivalent antigenic determinants. More specifically, each strain includes a number of antigenic determinants that are present on all strains of the virus but are slightly different from one viral strain to another. For example, HCV includes the antigenic determinant known as 5-1-1 (See, Figure 1). The 5-1-1 epitope is found at approximately positions 1694-1735, numbered relative to the HCV-1 polyprotein sequence. This particular antigenic determinant appears in three different forms in HCV-1, HCV-2 and HCV-3. Accordingly, in a preferred embodiment of the invention all three of these forms of 5-1-1 appear on the multiple epitope fusion antigen used in the subject immunoassays. Similarly, equivalent antigenic determinants from the core region of different HCV strains may also be present. In general, equivalent antigenic determinants have a high degree of homology in terms of amino acid sequence which degree of homology is generally 30% or more, preferably 40% or more, when aligned. The multiple copy epitope of the present invention can also include multiple copies which are exact copies of the same epitope.

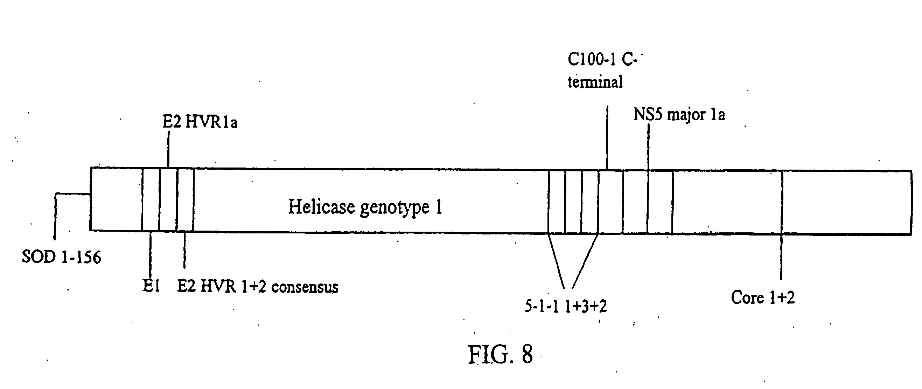

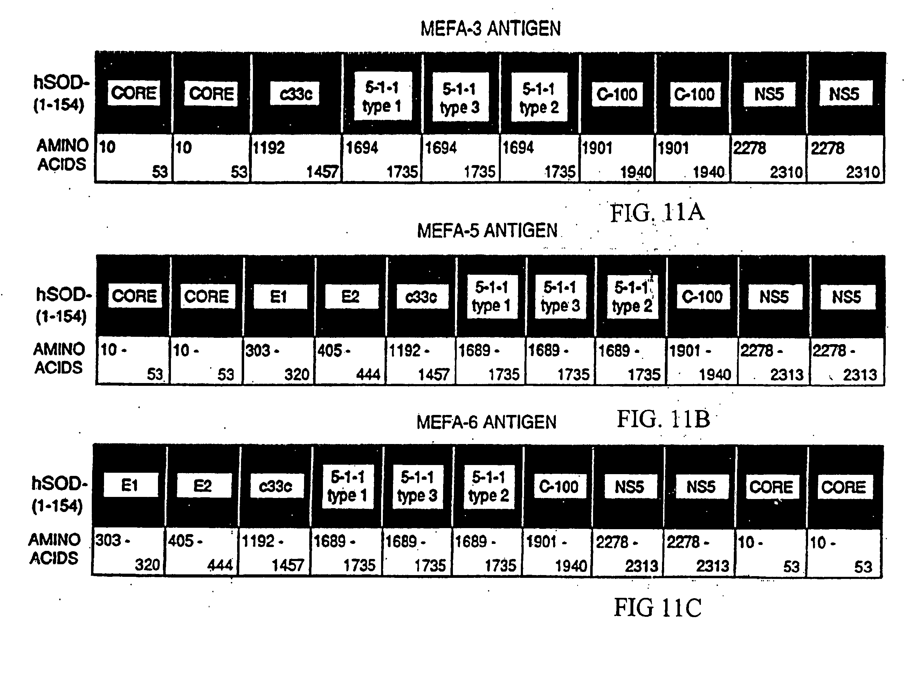

Figures 8 and 11A-11C show representative MEFAs for use in the present invention which are derived from HCV. However, it is to be understood that other epitopes derived from the HCV genome will also find use with the present assays.

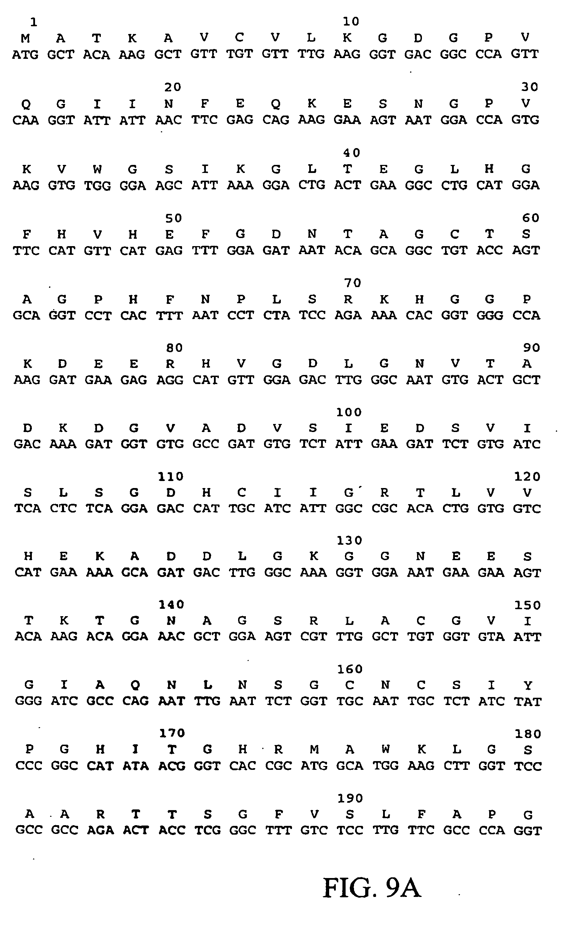

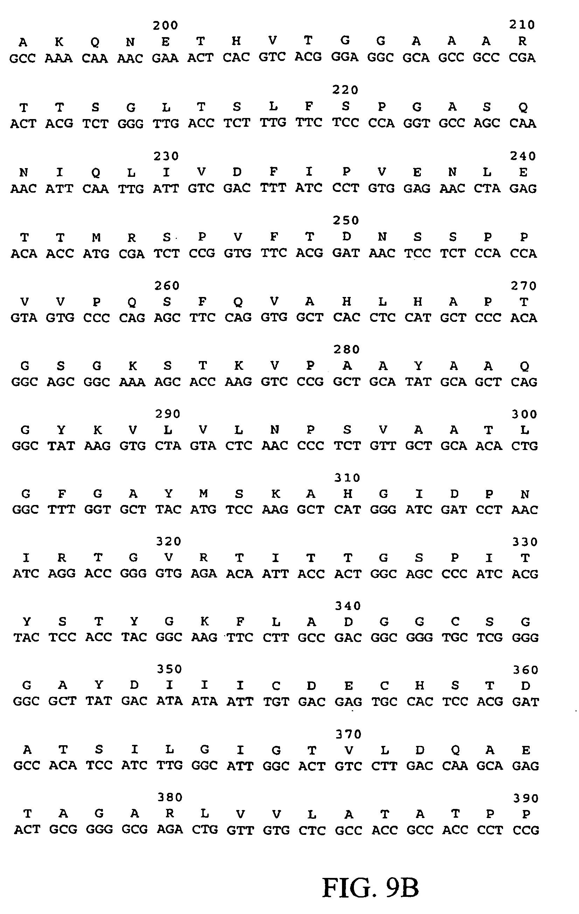

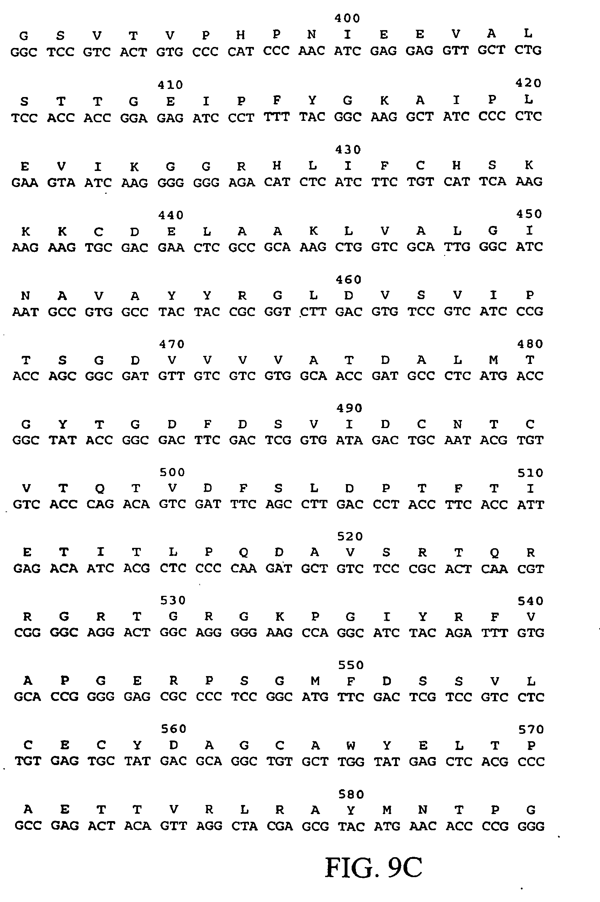

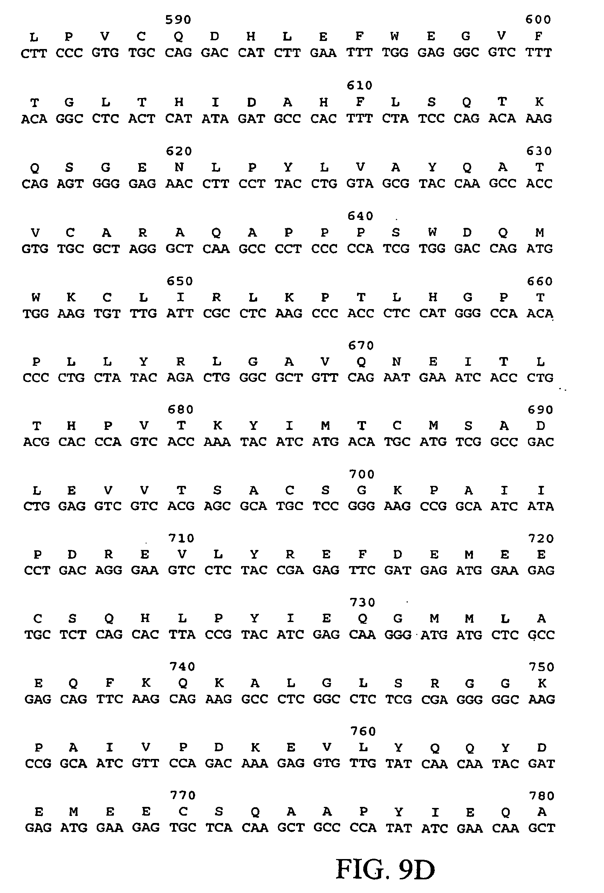

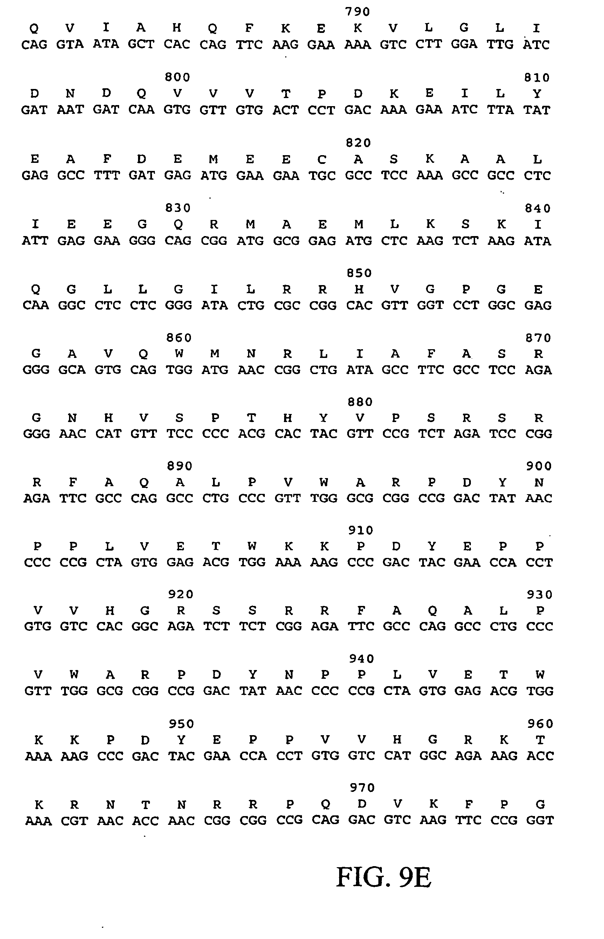

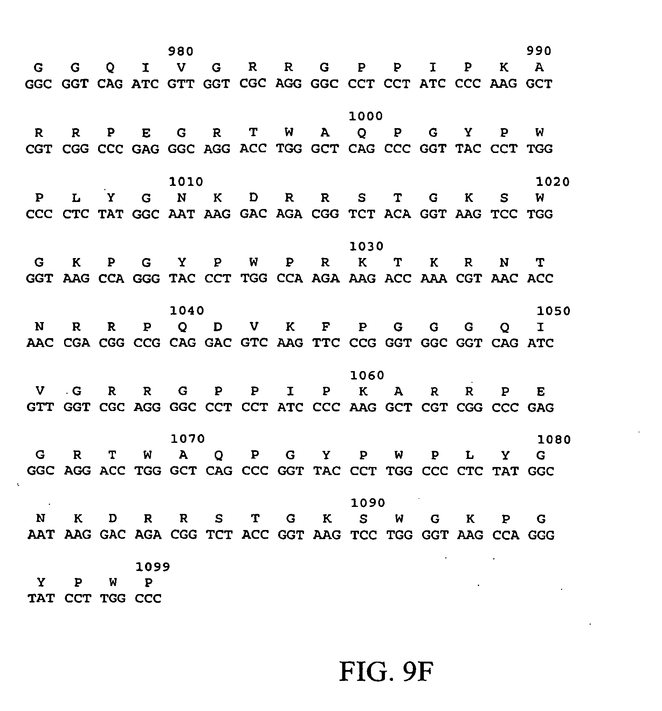

The DNA sequence and corresponding amino acid sequence of a representative multiple epitope fusion antigen, MEFA 7.1, is shown in Figures 9A-9F. This MEFA is also described in U.S. Patent No. 6,632,601. The general structural formula for MEFA 7.1 is shown in Figure 8 and is as follows: hSOD-E1(type 1)-E2 HVR consensus(type 1a)-E2 HVR consensus(types 1 and 2)-helicase(type 1)-5-1-1(type 1)-5-1-1(type 3)-5-1-1(type 2)-c100(type 1)-NS5(type 1)-NS5(type 1)-core(types 1+2)-core(types 1+2). This multiple copy epitope includes the following amino acid sequence, numbered relative to HCV-1 (the numbering of the amino acids set forth below follows the numbering designation provided in Choo, et al. (1991) Proc. Natl. Acad. Sci. USA 88:2451-2455, in which amino acid #1 is the first methionine encoded by the coding sequence of the core region): amino acids 1-156 of superoxide dismutase (SOD, used to enhance recombinant expression of the protein); amino acids 303 to 320 of the polyprotein from the E1 region; amino acids 390 to 410 of the polyprotein, representing a consensus sequence for the hypervariable region of HCV-1a E2; amino acids 384 to 414 of the polyprotein from region E2, representing a consensus sequence for the E2 hypervariable regions of HCV-1 and HCV-2; amino acids 1193-1658 of the HCV-1 polyprotein which define the helicase; three copies of an epitope from 5-1-1, amino acids 1689-1735, one from HCV-1, one from HCV-3 and one from HCV-2, which copies are equivalent antigenic determinants from the three different viral strains of HCV; HCV polypeptide C100 of HCV-1, amino acids 1901-1936 of the polyprotein; two exact copies of an epitope from the NS5 region of HCV-1, each with amino acids 2278 to 2313 of the HCV polyprotein; and two copies of an epitope from the core region, one from HCV-1 and one from HCV-2, which copies are equivalent antigenic determinants represented by amino acids 9 to 32, 39-42 and 64-88 of HCV-1 and 67-84 of HCV-2.

Table 2 shows the amino acid positions of the various epitopes with reference to Figures 9A-9F herein.

| Table 2. MEFA 7.1 | | | | |
|---|---|---|---|---|
| mefa aa# | 5' end site | epitope | hcv aa# | strain |
| 1-156 | *Nco*1 | hSOD | | |
| 159-176 | *Eco*R1 | E1 | 303-320 | 1 |
| 179-199 | *Hind*111 | E2 HVR1a consensus | 390-410 | 1 |
| 200-230 | | E2 HVR1+2 consensus | 384-414 | 1+2 |
| 231-696 | *Sal*1 | Helicase | 1193-1658 | 1 |
| 699-745 | *Sph*1 | 5-1-1 | 1689-1735 | 1 |
| 748-794 | *Nru*1 | 5-1-1 | 1689-1735 | 3 |
| 797-843 | *Cla*1 | 5-1-1 | 1689-1735 | 2 |
| 846-881 | *Ava*1 | C100 | 1901-1936 | 1 |
| 884-919 | *Xba*1 | NS5 | 2278-2313 | 1 |
| 922-957 | *Bgl*11 | NS5 | 2278-2313 | 1 |
| 958-1028 | *Nco*1 | core | 9-32, 39-42 | 1 |
| | | epitopes | 64-88 | 1 |
| | | | 67-84 | 2 |
| 1029-1099 | *Bal*1 | core | 9-32, 39-42, | 1 |
| | | epitopes | 64-88 | 1 |
| | | | 67-84 | 2 |

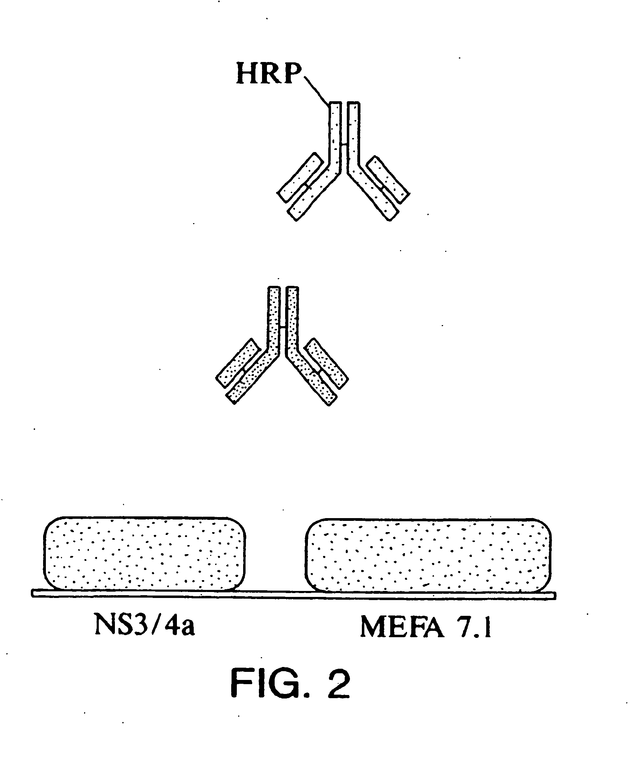

In one embodiment of the invention, depicted in Figure 2, a rapid capture ligand immunoassay is performed using a modified NS3 polypeptide, or fusion including a modified NS3 polypeptide, and one or more multiple epitope fusion antigens, such as MEFA 7.1. The sample is combined with the antigens, which may be present on a solid support, as described further below. If the sample is infected with HCV, anti HCV antibodies to those epitopes present on the solid support will bind to the solid support components. Detection is by the attachment of a detectable marker (such as horse radish peroxidase (HRP) as shown in Figure 2) to a member of the antigen/antibody complex. Attachment may be by covalent means or by subsequent binding of detectably labeled antibodies, such as in a standard sandwich assay, or by enzyme reaction, the product of which reaction is detectable. The detectable marker may include, but is not limited to, a chromophore, an antibody, an antigen, an enzyme, an enzyme reactive compound whose cleavage product is detectable, rhodamine or rhodamine derivative, biotin, avidin, strepavidin, a fluorescent compound, a chemiluminescent compound, such as dimethyl acridinium ester (DMAE), derivatives and/or combinations of these markers. A detectably labeled anti-human antibody, capable of detecting a human IgG molecule present, can be conveniently used.

### Production of HCV Antigens

As explained above, the molecules of the present invention are generally produced recombinantly. Thus, polynucleotides encoding HCV antigens for use with the present invention can be made using standard techniques of molecular biology. For example, polynucleotide sequences coding for the above-described molecules can be obtained using recombinant methods, such as by screening cDNA and genomic libraries from cells expressing the gene, or by deriving the gene from a vector known to include the same. Furthermore, the desired gene can be isolated directly from viral nucleic acid molecules, using techniques described in the art, such as in Houghton et al., U.S. Patent No. 5,350,671. The gene of interest can also be produced synthetically, rather than cloned. The molecules can be designed with appropriate codons for the particular sequence. The complete sequence is then assembled from overlapping oligonucleotides prepared by standard methods and assembled into a complete coding sequence. See, e.g., Edge (1981) Nature 292:756; Nambair et al. (1984) Science 223:1299; and Jay et al. (1984) J. Biol. Chem. 259:6311.

Thus, particular nucleotide sequences can be obtained from vectors harboring the desired sequences or synthesized completely or in part using various oligonucleotide synthesis techniques known in the art, such as site-directed mutagenesis and polymerase chain reaction (PCR) techniques where appropriate. See, e.g., Sambrook, *supra.* In particular, one method of obtaining nucleotide sequences encoding the desired sequences is by annealing complementary sets of overlapping synthetic oligonucleotides produced in a conventional, automated polynucleotide synthesizer, followed by ligation with an appropriate DNA ligase and amplification of the ligated nucleotide sequence via PVR. See, e.g., Jayaraman et al. (1991) Proc. Natl. Acad. Sci. USA 88:4084-4088. Additionally, oligonucleotide directed synthesis (Jones et al. (1986) Nature 54:75-82), oligonucleotide directed mutagenesis of pre-existing nucleotide regions (Riechmann et al. (1988) Nature 332:323-327 and Verhoeyen et al. (1988) Science 239:1534-1536), and enzymatic filling-in of gapped oligonucleotides using T₄ DNA polymerase (Queen et al. (1989) Proc. Natl. Acad. Sci. USA 86:10029-10033) can be used under the invention to provide molecules having altered or enhanced antigen-binding capabilities, and/or reduced immunogenicity.

Methods for producing mutants or analogs of the desired nucleotide sequence, such as NS3, or other HCV antigens, are well known. See, e.g., Dasmahapatra et al., U.S. Patent No. 5,843,752 and Zhang et al., U.S. Patent No. 5,990,276. Mutants or analogs of NS3 and other HCV proteins for use in immunoassays may be prepared by deletion of a portion of the sequence encoding the polypeptide of interest, by insertion of a sequence, and/or by substitution of one or more nucleotides within the sequence. Techniques for modifying nucleotide sequences, such as site-directed mutagenesis, and the like, are well known to those skilled in the art. See, e.g., Sambrook et al., supra; Kunkel, T.A. (1985) Proc. Natl. Acad. Sci. USA (1985) 82:448; Geisselsoder et al. (1987) BioTechniques 5:786; Zoller and Smith (1983) Methods Enzymol. 100:468; Dalbie-McFarland et al. (1982) Proc. Natl. Acad. Sci USA 79:6409.

Once coding sequences have been prepared or isolated, such sequences can be cloned into any suitable vector or replicon. Numerous cloning vectors are known to those of skill in the art, and the selection of an appropriate cloning vector is a matter of choice. Suitable vectors include, but are not limited to, plasmids, phages, transposons, cosmids, chromosomes or viruses which are capable of replication when associated with the proper control elements.

The coding sequence is then placed under the control of suitable control elements, depending on the system to be used for expression. Thus, the coding sequence can be placed under the control of a promoter, ribosome binding site (for bacterial expression) and, optionally, an operator, so that the DNA sequence of interest is transcribed into RNA by a suitable transformant. The coding sequence may or may not contain a signal peptide or leader sequence which can later be removed by the host in post-translational processing. See, e.g., U.S. Patent Nos. 4,431,739; 4,425,437; 4,338,397.

In addition to control sequences, it may be desirable to add regulatory sequences which allow for regulation of the expression of the sequences relative to the growth of the host cell. Regulatory sequences are known to those of skill in the art, and examples include those which cause the expression of a gene to be turned on or off in response to a chemical or physical stimulus, including the presence of a regulatory compound. Other types of regulatory elements may also be present in the vector. For example, enhancer elements may be used herein to increase expression levels of the constructs. Examples include the SV40 early gene enhancer (Dijkema et al. (1985) EMBO J. 4:761), the enhancer/promoter derived from the long terminal repeat (LTR) of the Rous Sarcoma Virus (Gorman et al. (1982) Proc. Natl. Acad. Sci. USA 79:6777) and elements derived from human CMV (Boshart et al. (1985) Cell 41:521), such as elements included in the CMV intron A sequence (U.S. Patent No. 5,688,688). The expression cassette may further include an origin of replication for autonomous replication in a suitable host cell, one or more selectable markers, one or more restriction sites, a potential for high copy number and a strong promoter.

An expression vector is constructed so that the particular coding sequence is located in the vector with the appropriate regulatory sequences, the positioning and orientation of the coding sequence with respect to the control sequences being such that the coding sequence is transcribed under the "control" of the control sequences (i.e., RNA polymerase which binds to the DNA molecule at the control sequences transcribes the coding sequence). Modification of the sequences encoding the molecule of interest may be desirable to achieve this end. For example, in some cases it may be necessary to modify the sequence so that it can be attached to the control sequences in the appropriate orientation; i.e., to maintain the reading frame. The control sequences and other regulatory sequences may be ligated to the coding sequence prior to insertion into a vector. Alternatively, the coding sequence can be cloned directly into an expression vector which already contains the control sequences and an appropriate restriction site.

The molecules can be expressed in a wide variety of systems, including insect, mammalian, bacterial, viral and yeast expression systems, all well known in the art. For example, insect cell expression systems, such as baculovirus systems, are known to those of skill in the art and described in, e.g., Summers and Smith, Texas Agricultural Experiment Station Bulletin No. 1555 (1987). Materials and methods for baculovirus/insect cell expression systems are commercially available in kit form from, *inter alia,* Invitrogen, San Diego CA ("MaxBac" kit). Similarly, bacterial and mammalian cell expression systems are well known in the art and described in, e.g., Sambrook et al., *supra.* Yeast expression systems are also known in the art and described in, e.g., Yeast Genetic Engineering (Barr et al., eds., 1989) Butterworths, London.

A number of appropriate host cells for use with the above systems are also known. For example, mammalian cell lines are known in the art and include immortalized cell lines available from the American Type Culture Collection (ATCC), such as, but not limited to, Chinese hamster ovary (CHO) cells, HeLa cells, baby hamster kidney (BHK) cells, monkey kidney cells (COS), human embryonic kidney cells, human hepatocellular carcinoma cells (e.g., Hep G2), Madin-Darby bovine kidney ("MDBK") cells, as well as others. Similarly, bacterial hosts such as *E*. *coli, Bacillus subtilis,* and *Streptococcus spp.,* will find use with the present expression constructs. Yeast hosts useful in the present invention include *inter alia, Saccharomyces cerevisiae, Candida albicans, Candida maltosa, Hansenula polymorpha, Kluyveromyces fragilis, Kluyveromyces lactis, Pichia guillerimondii, Pichia pastoris, Schizosaccharomyces pombe* and *Yarrowia lipolytica.* Insect cells for use with baculovirus expression vectors include, *inter alia, Aedes aegypti, Autographa californica, Bombyx mori, Drosophila melanogaster, Spodoptera frugiperda,* and *Trichoplusia ni.*

Nucleic acid molecules comprising nucleotide sequences of interest can be stably integrated into a host cell genome or maintained on a stable episomal element in a suitable host cell using various gene delivery techniques well known in the art. *See, e.g.,* U.S. Patent No. 5,399,346.

Depending on the expression system and host selected, the molecules are produced by growing host cells transformed by an expression vector described above under conditions whereby the protein is expressed. The expressed protein is then isolated from the host cells and purified. If the expression system secretes the protein into growth media, the product can be purified directly from the media. If it is not secreted, it can be isolated from cell lysates. The selection of the appropriate growth conditions and recovery methods are within the skill of the art.

The recombinant production of various HCV antigens, including antigens used in the various fusions described above, has been described. See, e.g., International Publication Nos. WO 94/01778, WO 93/00365, WO 04/00547 and WO 01/38360; U.S. Patent Nos. 5,350,671, 5,683,864, 6,346,375, 6,150,087, 6,514,731,6,428,792 and 6,632,601; Chien et al., J. Gastroent. Hepatol. (1993) 8:S33-39; Chien, D.Y., International Publication No. WO 94/01778; Chien et al., Proc. Natl. Acad. Sci. USA (1992) 89:10011-10015. A preferred method of producing modified NS3 polypeptides is described in Example 1.

### Immunodiagnostic Assays

Once produced, the HCV antigens may be used in virtually any assay format that employs a known antigen to detect antibodies. A common feature of all of these assays is that the antigen is contacted with the body component suspected of containing anti- HCV antibodies under conditions that permit the antigen to bind to any such antibodies present in the component. Such conditions will typically be physiologic temperature, pH and ionic strength using an excess of antigen. The incubation of the antigen with the specimen is followed by detection of immune complexes comprised of the antigen.

Design of the immunoassays is subject to a great deal of variation, and many formats are known in the art. Protocols may, for example, use solid supports, or immunoprecipitation. Most assays involve the use of labeled antibody or polypeptide; the labels may be, for example, enzymatic, fluorescent, chemiluminescent, radioactive, or dye molecules, as discussed in detail above. Assays which amplify the signals from the immune complex are also known; examples of which are assays which utilize biotin and avidin, and enzyme-labeled and mediated immunoassays, such as ELISA assays.

The immunoassay may be, without limitation, a heterogenous or a homogeneous format, and of a standard or competitive type. In a heterogeneous format, the polypeptide is typically bound to a solid matrix or support to facilitate separation of the sample from the polypeptide after incubation. A solid support, for the purposes of this invention, can be any material that is an insoluble matrix and can have a rigid or semi-rigid surface. Exemplary solid supports include, but are not limited to, substrates such as nitrocellulose (e.g., in membrane or microtiter well form); polyvinylchloride (e.g., sheets or microtiter wells); polystyrene latex (e.g., beads or microtiter plates); polyvinylidine fluoride; diazotized paper; nylon membranes; activated beads, magnetically responsive beads, and the like. Particular supports include plates, pellets, disks, capillaries, hollow fibers, needles, pins, solid fibers, cellulose beads, pore-glass beads, silica gels, polystyrene beads optionally cross-linked with divinylbenzene, grafted co-poly beads, polyacrylamide beads, latex beads, dimethylacrylamide beads optionally crosslinked with N-N'-bis-acryloylethylenediamine, and glass particles coated with a hydrophobic polymer.

If desired, the molecules to be added to the solid support can readily be functionalized to create styrene or acrylate moieties, thus enabling the incorporation of the molecules into polystyrene, polyacrylate or other polymers such as polyimide, polyacrylamide, polyethylene, polyvinyl, polydiacetylene, polyphenylene-vinylene, polypeptide, polysaccharide, polysulfone, polypyrrole, polyimidazole, polythiophene, polyether, epoxies, silica glass, silica gel, siloxane, polyphosphate, hydrogel, agarose, cellulose, and the like.

If more than one HCV antigen is used in the assays, for example, a modified NS3 polypeptide and a MEFA or another HCV antigen, the antigens can be provided on the same solid substrate or on different solid substrates that are combined in the assay. Thus, for example, the antigens can be present as discrete entities on, e.g., a plate, or can be present on, for example, individual microbeads that are added together for use in the assay of interest.

In one context, as depicted in Figure 2, a solid support is first reacted with the HCV antigens such as a modified NS3 polypeptide, e.g., NS3aPI.1165, NS3PI.1165 or d.4a.t.NS3PI.1165, and a MEFA, such as MEFA 7.1 (collectively called "the solid-phase components" herein), under suitable binding conditions such that the molecules are sufficiently immobilized to the support. Sometimes, immobilization to the support can be enhanced by first coupling the antigen to a protein with better solid phase-binding properties. Suitable coupling proteins include, but are not limited to, macromolecules such as serum albumins including bovine serum albumin (BSA), keyhole limpet hemocyanin, immunoglobulin molecules, thyroglobulin, ovalbumin, and other proteins well known to those skilled in the art. Other reagents that can be used to bind molecules to the support include polysaccharides, polylactic acids, polyglycolic acids, polymeric amino acids, amino acid copolymers, and the like. Such molecules and methods of coupling these molecules to antigens, are well known to those of ordinary skill in the art.
See, e.g., Brinkley, M.A. (1992) Bioconjugate Chem. 3:2-13; Hashida et al. (1984) J. Appl. Biochem. 6:56-63; and Anjaneyulu and Staros (1987) International J. of Peptide and Protein Res. 30:117-124.

After reacting the solid support with the solid-phase components, any nonimmobilized solid-phase components are removed from the support by washing, and the support-bound components are then contacted with a biological sample suspected of containing anti-HCV antibodies (collectively called "ligand molecules" herein) under suitable binding conditions. If anti HCV antibodies are present in the sample, they will form a complex with the HCV antigens. After washing to remove any nonbound ligand molecules, detectably labeled antibodies, such as anti-xenogenic (e.g., anti-human) antibodies, which recognize an epitope on anti-HCV antibodies, are added. These antibodies bind due to complex formation.

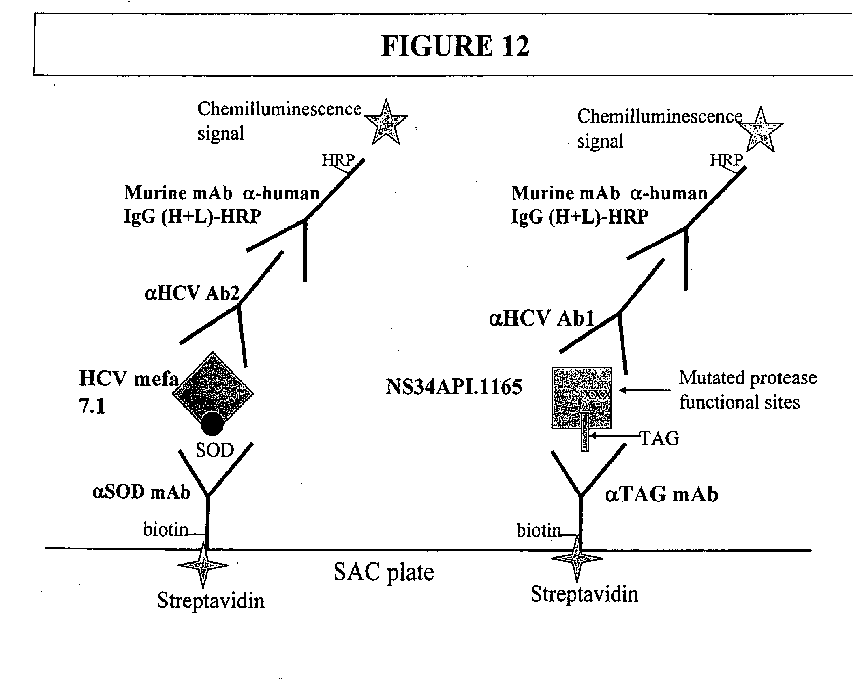

Another assay format is shown in Figure 12. This assay format, well known in the art, uses a streptavidin-coated solid support reacted with biotin labeled antibodies that bind the modified NS3 and, optionally, a MEFA, such as MEFA 7.1. The sample is added under suitable binding conditions. If anti HCV antibodies are present in the sample, they will form a complex with the HCV antigens. After washing to remove any nonbound ligand molecules, detectably labeled antibodies are added, as described above.

In a homogeneous format, the test sample is incubated with the combination of antigens in solution. For example, it may be under conditions that will precipitate any antigen-antibody complexes which are formed. Both standard and competitive formats for homogeneous assays are also known in the art.

In a standard format, the amount of HCV antibodies forming the antibody-antigen complex is directly monitored. This may be accomplished by determining whether labeled anti-xenogenic (e.g., anti-human) antibodies which recognize an epitope on anti-HCV antibodies will bind due to complex formation. In a competitive format, the amount of HCV antibodies in the sample is deduced by monitoring the competitive effect on the binding of a known amount of labeled antibody (or other competing ligand) in the complex.

More particularly, complexes formed comprising anti-HCV antibody (or, in the case of competitive assays, the amount of competing antibody) are detected by any of a number of known techniques, depending on the format. For example, unlabeled HCV antibodies in the complex may be detected using a conjugate of antixenogeneic Ig complexed with a label, (e.g., an enzyme label). In an immunoprecipitation or agglutination assay format, the reaction between the HCV antigens and the antibody forms a network that precipitates from the solution or suspension and forms a visible layer or film of precipitate. If no anti-HCV antibody is present in the test specimen, no visible precipitate is formed. The above-described assay reagents, including the immunoassay solid support with bound antibodies and antigens, as well as antibodies and antigens to be reacted with the captured sample, can be provided in kits, with suitable instructions and other necessary reagents, in order to conduct immunoassays as described above. The kit will normally contain in separate containers the combination of antigens (either already bound to a solid matrix or separate with reagents for binding them to the matrix), control antibody formulations (positive and/or negative), labeled antibody when the assay format requires same and signal generating reagents (e.g., enzyme substrate) if the label does not generate a signal directly. Instructions (e.g., written, tape, VCR, CD-ROM, etc.) for carrying out the assay usually will be included in the kit. The kit can also contain, depending on the particular immunoassay used, other packaged reagents and materials (i.e. wash buffers and the like). Standard immunoassays, such as those described above, can be conducted using these kits.

### III. Experimental

Below are examples of specific embodiments for carrying out the present invention. The examples are offered for illustrative purposes only, and are not intended to limit the scope of the present invention in any way.

Efforts have been made to ensure accuracy with respect to numbers used (e.g., amounts, temperatures, etc.), but some experimental error and deviation should, of course, be allowed for.

### EXAMPLE 1

### Production of Modified NS3 Proteins

### I. NS34aPI

NS34aPI, with mutations to the NS3 helicase domain but not the protease domain, was produced for expression by the yeast expression vector pBS24.1 as follows. NS34aPI includes the amino acid sequence for the native NS3/4a polypeptide, with the exception that amino acid Thr-1428 and Ser-1429, numbered relative to the HCV-1 full-length sequence, have been mutated to Pro and Ile, respectively. The yeast expression vector pBS24.1 contains 2µ sequences for autonomous replication in yeast and the yeast genes *leu2-d* and URA3 as selectable markers. The ß-lactamase gene and the ColEl origin of replication, required for plasmid replication in bacteria, are also present in this expression vector. Plasmid pd.hcvla.ns3ns4aPI, encoding NS34aPI was produced as follows. A two step procedure was used. First, the following DNA pieces were ligated together: (a) synthetic oligonucleotides which would provide a 5' *Hind*III cloning site, followed by the sequence ACAAAACAAA (SEQ ID NO:12), the initiator ATG, and codons for HCV1a, beginning with amino acid 1027 and continuing to *a Bgl*I site at amino acid 1046; (b) a 683 bp *Bgl*I*-Cla*I restriction fragment (encoding amino acids 1046-1274) from pAcHLTns3ns4aPI; and (c) a pSP72 vector (Promega, Madison, WI, GenBank/EMBL Accession Number X65332) which had been digested with *Hind*III and *ClaI,* dephosphorylated, and gel-purified. Plasmid pAcHLTns3ns4aPI was derived from pAcHLT, a baculovirus expression vector commercially available from BD Pharmingen (San Diego, CA). In particular, a pAcHLT *Eco*RI*-Pst*I vector was prepared, as well as the following fragments: *EcoR*I*- Alwn*I, 935 bp, corresponding to amino acids 1027-1336 of the HCV-1 genome; *Alwn*I*-Sac*II, 247 bp, corresponding to amino acids 1336-1419 of the HCV-1 genome; *Hinf*I*-Bgl*I, 175 bp, corresponding to amino acids 1449-1509 of the HCV-1 genome; *Bgl*I-*Pst*I, 619 bp, corresponding to amino acids 1510-1711 of the HCV-1 genome, plus the transcription termination codon. A *Sac*II*-Hinf*I synthetically generated fragment of 91 bp, corresponding to amino acids 1420-1448 of the HCV-1 genome and containing the PI mutations (Thr-1428 mutated to Pro, Ser-1429 mutated to Ile), was ligated with the 175 bp *Hinf*I*-Bgl*I fragment and the 619 bp *Bgl*I*-Pst*I fragment described above and subcloned into a pGEM-5Zf(+) vector digested with *Sac*II and *Pst*I. pGEM-5Zf(+) is a commercially available *E. coli* vector (Promega, Madison, WI, GenBank/EMBL Accession Number X65308). After transformation of competent HB101 cells, miniscreen analysis of individual clones and sequence verification, an 885 bp *Sac*II*-Pst*I fragment from pGEM5.PI clone2 was gel-purified. This fragment was ligated with the *Eco*RI*-Alwn*I 935 bp fragment, the *Alwn*I*-Sac*II 247 bp fragment and the pAcHLT *Eco*RI*-Pst*I vector, described above. The resultant construct was named pAcHLTns3ns4aPI.

The ligation mixture above was transformed into HB101-competent cells and plated on Luria agar plates containing 100 µg/ml ampicillin. Miniprep analyses of individual clones led to the identification of putative positives, two of which were amplified. The plasmid DNA for pSP72 1aHC, clones #1 and #2 were prepared with a Qiagen Maxiprep kit and were sequenced.

Next, the following fragments were ligated together: (a) a 761 bp *Hind*III*-Cla*I fragment from pSP721aHC #1 (pSP72.1aHC was generated by ligating together the following: pSP72 which had been digested with *Hind*III and *Cla*I, synthetic oligonucleotides which would provide a 5= *Hind*III cloning site, followed by the sequence ACAAAACAAA (SEQ ID NO:12), the initiation codon ATG, and codons for HCV1a, beginning with amino acid 1027 and continuing to a *Bgl*II site at amino acid 1046, and a 683 bp *Bgl*II*-Cla*I restriction fragment (encoding amino acids 1046-1274) from pAcHLTns3ns4aPI); (b) a 1353 bp *Bam*HI*-Hind*III fragment for the yeast hybrid promoter ADH2/GAPDH; (c) a 1320 bp *Cla*I*-Sal*I fragment (encoding HCV1a amino acids 1046-1711 with Thr 1428 mutated to Pro and Ser 1429 mutated to Ile) from pAcHLTns3ns4aPI; and (d) the pBS24.1 yeast expression vector which had been digested with *Bam*HI and *Sal*I, dephosphorylated and gel-purified. The ligation mixture was transformed into competent HB101 and plated on Luria agar plates containing 100 µg/ml ampicillin. Miniprep analyses of individual colonies led to the identification of clones with the expected 3446 bp *Bam*HI*-Sal*I insert which was comprised of the ADH2/GAPDH promoter, the initiator codon ATG and HCV1a NS34a from amino acids 1027-1711, with Thr 1428 mutated to Pro and Ser 1429 mutated to Ile. The construct was named pd.HCV1a.ns3ns4aPI.

*S. cerevisiae* strain AD3 was transformed with pd.HCV1a.ns3ns4aPI and single transformants were checked for expression after depletion of glucose in the medium. The recombinant protein was expressed at high levels in yeast, as detected by Coomassie blue staining and confirmed by immunoblot analysis using a polyclonal antibody to the helicase domain of NS3.

The NS3/4a protein was purified as follows. *S. cerevisiae* cells from above, expressing the NS34aPI protein were harvested and the cells were suspended in lysis buffer (50 mM Tris pH 8.0, 150 mM NaCl, 1 mM EDTA, 1 mM PMSF, 0.1 µM pepstatin, 1 µM leupeptin) and lysed in a Dyno-Mill (Wab Willy A. Bachofon, Basel, Switzerland) or equivalent apparatus using glass beads, at a ratio of 1:1:1 cells:buffer:0.5 mm glass beads. The lysate was centrifuged at 30100 x g for 30 min at 4°C and the pellet containing the insoluble protein fraction was added to wash buffer (6 ml/g start cell pellet weight) and rocked at room temperature for 15 min. The wash buffer consisted of 50 mM NaPO₄ pH 8.0, 0. 3 M NaCl, 5 mM β-mercaptoethanol, 10% glycerol, 0.05% octyl glucoside, 1 mM EDTA, 1 mM PMSF, 0.1 µM pepstatin, 1 µM leupeptin. Cell debris was removed by centrifugation at 30100 x g for 30 min at 4°C. The supernatant was discarded and the pellet retained.

Protein was extracted from the pellet as follows. 6 ml/g extraction buffer was added and rocked at room temperature for 15 min. The extraction buffer consisted of 50 mM Tris pH 8.0, 1 M NaCl, 5 mM β-mercaptoethanol, 10% glycerol, 1 mM EDTA, 1 mM PMSF, 0.1 µM pepstatin, 1 µM leupeptin. This was centrifuged at 30100 x g for 30 min at 4°C. The supernatant was retained and ammonium sulfate added to 17.5% using the following formula: volume of supernatant (ml) multiplied by x% ammonium sulfate/(1 - x% ammonium sulfate) = ml of 4.1 M saturated ammonium sulfate to add to the supernatant. The ammonium sulfate was added dropwise while stirring on ice and the solution stirred on ice for 10 min. The solution was centrifuged at 17700 x g for 30 min at 4°C and the pellet retained and stored at 2°C to 8°C for up to 48 hrs.

The pellet was resuspended and run on a Poly U column (Poly U Sepharose 4B, Amersham Pharmacia) at 4°C as follows. Pellet was resuspended in 6 ml Poly U equilibration buffer per gram of pellet weight. The equilibration buffer consisted of 25 mM HEPES pH 8.0,200 mM NaCl, 5 mM DTT (added fresh), 10% glycerol, 1.2 octyl glucoside. The solution was rocked at 4°C for 15 min and centrifuged at 31000 x g for 30 min at 4°C.

A Poly U column (1 ml resin per gram start pellet weight) was prepared. Linear flow rate was 60 cm/hr and packing flow rate was 133% of 60 cm/hr. The column was equilibrated with equilibration buffer and the supernatant of the resuspended ammonium sulfate pellet was loaded onto the equilibrated column. The column was washed to baseline with the equilibration buffer and protein eluted with a step elution in the following Poly U elution buffer: 25 mM HEPES pH 8.0, 1 M NaCl, 5 mM DTT (added fresh), 10% glycerol, 1.2 octyl glucoside. Column eluate was run on SDS-PAGE (Coomassie stained) and aliquots frozen and stored at -80°C. The presence of the NS34aPI protein was confirmed by Western blot, using a polyclonal antibody directed against the NS3 protease domain and a monoclonal antibody against the 5-1-1 epitope (HCV 4a).

Additionally, protease enzyme activity was monitored during purification as follows. An NS4a peptide (KKGSVVIVGRIVLSGKPAIIPKK, SEQ ID NO:13), and the sample containing the NS34aPI protein, were diluted in 90 µl of reaction buffer (25 mM Tris, pH 7.5, 0.15M NaCl, 0.5 mM EDTA, 10% glycerol, 0.05 n-Dodecyl B-D-Maltoside, 5 mM DTT) and allowed to mix for 30 minutes at room temperature. 90 µl of the mixture were added to a microtiter plate (Costar, Inc., Coming, NY) and 10 µl of HCV substrate (AnaSpec, Inc., San Jose CA) was added. The plate was mixed and read on a Fluostar plate reader. Results were expressed as relative fluorescence units (RFU) per minute.

Using these methods, the product of the 1 M NaCl extraction contained 3.7 RFU/min activity, the ammonium sulfate precipitate had an activity of 7.5 RFU/min and the product of the Poly U purification had an activity of 18.5 RFU/min.

### II. Modified NS3 Antigens with Mutations in the Protease Domain

Modified NS3 antigens, with a mutation to the NS3 protease domain were produced using site-directed mutagenesis of the NS34aPI sequence described above. In particular, the codon for Ser-1165, in the protease catalytic triad, was mutated to an Ala codon. A diagram of the NS34aPI construct including a substitution of Ala for Ser at position 1165 is shown in Figure 4A. The nucleotide and corresponding amino acid sequence of this construct is shown in Figures 5A-5C. This construct was termed "NS34aPI. 1165."

Additionally, the modified NS3 protein depicted in Figure 4C was produced. As with the protein described above, this modified NS3 also included the PI mutation in the helicase domain. The protein included amino acids 1678-1690 of NS4a, followed by the amino acid sequence SGS, then amino acids 1029 to 1657 of NS3. The SGS sequence provides a flexible linker sequence which allows the NS4a polypeptide to fold into the pocket within the protease to create an enzymatically active molecule. The nucleotide sequence and corresponding amino acid sequence of this protein is shown in Figures 7A-7C. This construct was termed "d.4a.t.NS3PI.1165."

Finally, a modified NS3 protein, shown in Figure 4B, which included the Ala substitution at amino acid 1165, as well as the PI substitutions in the helicase domain, was produced as follows. The nucleotide sequence and corresponding amino acid sequence of this protein is shown in Figures 6A-6C. This construct was termed "NS3PI.1165."

To construct the NS3PI.1165 yeast expression vector, the following steps were taken. First, plasmid pSP72NS3NS4a.PI.HindIII/Claᵢ #9 was generated by ligation of a *Bgl*I/*Cla*I fragment (isolated from pAcHLT NS3NS4aPI, described above) with a *Hind*III/*Bgl*I synthetic oligonucleotide of 97 bp (below) then transformed into HB101.
AGCTTACAAAACAAAATGCATCACCATCACCATCACGCGCC HBgI-1 (SEQ ID NO:14)
Hbgl-2 (SEQ ID NO:15)
Hbgl-5 (SEQ ID NO:16)
AGGCTGGTGATTATGCACCCTAGGAGGCCCCTTGTCTGCTGG Hbgl-6 (SEQ ID NO:17)

After miniscreens and sequence confirmation, plasmid pSP72NS3NS4a.PIHindIII/Claᵢ #9 was retransformed into SCS110 *E. coli* competent cells that were Dam⁻ methylation. This plasmid was then used as a template to mutate the Serine₁₁₆₅ of the HCV1a NS3 protease catalytic triad to Alanine using site-directed mutagenesis (GeneTailor Site-Directed Mutagenesis, Invitrogen, San Diego CA), using the following PCR primers:
5' TTTCCTACTTGAAAGGCTCCgcaGGGGGTCCGCT^{3'} (SEQ ID NO:18)
_{3'}GGGGCCGGGTAAAGGATGAACTTTCCGAGG_{5'} (SEQ ID NO:19)

After miniscreen analysis and sequence verification, the clone with the correct sequence was named pSP72NS3NS4aPImut1165 #32. Next, this plasmid was digested with *Hind*III and *Avr*II (*Bln*I) to prepare a vector for ligation with a 58 base pair synthetic oligonucleotide encoding the 14aa of the N-terminus from the original HCV1a NS3 domain, using yeast preferred codons.
HA-1 (SEQ ID NO:20)
HA-2 (SEQ ID NO:21)

After HB101 transformation, miniscreen analysis and sequence verification the new plasmid with the correct sequence was named pSP72H3/ClaINS3mut1165 #15. Since the *Cla*I site of HCV1a NS3 domain is methylated, SCS 110 *E. coli* competent cells were used to transform the pSP72H3/ClaINS3mut1165 #15 subclone. Next, this plasmid was digested with *Hind*III and *ClaI* to prepare a 761 bp fragment.

Second, a *Cla*I/*Eag*I fragment of 1129 bp was isolated from baculovirus expression plasmid pAcHLTns3ns4aPI. The *Cla*I/*Eag*I fragment was ligated with a 195 bp synthetic oligonucleotide encoding the C-terminus of the NS3 domain plus the NS4a peptide into the pSP72 *Cla*I*lSal*I vector to create subclone pSP72NS3NS4aPI Cla/Sal #30 which was retransformed into SCS110 competent cells, a strain in which the *ClaI* site was not methylated as it was in HB101:
PI3p-1 (SEQ ID NO:22)
PI3p-2 (SEQ ID NO:23)
PI3p-3 (SEQ ID NO:24)
PI3p-4 (SEQ ID NO:25)
CGAGAGTTCGATGAGATGGAAGAGTGCTGATAAG PI3p-5 (SEQ ID NO:26)
PI3p-6 (SEQ ID NO:27)

From this plasmid, a vector was prepared by digesting with *Ava*3 and *Sal*I restriction enzymes, thus eliminating the NS4a domain. Into this vector a 37 bp synthetic oligonucleotide was ligated and transformed into HB101.
TGTCGGCCGACCTGGAGGTCGTCACGTGATAAG avsal-1 (SEQ ID NO:28)
TCGACTTATCACGTGACGACCTCCAGGTCGGCCGACATGCA avsal-2 (SEQ ID NO:29)

After miniscreen analysis and sequence confirmation, pSP72 ClaISalI NS3PI subclone #101 was generated. This plasmid was again retransformed into SCS110, as described earlier to isolate a *Cla*I/*Sal*I fragment of 1320 bp.

Lastly, the *Bam*HI/*Hind*III ADH2/GAPDH promoter, *Hind*III*lCla*I 761 bp fragment and *Cla*I*lSal*I 1320 bp fragment were ligated into the pBS24.1 *Bam*HI*lSal*I yeast expression vector and transformed using *E. coli* strain HB101 competent cells. Miniprep analysis of the individual colonies led to the identification of clones with an expected fragment of 3284 bp. The construct was named pd.NS3PI.₁₁₆₅ #21. Expression in *S. cerevisiae* AD3 strain yeast was checked as described above for pd.HCV1a.ns3ns4aPI.

The d.4a.t.NS3PI.1165 yeast expression plasmid was produced as follows. A synthetic oligonucleotide of 100 bp encoding the NS4a peptide followed by the amino acid sequence Ser-Gly-Ser and amino acids 1029-1039 of the NS3 domain were ligated into the vector pSP72NS3NS4aPImut1165 #32 *Hind*III/*Avr*II (described above) and transformed into HB101.
AGCTTACAAAACAAAATGGGCTGCGTG HA-5 (SEQ ID NO:30)
GACCCTGCCCACTATGACCACGCAGCCCATTTTGTTTTGTA HA-6 (SEQ ID NO:31)
HA-7 (SEQ ID NO:32)
HA-8 (SEQ ID NO:33)

After miniscreens and sequence verification, pSP724a-t-NS3PI.₁₁₆₅ #14 was generated. The subsequent plasmid was then transformed into SCS 110 for reasons described above, and a *Hind*III/*Cla*I fragment of 803 bp was isolated. To create the d.4a.t.NS3PI.1165 yeast expression plasmid, the ADH2/GAPDH *Bam*HI/*Hind*III promoter was ligated with the 803 bp *Hind*III/*Cla*I fragment and the 1158 bp *Cla*I*lSal*I fragment encoding the 3' end of the NS3PI region into the pAB24 *Bam*HI/*Sal*I yeast expression vector. pd.4a-t-ns3.1165 #4 was transformed into *S. cerevisiae* AD3 yeast strain, and single transformants were checked for expression as described above for pd.HCV1a.ns3ns4aPI.

The proteins were purified as above. All of the mutant proteins had expression levels in yeast similar to that ofNS34aPI, and were purified to similar purity as NS34aPI (more than 90% purity). SDS-PAGE and Western blot confirmed the lack of proteolytic activity of modified NS3 polypeptides. In particular, NS34aPI.1165 was shown to be the uncleaved, full-length protein, and NS3PI.1165 and NS34aPI. 1165 did not cleave MEFA 7.1. Thus, the modified proteins lacked proteolytic activity.

### EXAMPLE 2

### Immunoassays Using the Mutated HCV Antigens

The HCV antigens and, in some cases as specified, MEFA 7.1 antigen, were coated onto plates for immunoassays as follows. MEFA 7.1 was produced as described in U.S. Patent No. 6,632,601.
MEFA 7.1 is a natural substrate for the NS3 protease. HCV coating buffer (50 mM NaPO4 pH 7.0, 2 mM EDTA and 0.1% Chloroacetamide) was filtered through a 0.22 µ filter unit. The following reagents were then added sequentially to the HCV coating buffer and stirred after each addition: 2 µg/ml BSA-Sulfhydryl Modified, from a 10 mg/ml solution (Bayer Corp. Pentex, Kankakee, Illinois); 5 mM DTT from a 1 M solution (Sigma, St. Louis, MO); 0.45 µg/ml NS3/4a (protein concentration of 0.3 mg/ml); 0.375 µg/ml MEFA 7.1 (protein concentration of 1 mg/ml). The final solution was stirred for 15 minutes at room temperature.

200 µl of the above solution was added to each well of a Costar high binding, flat bottom plate (Coming Inc., Corning, New York) and the plates were incubated overnight in a moisture chamber. The plates were then washed with wash buffer (1 x PBS, 0.1% TWEEN-20), Tapped dry and 285 µl Ortho Post-Coat Buffer (1 x PBS, pH 7.4, 1% BSA, 3% sucrose) added. The plates were incubated for at least 1 hour, tapped and dried overnight at 2-8°C. The plates were pouched with desiccants for future use.

The performance of the modified NS3 antigens in comparison with NS34aPI that lacked the mutation to the proteolytic domain, was studied. Panels of commercially available human blood samples were used which were HCV-infected. The PHV panels shown in the tables below were purchased from Boston Biomedica, Inc., West Bridgewater, MA (BBI).

The HCV assay was conducted as follows. 200 µl of specimen diluent buffer (1 g/l casein, 100 mg/l recombinant human SOD, 1 g/l chloracetamide, 10 g/l BSA, 500 mg/l yeast extract, 0.366 g/l EDTA, 1.162 g/l KPO₄, 5 ml/l Tween-20, 29.22 g/l NaCl, 1.627 g/l NaPO₄, 1% SDS) was added to the coated plates. 20 µl of sample was then added. This was incubated at 37°C for one hour. The plates were washed with wash buffer (1 x PBS, pH 7.4, 0.1% Tween-20). 200 µl conjugate solution (a mouse anti-human IgG-HRP, such as mouse anti-human IgG-HRP diluted 1:22,000 in ORTHO HCV 3.0 ELISA Test System with Enhanced SAVe bulk conjugate diluent (Ortho-Clinical Diagnostics, Raritan, New Jersey) was added and incubated for 60 minutes at 37°C. This was washed as above, and 200 µl substrate solution (1 OPD tablet/10ml) was added. The OPD tablet contains o-phenylenediamine dihydrochloride and hydrogen peroxide for horse radish peroxidase reaction color development. This was incubated for 30 minutes at room temperature in the dark. The reaction was stopped by addition of 50 µl 4N H₂SO₄ and the plates were read at 492 nm, relative to absorbance at 690 nm as control.

Results are shown in Tables 3-6. In particular, Table 3 shows the results of a comparison of immunoassay performance of NS3PI.1165 and NS34aPI.1165 versus NS34aPI. As shown in Table 3, both of the protease deficient mutants were immunoreactive, having approximately 80-90% of the reactivity of the NS34aPI control molecule that retained proteolytic activity when coated at 2X the amount. Table 6 shows the results of a comparison of immunoassay performance of d.4a.t.NS3PI.1165, NS3PI.1165 and NS34aPI.1165 versus NS34aPI. As can be seen in Table 6, when coated at appropriate levels, d.4a.t.NS3PI.1165, NS3PI.1165 and NS34aPI.1165 displayed similar immunoreactivity to that of NS34aPI with d.4a.t.NS3PI.1165 showing the closest immunoreactivity to NS34aPI.

As can be seen in Tables 4 and 7, NS3PI.1165, NS34aPI.1165 and d.4a.t.NS3PI.1165 also achieved very similar immunoreactivity as NS34aPI when used in combination with MEFA 7.1.

Table 5 shows the results of a comparison of immunoassay performance of NS3PI.1165 and d.4a.t.NS3PI.1165 versus NS34aPI, in assays either in combination with MEFA 7.1 or without MEFA 7.1. As can be seen, both mutants, either used alone or in combination with MEFA 7.1, achieved immunoreactivity very similar to that of native NS34aPI. Thus, all the three modified NS3 proteins tested have immunoreactivity.

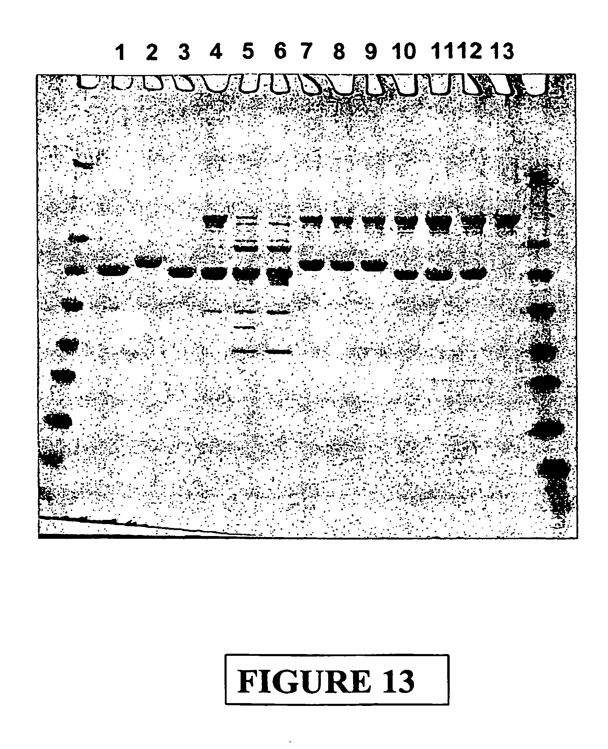

These three modified NS3 proteins had helicase activity (data not shown) but were devoid of protease activity. Moreover, as shown in Figures 13 and 14, the modified NS3 mutant proteins did not cleave MEFA 7.1, a natural substrate of the NS3 protease.

To the best of the inventors' knowledge, this is the first demonstration that immunoreactivity and protease activity of NS3 can be separated, and that eliminating the serine protease activity does not affect antibody recognition sites of the protein.

### Example 3

### Early Seroconversion Detection and Comparison with Commercially Available Assays

The C33c and C200 antigens are very immunoreactive, and antibodies to these antigens are found in early seroconversion panels. Thus, the performance of d.4a.t.NS3PI.1165 plus MEFA 7.1 or NS34aPI plus MEFA 7.1 in immunoassays was studied using well-characterized, commercially available c33c and c200 panels of HCV-infected human blood samples to assess seroconversion sensitivity and these results were compared to those obtained in commercial anti-HCV ELISA kits. In particular, the Abbott PRISM assay (Abbott Laboratories, Abbott Park, IL), is commercially available and is an antibody-based detection assay. The assay was performed using the manufacturer's instructions. The ORTHO HCV Version 3.0 ELISA Test System (HCV 3.0) (Ortho Clinical Diagnostics, Raritan, New Jersey) is an antibody-based detection assay. The assay was conducted using the manufacturer's instructions.

Results are shown in Table 7. For the panels tested, both NS34aPI plus MEFA 7.1 and d.4a.t.NS3PI.1165 plus MEFA 7.1 detected c33c- and c200-type antibody in the early seroconversion panels. The detections were 2-12 days ahead of Ortho HCV 3.0 and Abbott Prism assays.

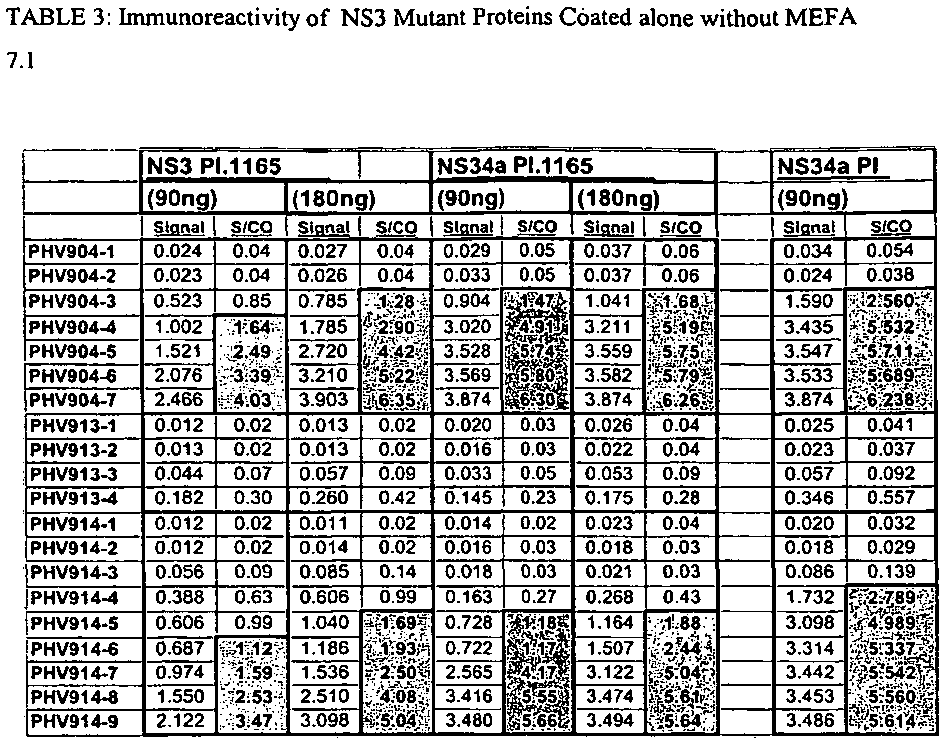

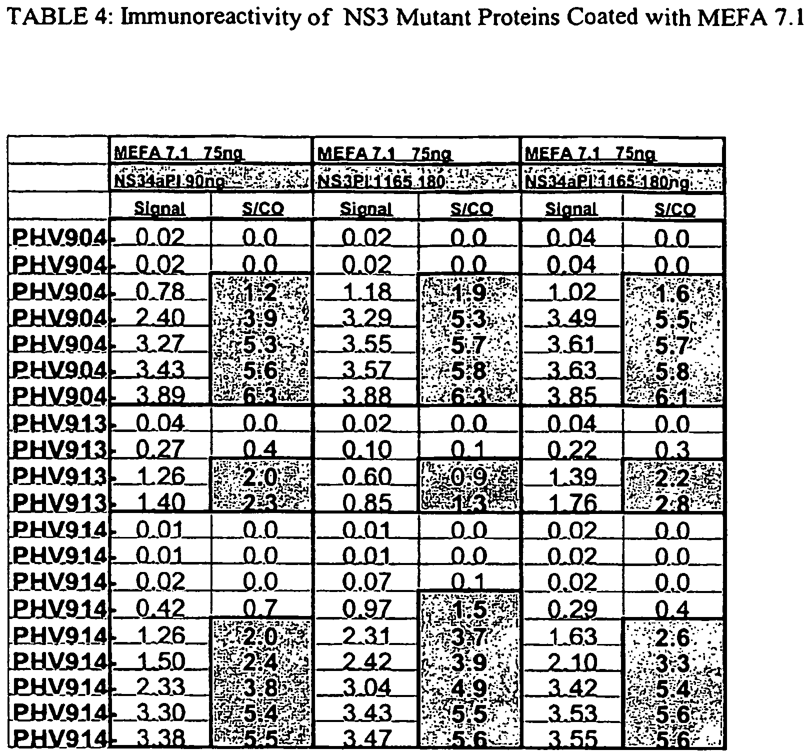

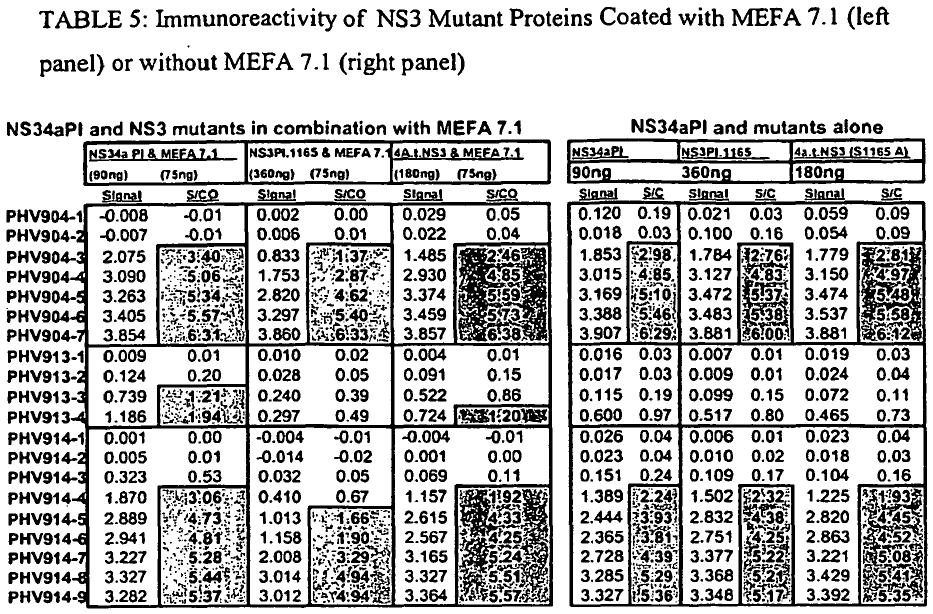

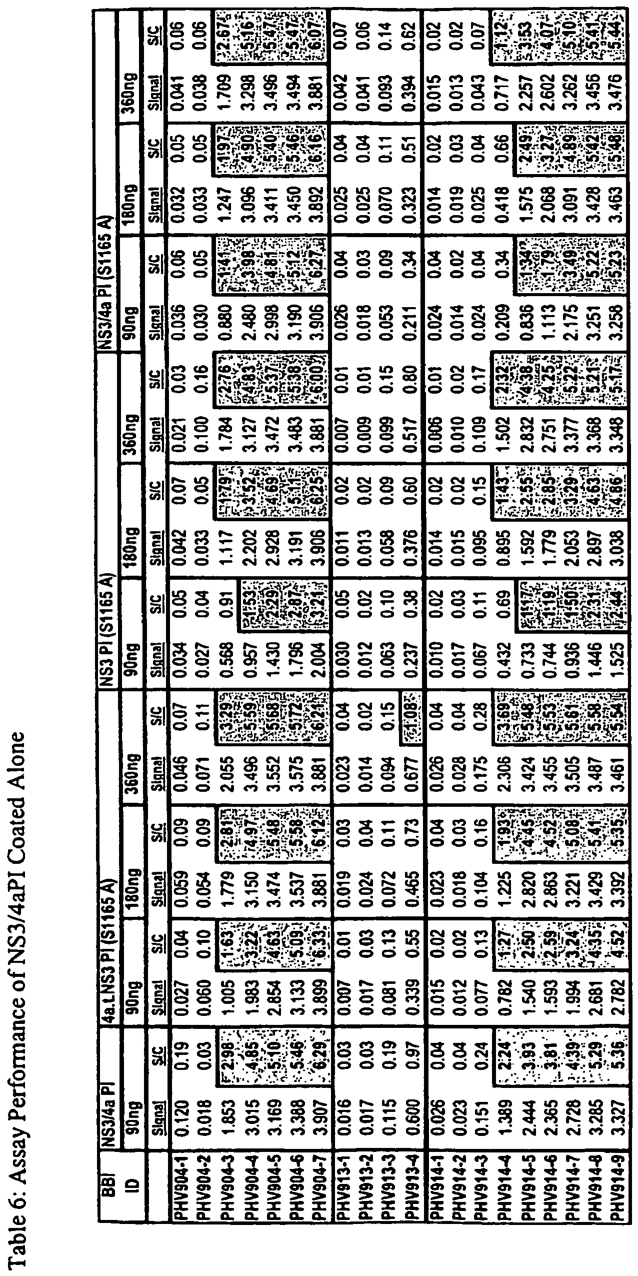

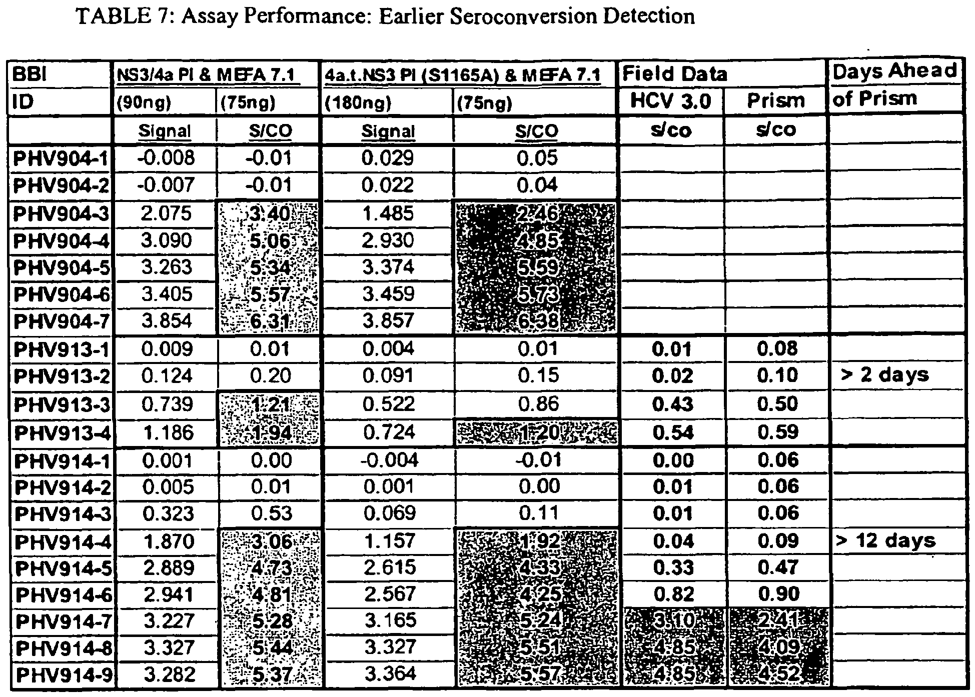

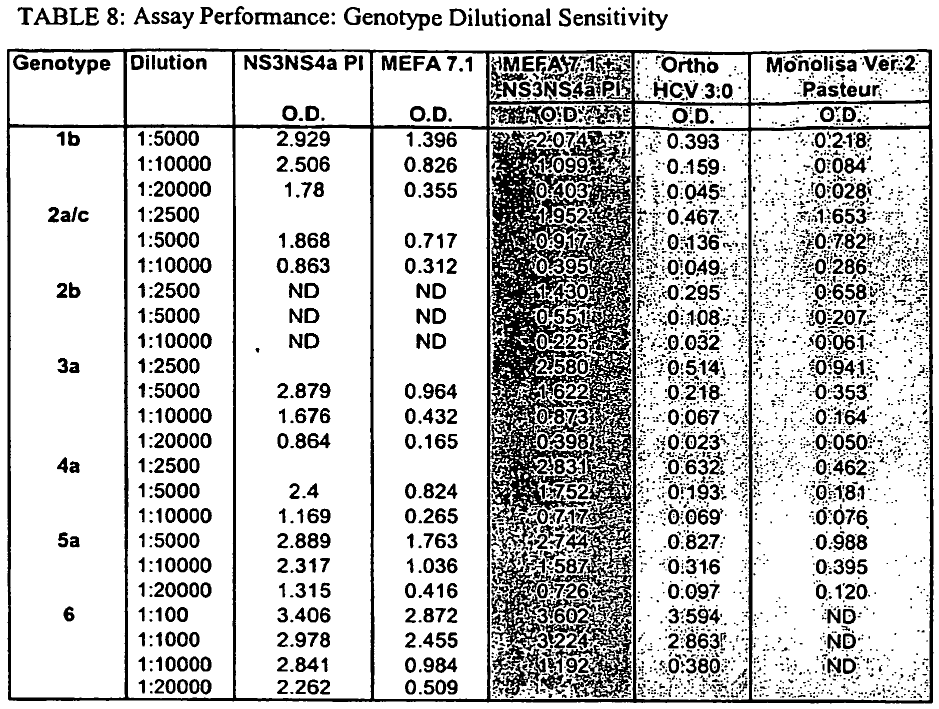

Accordingly, modified HCV NS3 proteins and use thereof in detection assays have been disclosed. From the foregoing, it will be appreciated that, although specific embodiments of the invention have been described herein for purposes of illustration, various modifications may be made without deviating from the scope thereof.

## Claims

1. An isolated polypeptide comprising the amino acid sequence of SEQ ID NO:2, 4 or 6, wherein the polypeptide comprises an NS3 conformational epitope.

2. An immunoassay solid support comprising a polypeptide of claim 1 which reacts specifically with anti-HCV antibodies present in a biological sample from an HCV-infected individual.

3. The immunoassay solid support of claim 2, wherein the solid support further comprises a multiple epitope fusion antigen bound to the support, wherein said polypeptide and/or said multiple epitope fusion antigen react specifically with anti-HCV antibodies present in a biological sample from an HCV-infected individual.

4. The immunoassay solid support of claim 3, wherein said multiple epitope fusion antigen comprises the amino acid sequence depicted in SEQ ID NO: 10, or an amino acid sequence with at least 80% sequence identity thereto which reacts specifically with anti-HCV antibodies present in a biological sample from an HCV- infected individual.

5. A method of detecting hepatitis C virus (HCV) infection in a biological sample, said method comprising:
(a) providing an immunoassay solid support according to any of claims 2-4;
(b) combining a biological sample with said solid support under conditions which allow anti-HCV antibodies, when present in the biological sample, to bind to said polypeptide and/or said multiple epitope fusion antigen to form a first immune complex;
(c) adding to the solid support from step (b) under complex forming conditions a detectably labeled antibody, wherein said labeled antibody is reactive with said immune complex;
(d) detecting second immune complexes formed between the detectably labeled antibody and the first immune complex, if any, as an indication of HCV infection in the biological sample.

6. An immunodiagnostic test kit comprising the immunoassay solid support of any of claims 2-4, and instructions for conducting the immunodiagnostic test.

7. A polynucleotide comprising a coding sequence for the polypeptide of claim 1.

8. A recombinant vector comprising:
(a) a polynucleotide according to claim 7;
(b) and control elements operably linked to said polynucleotide whereby the coding sequence can be transcribed and translated in a host cell.

9. A host cell transformed with the recombinant vector of claim 8.

10. A method of producing a recombinant polypeptide comprising:
(a) providing a population of host cells according to claim 9; and
(b) culturing said population of cells under conditions whereby the polypeptide encoded by the coding sequence present in said recombinant vector is expressed.

11. A method of producing an immunoassay solid support, comprising:
(a) providing a solid support; and
(b) binding to the solid support at least one polypeptide according to claim 1.

12. The method of claim 11, further comprising binding to the solid support at a discrete position a multiple epitope fusion antigen.

## Patentansprüche

1. Isoliertes Polypeptid, umfassend die Aminosäuresequenz der SEQ ID NO:2, 4 oder 6, wobei das Polypeptid ein NS3-Konformationsepitop umfasst.

2. Fester Immuntestträger, umfassend ein Polypeptid nach Anspruch 1, das spezifisch mit in einer biologischen Probe aus einem mit HCV infizierten Individuum vorliegenden Anti-HCV-Antikörpern reagiert.

3. Fester Immuntestträger nach Anspruch 2, wobei der feste Träger ferner ein an den Träger gebundenes Mehrfachepitop-Fusionsantigen umfasst, wobei das Polypeptid und/oder das Mehrfachepitop-Fusionsantigen spezifisch mit in einer biologischen Probe aus einem mit HCV infizierten Individuum vorliegenden Anti-HCV-Antikörpern reagieren.

4. Fester Immuntestträger nach Anspruch 3, wobei das Mehrfachepitop-Fusionsantigen die in SEQ ID NO: 10 dargestellte Aminosäuresequenz oder eine damit zu wenigstens 80% sequenzidentische Aminosäuresequenz umfasst, die spezifisch mit in einer biologischen Probe aus einem mit HCV infizierten Individuum vorliegenden Anti-HCV-Antikörpern reagiert.

5. Verfahren zum Nachweis einer Infektion mit Hepatitis-C-Virus (HCV) in einer biologischen Probe, wobei man in dem Verfahren
(a) einen festen Immuntestträger gemäß einem der Ansprüche 2-4 bereitstellt,
(b) eine biologische Probe mit dem festen Träger unter Bedingungen kombiniert, die bei Vorliegen von Anti-HCV-Antikörpern in der biologischen Probe diesen die Bindung an das Polypeptid und/oder das Mehrfachepitop-Fusionsantigen unter Bildung eines ersten Immunkomplexes gestatten,
(c) den festen Träger aus Schritt (b) unter komplexbildenden Bedingungen mit einem nachweisbar markierten Antikörper versetzt, wobei der markierte Antikörper gegenüber dem Immunkomplex reaktiv ist,
(d) zweite Immunkomplexe, die sich eventuell zwischen dem nachweisbar markierten Antikörper und dem ersten Immunkomplex ausbilden, als Anzeichen für eine HCV-Infektion in der biologischen Probe nachweist.

6. Immundiagnostisches Testkit, umfassend den festen Immuntestträger nach einem der Ansprüche 2-4 sowie Anweisungen zur Ausführung des immundiagnostischen Tests.

7. Polynukleotid, umfassend eine codierende Sequenz für das Polypeptid nach Anspruch 1.

8. Rekombinanter Vektor, umfassend:
(a) ein Polynukleotid gemäß Anspruch 7;
(b) und Kontrollelemente in operativer Verknüpfung mit dem Polynukleotid, womit die codierende Sequenz in einer Wirtszelle transkribiert und translatiert werden kann.

9. Wirtszelle, transformiert mit dem rekombinanten Vektor nach Anspruch 8.

10. Verfahren zur Herstellung eines rekombinanten Polypeptids, wobei man:
(a) eine Population von Wirtszellen gemäß Anspruch 9 bereitstellt und
(b) die Population von Zellen unter Bedingungen kultiviert, wodurch das von der in dem rekombinanten Vektor vorhandenen codierenden Sequenz codierte Polypeptid exprimiert wird.

11. Verfahren zur Herstellung eines festen Immuntestträgers, wobei man:
(a) einen festen Träger bereitstellt und
(b) an den festen Träger wenigstens ein Polypeptid gemäß Anspruch 1 bindet.

12. Verfahren nach Anspruch 11, bei dem man ferner an den festen Träger ein Mehrfachepitop-Fusionsantigen an einer diskreten Position bindet.

## Revendications

1. Polypeptide isolé comprenant la séquence d'acides aminés de SEQ ID n°: 2, 4 ou 6, dans laquelle le polypeptide comprend un épitope conformationnel NS3.

2. Support solide d'immunodosage comprenant un polypeptide, selon la revendication 1, qui réagît spécifiquement avec des anticorps anti-VHC présents dans un échantillon biologique provenant d'un individu infecté par un VHC.

3. Support solide d'immunodosage selon la revendication 2, dans laquelle le support solide comprend en outre un antigène de fusion à épitopes multiples lié au support, dans laquelle ledit polypeptide et/ou ledit antigène de fusion à épitopes multiples réagît spécifiquement avec des anticorps anti-VHC présents dans un échantillon biologique qui provient d'un individu infecté par un VHC.

4. Support solide d'immunodosage selon la revendication 3, dans laquelle ledit antigène de fusion à épitopes multiples comprend la séquence d'acides aminés décrite à la SEQ ID n°: 10 ou une séquence d'acides aminés ayant une identité de séquence d'au moins 80% avec celle-ci, lequel réagît spécifiquement avec des anticorps anti-VHC présents dans un échantillon biologique qui provient d'un individu infecté par un VHC.

5. Procédé de détection d'une infection par virus de l'hépatite C (VHC) dans un échantillon biologique, ledit procédé comprenant les étapes consistant à :
(a) fournir un support solide d'immunodosage, selon l'une quelconque des revendications 2 à 4 ;
(b) combiner un échantillon biologique avec ledit support solide dans des conditions qui permettent à des anticorps anti-VHC, lorsqu'ils sont présents dans l'échantillon biologique, de se lier au dit polypeptide et/ou au dit antigène de fusion à épitopes multiples pour former un premier complexe immun ;
(c) ajouter au support solide de l'étape (b), dans des conditions de formation d'un complexe, un anticorps marqué de manière détectable, dans lequel ledit anticorps marqué est réactif avec ledit complexe immun ;
(d) détecter des deuxièmes complexes immuns formés entre l'anticorps marqué de manière détectable et le premier complexe immun, s'il y en a, comme indication d'une infection à VHC dans l'échantillon biologique.

6. Trousse de test immunodiagnostique comprenant le support solide d'immunodosage, selon l'une quelconque des revendications 2 à 4, et des instructions pour exécuter le test immunodiagnostique.

7. Polynucléotide comprenant une séquence codant pour le polypeptide selon la revendication 1.

8. Vecteur recombinant comprenant :
(a) un polynucléotide selon la revendication 7 ;
(b) et des éléments de contrôle qui sont liés, de manière opérationnelle, audit polynucléotide, moyennant quoi la séquence codante peut être transcrite et traduite dans une cellule hôte.

9. Cellule hôte transformée avec le vecteur recombinant selon la revendication 8.

10. Procédé de production d'un polypeptide recombinant comprenant les étapes consistant à :
(a) fournir une population de cellules hôtes selon la revendication 9 ; et
(b) cultiver ladite population de cellules dans des conditions, moyennant quoi le polypeptide codé par la séquence codante, qui est présente dans ledit vecteur recombinant, est exprimé.

11. Procédé de production d'un support solide d'immunodosage, comprenant les étapes consistant à :
(a) fournir un support solide ; et
(b) lier au moins un polypeptide, selon la revendication 1, au support solide.

12. Procédé, selon la revendication 11, comprenant en outre la liaison au support solide, en une position discrète, d'un antigène de fusion à épitopes multiples.


### SEQUENCE LISTING

<110> CHIRON CORPORATION
<120> HCV NON-STRUCTURAL PROTEIN MUTANTS AND USES THEREOF
<130> 2300-22731.40 (PP22731.0004)
<150> 60/621,790 <151> 2004-10-25
<150> 60/621,502 <151> 2004-10-22
<150> 60/618,390 <151> 2004-10-12
<150> 60/604,858 <151> 2004-08-27
<160> 33
<170> PatentIn version 3.3
<210> 1
   <211> 2061
   <212> DNA
   <213> Artificial
<220>
   <223> NS34aPI.1165
<400> 1
   atggcgccca tcacggcgta cgcccagcag acaaggggcc tcctagggtg cataatcacc 60
   agcctaactg gccgggacaa aaaccaagtg gagggtgagg tccagattgt gtcaactgct 120
   gcccaaacct tcctggcaac gtgcatcaat ggggtgtgct ggactgtcta ccacggggcc 180
   ggaacgagga ccatcgcgtc acccaagggt cctgtcatcc agatgtatac caatgtagac 240
   caagaccttg tgggctggcc cgctccgcaa ggtagccgat cattgacacc ctgcacttgc 300
   ggctcctcgg acctttacct ggtcacgagg cacgccgatg tcattcccgt gcgccggcgg 360
   ggtgatagca ggggcagcct gctgtcgccc cggcccattt cctacttgaa aggctccgca 420
   gggggtccgc tgttgtgccc cgcggggcac gccgtgggca tatttagggc cgcggtgtgc 480
   acccgtggag tggctaaggc ggtggacttt atccctgtgg agaacctaga gacaaccatg
   540
   aggtccccgg tgttcacgga taactcctct ccaccagtag tgccccagag cttccaggtg
   600
   gctcacctcc atgctcccac aggcagcggc aaaagcacca aggtcccggc tgcatatgca
   660
   gctcagggct ataaggtgct agtactcaac ccctctgttg ctgcaacact gggctttggt
   720
   gcttacatgt ccaaggctca tgggatcgat cctaacatca ggaccggggt gagaacaatt
   780
   accactggca gccccatcac gtactccacc tacggcaagt tccttgccga cggcgggtgc
   840
   tcggggggcg cttatgacat aataatttgt gacgagtgcc actccacgga tgccacatcc
   900
   atcttgggca ttggcactgt ccttgaccaa gcagagactg cgggggcgag actggttgtg
   960
   ctcgccaccg ccacccctcc gggctccgtc actgtgcccc atcccaacat cgaggaggtt
   1020
   gctctgtcca ccaccggaga gatccctttt tacggcaagg ctatccccct cgaagtaatc
   1080
   aaggggggga gacatctcat cttctgtcat tcaaagaaga agtgcgacga actcgccgca
   1140
   aagctggtcg cattgggcat caatgccgtg gcctactacc gcggtcttga cgtgtccgtc
   1200
   atcccgccca tcggcgatgt tgtcgtcgtg gcaaccgatg ccctcatgac cggctatacc
   1260
   ggcgacttcg actcggtgat agactgcaat acgtgtgtca cccagacagt cgatttcagc
   1320
   cttgacccta ccttcaccat tgagacaatc acgctccccc aagatgctgt ctcccgcact
   1380
   caacgtcggg gcaggactgg cagggggaag ccaggcatct acagatttgt ggcaccgggg
   1440
   gagcgcccct ccggcatgtt cgactcgtcc gtcctctgtg agtgctatga cgcaggctgt
   1500
   gcttggtatg agctcacgcc cgccgagact acagttaggc tacgagcgta catgaacacc
   1560
   ccggggcttc ccgtgtgcca ggaccatctt gaattttggg agggcgtctt tacaggcctc
   1620
   actcatatag atgcccactt tctatcccag acaaagcaga gtggggagaa ccttccttac 1680
   ctggtagcgt accaagccac cgtgtgcgct agggctcaag cccctccccc atcgtgggac 1740
   cagatgtgga agtgtttgat tcgcctcaag cccaccctcc atgggccaac acccctgcta 1800
   tacagactgg gcgctgttca gaatgaaatc accctgacgc acccagtcac caaatacatc 1860
   atgacatgca tgtcggccga cctggaggtc gtcacgagca cctgggtgct cgttggcggc 1920
   gtcctggctg ctttggccgc gtattgcctg tcaacaggct gcgtggtcat agtgggcagg 1980
   gtcgtcttgt ccgggaagcc ggcaatcata cctgacaggg aagtcctcta ccgagagttc 2040
   gatgagatgg aagagtgctg a 2061
<210> 2
   <211> 686
   <212> PRT
   <213> Artificial
<220>
   <223> NS34aPI.1165
<400> 2
   Met Ala Pro Ile Thr Ala Tyr Ala Gln Gln Thr Arg Gly Leu Leu Gly
   1 5 10 15
   Cys Ile Ile Thr Ser Leu Thr Gly Arg Asp Lys Asn Gln Val Glu Gly
   20 25 30
   Glu Val Gln Ile Val Ser Thr Ala Ala Gln Thr Phe Leu Ala Thr Cys
   35 40 45
   Ile Asn Gly Val Cys Trp Thr Val Tyr His Gly Ala Gly Thr Arg Thr
   50 55 60
   Ile Ala Ser Pro Lys Gly Pro Val Ile Gln Met Tyr Thr Asn Val Asp
   65 70 75 80
   Gln Asp Leu Val Gly Trp Pro Ala Pro Gln Gly Ser Arg Ser Leu Thr
   85 90 95
   Pro Cys Thr Cys Gly Ser Ser Asp Leu Tyr Leu Val Thr Arg His Ala
   100 105 110
   Asp Val Ile Pro Val Arg Arg Arg Gly Asp Ser Arg Gly Ser Leu Leu
   115 120 125
   Ser Pro Arg Pro Ile Ser Tyr Leu Lys Gly Ser Ala Gly Gly Pro Leu
   130 135 140
   Leu Cys Pro Ala Gly His Ala Val Gly Ile Phe Arg Ala Ala Val Cys
   145 150 155 160
   Thr Arg Gly Val Ala Lys Ala Val Asp Phe Ile Pro Val Glu Asn Leu
   165 170 175
   Glu Thr Thr Met Arg Ser Pro Val Phe Thr Asp Asn Ser Ser Pro Pro
   180 185 190
   Val Val Pro Gln Ser Phe Gln Val Ala His Leu His Ala Pro Thr Gly
   195 200 205
   Ser Gly Lys Ser Thr Lys Val Pro Ala Ala Tyr Ala Ala Gln Gly Tyr
   210 215 220
   Lys Val Leu Val Leu Asn Pro Ser Val Ala Ala Thr Leu Gly Phe Gly
   225 230 235 240
   Ala Tyr Met Ser Lys Ala His Gly Ile Asp Pro Asn Ile Arg Thr Gly
   245 250 255
   Val Arg Thr Ile Thr Thr Gly Ser Pro Ile Thr Tyr Ser Thr Tyr Gly
   260 265 270
   Lys Phe Leu Ala Asp Gly Gly Cys Ser Gly Gly Ala Tyr Asp Ile Ile
   275 280 285
   Ile Cys Asp Glu Cys His Ser Thr Asp Ala Thr Ser Ile Leu Gly Ile
   290 295 300
   Gly Thr Val Leu Asp Gln Ala Glu Thr Ala Gly Ala Arg Leu Val Val
   305 310 315 320
   Leu Ala Thr Ala Thr Pro Pro Gly Ser Val Thr Val Pro His Pro Asn
                   325                 330                 335
   Ile Glu Glu Val Ala Leu Ser Thr Thr Gly Glu Ile Pro Phe Tyr Gly
               340                 345                 350
   Lys Ala Ile Pro Leu Glu Val Ile Lys Gly Gly Arg His Leu Ile Phe
           355                 360                 365
   Cys His Ser Lys Lys Lys Cys Asp Glu Leu Ala Ala Lys Leu Val Ala
       370                 375                 380
   Leu Gly Ile Asn Ala Val Ala Tyr Tyr Arg Gly Leu Asp Val Ser Val
   385                 390                 395                 400
   Ile Pro Pro Ile Gly Asp Val Val Val Val Ala Thr Asp Ala Leu Met
                   405                 410                 415
   Thr Gly Tyr Thr Gly Asp Phe Asp Ser Val Ile Asp Cys Asn Thr Cys
               420                 425                 430
   Val Thr Gln Thr Val Asp Phe Ser Leu Asp Pro Thr Phe Thr Ile Glu
           435                 440                 445
   Thr Ile Thr Leu Pro Gln Asp Ala Val Ser Arg Thr Gln Arg Arg Gly
       450                 455                 460
   Arg Thr Gly Arg Gly Lys Pro Gly Ile Tyr Arg Phe Val Ala Pro Gly
   465                 470                 475                 480
   Glu Arg Pro Ser Gly Met Phe Asp Ser Ser Val Leu Cys Glu Cys Tyr
                   485                 490                 495
   Asp Ala Gly Cys Ala Trp Tyr Glu Leu Thr Pro Ala Glu Thr Thr Val
               500                 505                 510
   Arg Leu Arg Ala Tyr Met Asn Thr Pro Gly Leu Pro Val Cys Gln Asp
           515                 520                 525
   His Leu Glu Phe Trp Glu Gly Val Phe Thr Gly Leu Thr His Ile Asp
       530                 535                 540
   Ala His Phe Leu Ser Gln Thr Lys Gln Ser Gly Glu Asn Leu Pro Tyr
   545 550 555 560
   Leu Val Ala Tyr Gln Ala Thr Val Cys Ala Arg Ala Gln Ala Pro Pro
   565 570 575
   Pro Ser Trp Asp Gln Met Trp Lys Cys Leu Ile Arg Leu Lys Pro Thr
   580 585 590
   Leu His Gly Pro Thr Pro Leu Leu Tyr Arg Leu Gly Ala Val Gln Asn
   595 600 605
   Glu Ile Thr Leu Thr His Pro Val Thr Lys Tyr Ile Met Thr Cys Met
   610 615 620
   Ser Ala Asp Leu Glu Val Val Thr Ser Thr Trp Val Leu Val Gly Gly
   625 630 635 640
   Val Leu Ala Ala Leu Ala Ala Tyr Cys Leu Ser Thr Gly Cys Val Val
   645 650 655
   Ile Val Gly Arg Val Val Leu Ser Gly Lys Pro Ala Ile Ile Pro Asp
   660 665 670
   Arg Glu Val Leu Tyr Arg Glu Phe Asp Glu Met Glu Glu Cys
   675 680 685
<210> 3
   <211> 1899
   <212> DNA
   <213> Artificial
<220>
   <223> NS3PI.1165
<400> 3
   atggcgccaa tcactgctta cgctcaacaa accagaggcc tcctagggtg cataatcacc
   60
   agcctaactg gccgggacaa aaaccaagtg gagggtgagg tccagattgt gtcaactgct
   120
   gcccaaacct tcctggcaac gtgcatcaat ggggtgtgct ggactgtcta ccacggggcc
   180
   ggaacgagga ccatcgcgtc acccaagggt cctgtcatcc agatgtatac caatgtagac
   240
   caagaccttg tgggctggcc cgctccgcaa ggtagccgat cattgacacc ctgcacttgc 300
   ggctcctcgg acctttacct ggtcacgagg cacgccgatg tcattcccgt gcgccggcgg 360
   ggtgatagca ggggcagcct gctgtcgccc cggcccattt cctacttgaa aggctccgca 420
   gggggtccgc tgttgtgccc cgcggggcac gccgtgggca tatttagggc cgcggtgtgc 480
   acccgtggag tggctaaggc ggtggacttt atccctgtgg agaacctaga gacaaccatg 540
   aggtccccgg tgttcacgga taactcctct ccaccagtag tgccccagag cttccaggtg 600
   gctcacctcc atgctcccac aggcagcggc aaaagcacca aggtcccggc tgcatatgca 660
   gctcagggct ataaggtgct agtactcaac ccctctgttg ctgcaacact gggctttggt 720
   gcttacatgt ccaaggctca tgggatcgat cctaacatca ggaccggggt gagaacaatt 780
   accactggca gccccatcac gtactccacc tacggcaagt tccttgccga cggcgggtgc 840
   tcggggggcg cttatgacat aataatttgt gacgagtgcc actccacgga tgccacatcc 900
   atcttgggca ttggcactgt ccttgaccaa gcagagactg cgggggcgag actggttgtg 960
   ctcgccaccg ccacccctcc gggctccgtc actgtgcccc atcccaacat cgaggaggtt 1020
   gctctgtcca ccaccggaga gatccctttt tacggcaagg ctatccccct cgaagtaatc 1080
   aaggggggga gacatctcat cttctgtcat tcaaagaaga agtgcgacga actcgccgca 1140
   aagctggtcg cattgggcat caatgccgtg gcctactacc gcggtcttga cgtgtccgtc 1200
   atcccgccca tcggcgatgt tgtcgtcgtg gcaaccgatg ccctcatgac cggctatacc 1260
   ggcgacttcg actcggtgat agactgcaat acgtgtgtca cccagacagt cgatttcagc 1320
   cttgacccta ccttcaccat tgagacaatc acgctccccc aagatgctgt ctcccgcact 1380
   caacgtcggg gcaggactgg cagggggaag ccaggcatct acagatttgt ggcaccgggg 1440
   gagcgcccct ccggcatgtt cgactcgtcc gtcctctgtg agtgctatga cgcaggctgt 1500
   gcttggtatg agctcacgcc cgccgagact acagttaggc tacgagcgta catgaacacc 1560
   ccggggcttc ccgtgtgcca ggaccatctt gaattttggg agggcgtctt tacaggcctc 1620
   actcatatag atgcccactt tctatcccag acaaagcaga gtggggagaa ccttccttac 1680
   ctggtagcgt accaagccac cgtgtgcgct agggctcaag cccctccccc atcgtgggac 1740
   cagatgtgga agtgtttgat tcgcctcaag cccaccctcc atgggccaac acccctgcta 1800
   tacagactgg gcgctgttca gaatgaaatc accctgacgc acccagtcac caaatacatc 1860
   atgacatgca tgtcggccga cctggaggtc gtcacgtga 1899
<210> 4
   <211> 632
   <212> PRT
   <213> Artificial
<220>
   <223> NS3PI.1165
<400> 4
   Met Ala Pro Ile Thr Ala Tyr Ala Gln Gln Thr Arg Gly Leu Leu Gly
   1 5 10 15
   Cys Ile Ile Thr Ser Leu Thr Gly Arg Asp Lys Asn Gln Val Glu Gly
   20 25 30
   Glu Val Gln Ile Val Ser Thr Ala Ala Gln Thr Phe Leu Ala Thr Cys
   35 40 45
   Ile Asn Gly Val Cys Trp Thr Val Tyr His Gly Ala Gly Thr Arg Thr
   50 55 60
   Ile Ala Ser Pro Lys Gly Pro Val Ile Gln Met Tyr Thr Asn Val Asp
   65 70 75 80
   Gln Asp Leu Val Gly Trp Pro Ala Pro Gln Gly Ser Arg Ser Leu Thr
                   85                  90                  95
   Pro Cys Thr Cys Gly Ser Ser Asp Leu Tyr Leu Val Thr Arg His Ala
               100                 105                 110
   Asp Val Ile Pro Val Arg Arg Arg Gly Asp Ser Arg Gly Ser Leu Leu
           115                 120                 125
   Ser Pro Arg Pro Ile Ser Tyr Leu Lys Gly Ser Ala Gly Gly Pro Leu
       130                 135                 140
   Leu Cys Pro Ala Gly His Ala Val Gly Ile Phe Arg Ala Ala Val Cys
   145                 150                 155                 160
   Thr Arg Gly Val Ala Lys Ala Val Asp Phe Ile Pro Val Glu Asn Leu
                   165                 170                 175
   Glu Thr Thr Met Arg Ser Pro Val Phe Thr Asp Asn Ser Ser Pro Pro
               180                 185                 190
   Val Val Pro Gln Ser Phe Gln Val Ala His Leu His Ala Pro Thr Gly
           195                 200                 205
   Ser Gly Lys Ser Thr Lys Val Pro Ala Ala Tyr Ala Ala Gln Gly Tyr
       210                 215                 220
   Lys Val Leu Val Leu Asn Pro Ser Val Ala Ala Thr Leu Gly Phe Gly
   225                 230                 235                 240
   Ala Tyr Met Ser Lys Ala His Gly Ile Asp Pro Asn Ile Arg Thr Gly
                   245                 250                 255
   Val Arg Thr Ile Thr Thr Gly Ser Pro Ile Thr Tyr Ser Thr Tyr Gly
               260                 265                 270
   Lys Phe Leu Ala Asp Gly Gly Cys Ser Gly Gly Ala Tyr Asp Ile Ile
           275                 280                 285
   Ile Cys Asp Glu Cys His Ser Thr Asp Ala Thr Ser Ile Leu Gly Ile
       290                 295                 300
   Gly Thr Val Leu Asp Gln Ala Glu Thr Ala Gly Ala Arg Leu Val Val
   305 310 315 320
   Leu Ala Thr Ala Thr Pro Pro Gly Ser Val Thr Val Pro His Pro Asn
   325 330 335
   Ile Glu Glu Val Ala Leu Ser Thr Thr Gly Glu Ile Pro Phe Tyr Gly
   340 345 350
   Lys Ala Ile Pro Leu Glu Val Ile Lys Gly Gly Arg His Leu Ile Phe
   355 360 365
   Cys His Ser Lys Lys Lys Cys Asp Glu Leu Ala Ala Lys Leu Val Ala
   370 375 380
   Leu Gly Ile Asn Ala Val Ala Tyr Tyr Arg Gly Leu Asp Val Ser Val
   385 390 395 400
   Ile Pro Pro Ile Gly Asp Val Val Val Val Ala Thr Asp Ala Leu Met
   405 410 415
   Thr Gly Tyr Thr Gly Asp Phe Asp Ser Val Ile Asp Cys Asn Thr Cys
   420 425 430
   Val Thr Gln Thr Val Asp Phe Ser Leu Asp Pro Thr Phe Thr Ile Glu
   435 440 445
   Thr Ile Thr Leu Pro Gln Asp Ala Val Ser Arg Thr Gln Arg Arg Gly
   450 455 460
   Arg Thr Gly Arg Gly Lys Pro Gly Ile Tyr Arg Phe Val Ala Pro Gly
   465 470 475 480
   Glu Arg Pro Ser Gly Met Phe Asp Ser Ser Val Leu Cys Glu Cys Tyr
   485 490 495
   Asp Ala Gly Cys Ala Trp Tyr Glu Leu Thr Pro Ala Glu Thr Thr Val
   500 505 510
   Arg Leu Arg Ala Tyr Met Asn Thr Pro Gly Leu Pro Val Cys Gln Asp
   515 520 525
   His Leu Glu Phe Trp Glu Gly Val Phe Thr Gly Leu Thr His Ile Asp
   530 535 540
   Ala His Phe Leu Ser Gln Thr Lys Gln Ser Gly Glu Asn Leu Pro Tyr
   545 550 555 560
   Leu Val Ala Tyr Gln Ala Thr Val Cys Ala Arg Ala Gln Ala Pro Pro
   565 570 575
   Pro Ser Trp Asp Gln Met Trp Lys Cys Leu Ile Arg Leu Lys Pro Thr
   580 585 590
   Leu His Gly Pro Thr Pro Leu Leu Tyr Arg Leu Gly Ala Val Gln Asn
   595 600 605
   Glu Ile Thr Leu Thr His Pro Val Thr Lys Tyr Ile Met Thr Cys Met
   610 615 620
   Ser Ala Asp Leu Glu Val Val Thr
   625 630
<210> 5
   <211> 1941
   <212> DNA
   <213> Artificial
<220>
   <223> d.4a.t.NS3PI.1165
<400> 5
   atgggctgcg tggtcatagt gggcagggtc gtcttgtccg gttccggttc catcactgct
   60
   tacgctcaac aaaccagagg cctcctaggg tgcataatca ccagcctaac tggccgggac
   120
   aaaaaccaag tggagggtga ggtccagatt gtgtcaactg ctgcccaaac cttcctggca
   180
   acgtgcatca atggggtgtg ctggactgtc taccacgggg ccggaacgag gaccatcgcg
   240
   tcacccaagg gtcctgtcat ccagatgtat accaatgtag accaagacct tgtgggctgg
   300
   cccgctccgc aaggtagccg atcattgaca ccctgcactt gcggctcctc ggacctttac
   360
   ctggtcacga ggcacgccga tgtcattccc gtgcgccggc ggggtgatag caggggcagc
   420
   ctgctgtcgc cccggcccat ttcctacttg aaaggctccg cagggggtcc gctgttgtgc
   480
   cccgcggggc acgccgtggg catatttagg gccgcggtgt gcacccgtgg agtggctaag
   540
   gcggtggact ttatccctgt ggagaaccta gagacaacca tgaggtcccc ggtgttcacg
   600
   gataactcct ctccaccagt agtgccccag agcttccagg tggctcacct ccatgctccc
   660
   acaggcagcg gcaaaagcac caaggtcccg gctgcatatg cagctcaggg ctataaggtg
   720
   ctagtactca acccctctgt tgctgcaaca ctgggctttg gtgcttacat gtccaaggct
   780
   catgggatcg atcctaacat caggaccggg gtgagaacaa ttaccactgg cagccccatc
   840
   acgtactcca cctacggcaa gttccttgcc gacggcgggt gctcgggggg cgcttatgac
   900
   ataataattt gtgacgagtg ccactccacg gatgccacat ccatcttggg cattggcact
   960
   gtccttgacc aagcagagac tgcgggggcg agactggttg tgctcgccac cgccacccct
   1020
   ccgggctccg tcactgtgcc ccatcccaac atcgaggagg ttgctctgtc caccaccgga
   1080
   gagatccctt tttacggcaa ggctatcccc ctcgaagtaa tcaagggggg gagacatctc
   1140
   atcttctgtc attcaaagaa gaagtgcgac gaactcgccg caaagctggt cgcattgggc
   1200
   atcaatgccg tggcctacta ccgcggtctt gacgtgtccg tcatcccgcc catcggcgat
   1260
   gttgtcgtcg tggcaaccga tgccctcatg accggctata ccggcgactt cgactcggtg
   1320
   atagactgca atacgtgtgt cacccagaca gtcgatttca gccttgaccc taccttcacc
   1380
   attgagacaa tcacgctccc ccaagatgct gtctcccgca ctcaacgtcg gggcaggact
   1440
   ggcaggggga agccaggcat ctacagattt gtggcaccgg gggagcgccc ctccggcatg
   1500
   ttcgactcgt ccgtcctctg tgagtgctat gacgcaggct gtgcttggta tgagctcacg
   1560
   cccgccgaga ctacagttag gctacgagcg tacatgaaca ccccggggct tcccgtgtgc 1620
   caggaccatc ttgaattttg ggagggcgtc tttacaggcc tcactcatat agatgcccac 1680
   tttctatccc agacaaagca gagtggggag aaccttcctt acctggtagc gtaccaagcc 1740
   accgtgtgcg ctagggctca agcccctccc ccatcgtggg accagatgtg gaagtgtttg 1800
   attcgcctca agcccaccct ccatgggcca acacccctgc tatacagact gggcgctgtt 1860
   cagaatgaaa tcaccctgac gcacccagtc accaaataca tcatgacatg catgtcggcc 1920
   gacctggagg tcgtcacgtg a 1941
<210> 6
   <211> 646
   <212> PRT
   <213> Artificial
<220>
   <223> d.4a.t.NS3PI.1165
<400> 6
   Met Gly Cys Val Val Ile Val Gly Arg Val Val Leu Ser Gly Ser Gly
   1 5 10 15
   Ser Ile Thr Ala Tyr Ala Gln Gln Thr Arg Gly Leu Leu Gly Cys Ile
   20 25 30
   Ile Thr Ser Leu Thr Gly Arg Asp Lys Asn Gln Val Glu Gly Glu Val
   35 40 45
   Gln Ile Val Ser Thr Ala Ala Gln Thr Phe Leu Ala Thr Cys Ile Asn
   50 55 60
   Gly Val Cys Trp Thr Val Tyr His Gly Ala Gly Thr Arg Thr Ile Ala
   65 70 75 80
   Ser Pro Lys Gly Pro Val Ile Gln Met Tyr Thr Asn Val Asp Gln Asp
   85 90 95
   Leu Val Gly Trp Pro Ala Pro Gln Gly Ser Arg Ser Leu Thr Pro Cys
   100 105 110
   Thr Cys Gly Ser Ser Asp Leu Tyr Leu Val Thr Arg His Ala Asp Val
   115 120 125
   Ile Pro Val Arg Arg Arg Gly Asp Ser Arg Gly Ser Leu Leu Ser Pro
   130 135 140
   Arg Pro Ile Ser Tyr Leu Lys Gly Ser Ala Gly Gly Pro Leu Leu Cys
   145 150 155 160
   Pro Ala Gly His Ala Val Gly Ile Phe Arg Ala Ala Val Cys Thr Arg
   165 170 175
   Gly Val Ala Lys Ala Val Asp Phe Ile Pro Val Glu Asn Leu Glu Thr
   180 185 190
   Thr Met Arg Ser Pro Val Phe Thr Asp Asn Ser Ser Pro Pro Val Val
   195 200 205
   Pro Gln Ser Phe Gln Val Ala His Leu His Ala Pro Thr Gly Ser Gly
   210 215 220
   Lys Ser Thr Lys Val Pro Ala Ala Tyr Ala Ala Gln Gly Tyr Lys Val
   225 230 235 240
   Leu Val Leu Asn Pro Ser Val Ala Ala Thr Leu Gly Phe Gly Ala Tyr
   245 250 255
   Met Ser Lys Ala His Gly Ile Asp Pro Asn Ile Arg Thr Gly Val Arg
   260 265 270
   Thr Ile Thr Thr Gly Ser Pro Ile Thr Tyr Ser Thr Tyr Gly Lys Phe
   275 280 285
   Leu Ala Asp Gly Gly Cys Ser Gly Gly Ala Tyr Asp Ile Ile Ile Cys
   290 295 300
   Asp Glu Cys His Ser Thr Asp Ala Thr Ser Ile Leu Gly Ile Gly Thr
   305 310 315 320
   Val Leu Asp Gln Ala Glu Thr Ala Gly Ala Arg Leu Val Val Leu Ala
   325 330 335
   Thr Ala Thr Pro Pro Gly Ser Val Thr Val Pro His Pro Asn Ile Glu
   340 345 350
   Glu Val Ala Leu Ser Thr Thr Gly Glu Ile Pro Phe Tyr Gly Lys Ala
   355 360 365
   Ile Pro Leu Glu Val Ile Lys Gly Gly Arg His Leu Ile Phe Cys His
   370 375 380
   Ser Lys Lys Lys Cys Asp Glu Leu Ala Ala Lys Leu Val Ala Leu Gly
   385 390 395 400
   Ile Asn Ala Val Ala Tyr Tyr Arg Gly Leu Asp Val Ser Val Ile Pro
   405 410 415
   Pro Ile Gly Asp Val Val Val Val Ala Thr Asp Ala Leu Met Thr Gly
   420 425 430
   Tyr Thr Gly Asp Phe Asp Ser Val Ile Asp Cys Asn Thr Cys Val Thr
   435 440 445
   Gln Thr Val Asp Phe Ser Leu Asp Pro Thr Phe Thr Ile Glu Thr Ile
   450 455 460
   Thr Leu Pro Gln Asp Ala Val Ser Arg Thr Gln Arg Arg Gly Arg Thr
   465 470 475 480
   Gly Arg Gly Lys Pro Gly Ile Tyr Arg Phe Val Ala Pro Gly Glu Arg
   485 490 495
   Pro Ser Gly Met Phe Asp Ser Ser Val Leu Cys Glu Cys Tyr Asp Ala
   500 505 510
   Gly Cys Ala Trp Tyr Glu Leu Thr Pro Ala Glu Thr Thr Val Arg Leu
   515 520 525
   Arg Ala Tyr Met Asn Thr Pro Gly Leu Pro Val Cys Gln Asp His Leu
   530 535 540
   Glu Phe Trp Glu Gly Val Phe Thr Gly Leu Thr His Ile Asp Ala His
   545 550 555 560
   Phe Leu Ser Gln Thr Lys Gln Ser Gly Glu Asn Leu Pro Tyr Leu Val
   565 570 575
   Ala Tyr Gln Ala Thr Val Cys Ala Arg Ala Gln Ala Pro Pro Pro Ser
   580 585 590
   Trp Asp Gln Met Trp Lys Cys Leu Ile Arg Leu Lys Pro Thr Leu His
   595 600 605
   Gly Pro Thr Pro Leu Leu Tyr Arg Leu Gly Ala Val Gln Asn Glu Ile
   610 615 620
   Thr Leu Thr His Pro Val Thr Lys Tyr Ile Met Thr Cys Met Ser Ala
   625 630 635 640
   Asp Leu Glu Val Val Thr
   645
<210> 7
   <211> 546
   <212> DNA
   <213> Artificial
<220>
   <223> native, unmodified NS3 protease domain
<400> 7
   atggcgccca tcacggcgta cgcccagcag acaaggggcc tcctagggtg cataatcacc
   60
   agcctaactg gccgggacaa aaaccaagtg gagggtgagg tccagattgt gtcaactgct
   120
   gcccaaacct tcctggcaac gtgcatcaat ggggtgtgct ggactgtcta ccacggggcc
   180
   ggaacgagga ccatcgcgtc acccaagggt cctgtcatcc agatgtatac caatgtagac
   240
   caagaccttg tgggctggcc cgctccgcaa ggtagccgat cattgacacc ctgcacttgc
   300
   ggctcctcgg acctttacct ggtcacgagg cacgccgatg tcattcccgt gcgccggcgg
   360
   ggtgatagca ggggcagcct gctgtcgccc cggcccattt cctacttgaa aggctcctcg
   420
   gggggtccgc tgttgtgccc cgcggggcac gccgtgggca tatttagggc cgcggtgtgc
   480
   acccgtggag tggctaaggc ggtggacttt atccctgtgg agaacctaga gacaaccatg
   540
   aggtcc
   546
<210> 8
   <211> 182
   <212> PRT
   <213> Artificial
<220>
   <223> native, unmodified NS3 protease domain
<400> 8
   Met Ala Pro Ile Thr Ala Tyr Ala Gln Gln Thr Arg Gly Leu Leu Gly
   1 5 10 15
   Cys Ile Ile Thr Ser Leu Thr Gly Arg Asp Lys Asn Gln Val Glu Gly
   20 25 30
   Glu Val Gln Ile Val Ser Thr Ala Ala Gln Thr Phe Leu Ala Thr Cys
   35 40 45
   Ile Asn Gly Val Cys Trp Thr Val Tyr His Gly Ala Gly Thr Arg Thr
   50 55 60
   Ile Ala Ser Pro Lys Gly Pro Val Ile Gln Met Tyr Thr Asn Val Asp
   65 70 75 80
   Gln Asp Leu Val Gly Trp Pro Ala Pro Gln Gly Ser Arg Ser Leu Thr
   85 90 95
   Pro Cys Thr Cys Gly Ser Ser Asp Leu Tyr Leu Val Thr Arg His Ala
   100 105 110
   Asp Val Ile Pro Val Arg Arg Arg Gly Asp Ser Arg Gly Ser Leu Leu
   115 120 125
   Ser Pro Arg Pro Ile Ser Tyr Leu Lys Gly Ser Ser Gly Gly Pro Leu
   130 135 140
   Leu Cys Pro Ala Gly His Ala Val Gly Ile Phe Arg Ala Ala Val Cys
   145 150 155 160
   Thr Arg Gly Val Ala Lys Ala Val Asp Phe Ile Pro Val Glu Asn Leu
   165 170 175
   Glu Thr Thr Met Arg Ser
   180
<210> 9
   <211> 3297
   <212> DNA
   <213> Artificial
<220>
   <223> MEFA 7.1
<400> 9
   atggctacaa aggctgtttg tgttttgaag ggtgacggcc cagttcaagg tattattaac 60
   ttcgagcaga aggaaagtaa tggaccagtg aaggtgtggg gaagcattaa aggactgact 120
   gaaggcctgc atggattcca tgttcatgag tttggagata atacagcagg ctgtaccagt 180
   gcaggtcctc actttaatcc tctatccaga aaacacggtg ggccaaagga tgaagagagg 240
   catgttggag acttgggcaa tgtgactgct gacaaagatg gtgtggccga tgtgtctatt 300
   gaagattctg tgatctcact ctcaggagac cattgcatca ttggccgcac actggtggtc 360
   catgaaaaag cagatgactt gggcaaaggt ggaaatgaag aaagtacaaa gacaggaaac 420
   gctggaagtc gtttggcttg tggtgtaatt gggatcgccc agaatttgaa ttctggttgc 480
   aattgctcta tctatcccgg ccatataacg ggtcaccgca tggcatggaa gcttggttcc 540
   gccgccagaa ctacctcggg ctttgtctcc ttgttcgccc caggtgccaa acaaaacgaa 600
   actcacgtca cgggaggcgc agccgcccga actacgtctg ggttgacctc tttgttctcc 660
   ccaggtgcca gccaaaacat tcaattgatt gtcgacttta tccctgtgga gaacctagag 720
   acaaccatgc gatctccggt gttcacggat aactcctctc caccagtagt gccccagagc 780
   ttccaggtgg ctcacctcca tgctcccaca ggcagcggca aaagcaccaa ggtcccggct 840
   gcatatgcag ctcagggcta taaggtgcta gtactcaacc cctctgttgc tgcaacactg 900
   ggctttggtg cttacatgtc caaggctcat gggatcgatc ctaacatcag gaccggggtg 960
   agaacaatta ccactggcag ccccatcacg tactccacct acggcaagtt ccttgccgac 1020
   ggcgggtgct cggggggcgc ttatgacata ataatttgtg acgagtgcca ctccacggat
   1080
   gccacatcca tcttgggcat tggcactgtc cttgaccaag cagagactgc gggggcgaga
   1140
   ctggttgtgc tcgccaccgc cacccctccg ggctccgtca ctgtgcccca tcccaacatc
   1200
   gaggaggttg ctctgtccac caccggagag atcccttttt acggcaaggc tatccccctc
   1260
   gaagtaatca agggggggag acatctcatc ttctgtcatt caaagaagaa gtgcgacgaa
   1320
   ctcgccgcaa agctggtcgc attgggcatc aatgccgtgg cctactaccg cggtcttgac
   1380
   gtgtccgtca tcccgaccag cggcgatgtt gtcgtcgtgg caaccgatgc cctcatgacc
   1440
   ggctataccg gcgacttcga ctcggtgata gactgcaata cgtgtgtcac ccagacagtc
   1500
   gatttcagcc ttgaccctac cttcaccatt gagacaatca cgctccccca agatgctgtc
   1560
   tcccgcactc aacgtcgggg caggactggc agggggaagc caggcatcta cagatttgtg
   1620
   gcaccggggg agcgcccctc cggcatgttc gactcgtccg tcctctgtga gtgctatgac
   1680
   gcaggctgtg cttggtatga gctcacgccc gccgagacta cagttaggct acgagcgtac
   1740
   atgaacaccc cggggcttcc cgtgtgccag gaccatcttg aattttggga gggcgtcttt
   1800
   acaggcctca ctcatataga tgcccacttt ctatcccaga caaagcagag tggggagaac
   1860
   cttccttacc tggtagcgta ccaagccacc gtgtgcgcta gggctcaagc cctcccccca
   1920
   tcgtgggacc agatgtggaa gtgtttgatt cgcctcaagc ccaccctcca tgggccaaca
   1980
   cccctgctat acagactggg cgctgttcag aatgaaatca ccctgacgca cccagtcacc
   2040
   aaatacatca tgacatgcat gtcggccgac ctggaggtcg tcacgagcgc atgctccggg
   2100
   aagccggcaa tcatacctga cagggaagtc ctctaccgag agttcgatga gatggaagag
   2160
   tgctctcagc acttaccgta catcgagcaa gggatgatgc tcgccgagca gttcaagcag
   2220
   aaggccctcg gcctctcgcg agggggcaag ccggcaatcg ttccagacaa agaggtgttg
   2280
   tatcaacaat acgatgagat ggaagagtgc tcacaagctg ccccatatat cgaacaagct
   2340
   caggtaatag ctcaccagtt caaggaaaaa gtccttggat tgatcgataa tgatcaagtg
   2400
   gttgtgactc ctgacaaaga aatcttatat gaggcctttg atgagatgga agaatgcgcc
   2460
   tccaaagccg ccctcattga ggaagggcag cggatggcgg agatgctcaa gtctaagata
   2520
   caaggcctcc tcgggatact gcgccggcac gttggtcctg gcgagggggc agtgcagtgg
   2580
   atgaaccggc tgatagcctt cgcctccaga gggaaccatg tttcccccac gcactacgtt
   2640
   ccgtctagat cccggagatt cgcccaggcc ctgcccgttt gggcgcggcc ggactataac
   2700
   cccccgctag tggagacgtg gaaaaagccc gactacgaac cacctgtggt ccacggcaga
   2760
   tcttctcgga gattcgccca ggccctgccc gtttgggcgc ggccggacta taaccccccg
   2820
   ctagtggaga cgtggaaaaa gcccgactac gaaccacctg tggtccatgg cagaaagacc
   2880
   aaacgtaaca ccaaccggcg gccgcaggac gtcaagttcc cgggtggcgg tcagatcgtt
   2940
   ggtcgcaggg gccctcctat ccccaaggct cgtcggcccg agggcaggac ctgggctcag
   3000
   cccggttacc cttggcccct ctatggcaat aaggacagac ggtctacagg taagtcctgg
   3060
   ggtaagccag ggtacccttg gccaagaaag accaaacgta acaccaaccg acggccgcag
   3120
   gacgtcaagt tcccgggtgg cggtcagatc gttggtcgca ggggccctcc tatccccaag
   3180
   gctcgtcggc ccgagggcag gacctgggct cagcccggtt acccttggcc cctctatggc
   3240
   aataaggaca gacggtctac cggtaagtcc tggggtaagc cagggtatcc ttggccc
   3297
<210> 10
   <211> 1099
   <212> PRT
   <213> Artificial
<220>
   <223> MEFA 7.1
<400> 10
<210> 11
   <211> 21
   <212> PRT
   <213> Artificial
<220>
   <223> HCV epitopes
<400> 11
   Gly Ser Ala Ala Arg Thr Thr Ser Gly Phe Val Ser Leu Phe Ala Pro
   1 5 10 15
   Gly Ala Lys Gln Asn
   20
<210> 12
   <211> 10
   <212> DNA
   <213> Artificial
<220>
   <223> synthetic oligonucleotide
<400> 12
   acaaaacaaa
   10
<210> 13
   <211> 23
   <212> PRT
   <213> Artificial
<220>
   <223> an NS4a peptide
<400> 13
   Lys Lys Gly Ser Val Val Ile Val Gly Arg Ile Val Leu Ser Gly Lys
   1 5 10 15
   Pro Ala Ile Ile Pro Lys Lys
   20
<210> 14
   <211> 41
   <212> DNA
   <213> Artificial
<220>
   <223> HBgl-1
<400> 14
   agcttacaaa acaaaatgca tcaccatcac catcacgcgc c
   41
<210> 15
   <211> 52
   <212> DNA
   <213> Artificial
<220>
   <223> Hbgl-2
<400> 15
   gcgtacgccg tgatgggcgc gtgatggtga tggtgatgca ttttgttttg ta
   52
<210> 16
   <211> 60
   <212> DNA
   <213> Artificial
<220>
   <223> Hbgl-5
<400> 16
   catcacggcg tacgcccagc agacaagggg cctcctaggg tgcataatca ccagcctaac
   60
<210> 17
   <211> 42
   <212> DNA
   <213> Artificial
<220>
   <223> Hbgl-6
<400> 17
   aggctggtga ttatgcaccc taggaggccc cttgtctgct gg
   42
<210> 18
   <211> 34
   <212> DNA
   <213> Artificial
<220>
   <223> PCR primer
<400> 18
   tttcctactt gaaaggctcc gcagggggtc cgct
   34
<210> 19
   <211> 30
   <212> DNA
   <213> Artificial
<220>
   <223> PCR primer
<400> 19
   ggagcctttc aagtaggaaa tgggccgggg
   30
<210> 20
   <211> 57
   <212> DNA
   <213> Artificial
<220>
   <223> HA-1
<400> 20
   agcttacaaa acaaaatggc gccaatcact gcttacgctc aacaaaccag aggcctc
   57
<210> 21
   <211> 57
   <212> DNA
   <213> Artificial
<220>
   <223> HA-2
<400> 21
   ctaggaggcc tctggtttgt tgagcgtaag cagtgattgg cgccattttg ttttgta
   57
<210> 22
   <211> 84
   <212> DNA
   <213> Artificial
<220>
   <223> PI3p-1
<400> 22
   ggccgacctg gaggtcgtca cgagcacctg ggtgctcgtt ggcggcgtcc tggctgcttt 60
   ggccgcgtat tgcctgtcaa cagg 84
<210> 23
   <211> 73
   <212> DNA
   <213> Artificial
<220>
   <223> PI3p-2
<400> 23
   ctgcgtggtc atagtgggca gggtcgtctt gtccgggaag ccggcaatca tacctgacag 60
   ggaagtcctc tac 73
<210> 24
   <211> 78
   <212> DNA
   <213> Artificial
<220>
   <223> PI3p-3
<400> 24
   gtcaggtatg attgccggct tcccggacaa gacgaccctg cccactatga ccacgcagcc 60
   tgttgacagg caatacgc 78
<210> 25
   <211> 60
   <212> DNA
   <213> Artificial
<220>
   <223> PI3p-4
<400> 25
   ggccaaagca gccaggacgc cgccaacgag cacccaggtg ctcgtgacga cctccaggtc 60
<210> 26
   <211> 34
   <212> DNA
   <213> Artificial
<220>
   <223> PI3p-5
<400> 26
   cgagagttcg atgagatgga agagtgctga taag
   34
<210> 27
   <211> 53
   <212> DNA
   <213> Artificial
<220>
   <223> PI3p-6
<400> 27
   tcgacttatc agcactcttc catctcatcg aactctcggt agaggacttc cct
   53
<210> 28
   <211> 33
   <212> DNA
   <213> Artificial
<220>
   <223> avsal-1
<400> 28
   tgtcggccga cctggaggtc gtcacgtgat aag
   33
<210> 29
   <211> 41
   <212> DNA
   <213> Artificial
<220>
   <223> aveal-2
<400> 29
   tcgacttatc acgtgacgac ctccaggtcg gccgacatgc a
   41
<210> 30
   <211> 27
   <212> DNA
   <213> Artificial
<220>
   <223> HA-5
<400> 30
   agcttacaaa acaaaatggg ctgcgtg
   27
<210> 31
   <211> 41
   <212> DNA
   <213> Artificial
<220>
   <223> HA-6
<400> 31
   gaccctgccc actatgacca cgcagcccat tttgttttgt a
   41
<210> 32
   <211> 72
   <212> DNA
   <213> Artificial
<220>
   <223> HA-7
<400> 32
   gtcatagtgg gcagggtcgt cttgtccggt tccggttcca tcactgctta cgctcaacaa
   60
   accagaggcc tc
   72
<210> 33
   <211> 58
   <212> DNA
   <213> Artificial
<220>
   <223> HA-8
<400> 33
   ctaggaggcc tctggtttgt tgagcgtaag cagtgatgga accggaaccg gacaagac
   58